# FASCICULE DE BREVET EUROPEEN

(11) **EP 1 687 312 B1**
(45) Date de publication et mention de la délivrance du brevet: **14.07.2010**
(21) Numéro de dépôt: 04805417.5
(22) Date de dépôt: 09.11.2004
(51) Int. Cl.: C07D 491/10, A61K 31/47, A61P 33/06

(54) **MOLECULES DUALES CONTENANT UN DERIVE PEROXYDIQUE, LEUR SYNTHESE ET LEURS APPLICATIONS THERAPEUTIQUES**
EIN PEROXYDERIVAT ENTHALTENDE DUALE MOLEKÜLE, DEREN SYNTHESE UND DEREN THERAPEUTISCHE ANWENDUNGEN
DUAL MOLECULES CONTAINING PEROXY DERIVATIVE, THE SYNTHESIS AND THERAPEUTIC APPLICATIONS THEREOF

(30) Priorité: 14.11.2003 FR 0313371
(43) Date de publication de la demande: 09.08.2006
(73) Titulaire: CENTRE NATIONAL DE LA RECHERCHE SCIENTIFIQUE, 75794 Paris Cédex 14 (FR); Palumed SA, 31682 Labege Cedex (FR); Sanofi-Aventis, 75013 Paris (FR)
(72) Inventeur: CAZELLES, Jérôme, FR-31130 Balma (FR); COSLEDAN, Frédéric, F-31750 Labege (FR); MEUNIER, Bernard, F-31320 Castanet (FR); PELLET, Alain, FR-31870 Beaumont Sur Leze (FR)
(74) Mandataire: Kugel, Dominique
(86) Numéro de dépôt international: PCT/FR2004/002874
(87) Numéro de publication internationale: WO 2005/049619

(56) Documents cités:
- WO-A-01/77105
- WO-A-03/000676

## Description

L'invention a pour objet des molécules duales contenant un dérivé peroxydique, possédant notamment une activité antipaludique, leur synthèse et leurs applications thérapeutiques.

Le paludisme est l'une des premières causes infectieuses de mortalité au monde et touche chaque année 100 à 200 millions de personnes. La forte recrudescence de la maladie observée depuis quelques années est due à plusieurs facteurs, dont :
- les vecteurs, à savoir les anophèles, qui deviennent résistants aux insecticides classiques et bon marché, comme le DDT (abréviation de trichloro-1,1,1-bis(p-chlorophényl)-2,2-éthane) ;
- l'augmentation de la population dans les zones à risque et, principalement,
- la résistance de nombreuses souches de *Plasmodium falciparum,* parasite responsable des formes mortelles de la maladie, aux médicaments classiquement utilisés, tels que la chloroquine et la méfloquine. La découverte de l'artémisinine, puissant antipaludique extrait de *Artemisia annua,* a attiré l'attention vers des molécules présentant, comme l'artémisinine, une fonction endoperoxyde.

L'artémisinine et certains de ses dérivés hémi-synthétiques, tels que l'artéméther et l'artésunate, se sont révélés très actifs sur les souches résistantes de *P. falciparum.* Cependant, le coût élevé de ces composés d'origine naturelle et les aléas d'approvisionnement représentent des inconvénients majeurs. On mesurera donc l'intérêt de composés antipaludiques de synthèse, qui seraient accessibles à bas prix, et présenteraient un mécanisme d'action semblable à celui de l'artémisinine, à savoir un effet alkylant sur l'hème (un des groupements constitutifs de l'hémoglobine) et/ou les protéines parasitaires.

Des peroxydes de synthèse à effet antipaludique ont par exemple été décrits dans la demande internationale PCT WO 03/000676.

La recherche de composés présentant des propriétés pharmacologiques améliorées, les rendant aptes à l'utilisation en tant que médicament antipaludique, a amené les inventeurs à développer une nouvelle stratégie de synthèse basée sur l'utilisation de composés susceptibles à la fois d'être accumulés efficacement dans le parasite et d'exercer un effet du type de celui de l'artémisinine.

Les inventeurs ont constaté qu'en formant un adduit covalent entre un composé doté de propriétés antipaludiques et un dérivé de type peroxydique, on disposait de produits de couplage avec, de manière surprenante, un effet synergique entre la faculté de pénétration et l'activité des constituants respectifs sur des souches chloroquino-résistantes, et de manière générale une grande efficacité pour un large spectre de parasites. De telles molécules duales, se présentant sous forme de produits de couplage, font l'objet de la demande de brevet WO 01177105. Les composés décrits dans WO01/77105 présentent notamment un groupe carbocyclique condensé avec le groupe peroxydique cyclique.

Les inventeurs ont à présent développé une famille de molécules du type indiqué ci-dessus, mais présentant un nombre réduit de stéréoisomères, avantage pour leur utilisation en tant que médicament. L'invention porte donc sur de telles molécules duales, leur synthèse et leurs applications biologiques, qui se révèlent du plus grand intérêt pour traiter les maladies parasitaires telles que le paludisme.

Les molécules duales selon la présente invention répondent à la formule (I) : 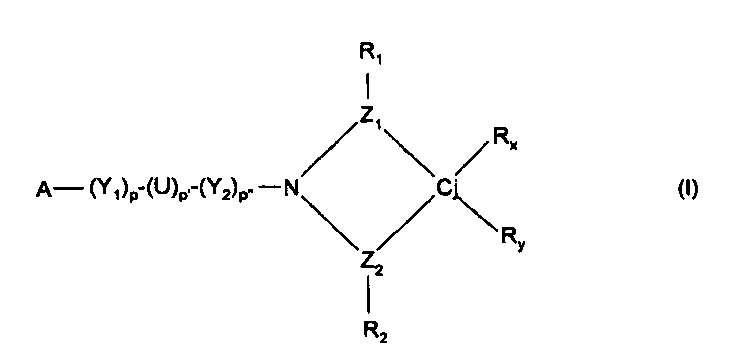 dans laquelle :
- A représente un résidu de molécule à activité antipaludique et/ou un résidu facilitant la biodisponibilité,
- p, p', p" représentent indépendamment les uns des autres 0 ou 1, l'un au moins de p ou de p" étant égal à 1,
- Y₁ et Y₂, identiques ou différents, représentent une chaîne alkylène en C1 à C6, linéaire, ramifiée ou cyclique, comportant éventuellement un ou plusieurs radicaux amine, amide, thioamide, sulfonyle, sulfonate, sulfonamide, carbonyle, thiocarbonyle, carboxyle, thiocarboxyle, éther ou thioéther, cette chaîne alkylène étant éventuellement substituée par un ou plusieurs groupes choisis parmi les atomes d'halogène, les groupes hydroxyles, les groupes acétals et les radicaux alkyles en C1 à C5, linéaires, ramifiés ou cycliques,
- U représente un radical amine, amide, thioamide, sulfonyle, sulfonate, sulfonamide, carbonyle, thiocarbonyle, carboxyle, thiocarboxyle, éther ou thioéther,
- Z₁ et Z₂, identiques ou différents, représentent un radical alkylène en C1 à C4 linéaire, ramifié ou cyclique, saturé ou insaturé, l'un de Z₁ ou Z₂ pouvant être absent, ou

groupe fonctionnel capable d'augmenter l'hydrosolubilité de la molécule duale,
- Rₓ et R_{y} forment ensemble un peroxyde cyclique comprenant de 4 à 8 chaînons et comportant 1 ou 2 atomes d'oxygène supplémentaires dans la structure cyclique (soit au total de 3 à 4 atomes d'oxygène dans le cycle), Cj étant l'un des sommets de ce peroxyde cyclique,
   . ledit peroxyde cyclique étant substitué par 1 à 8 groupes R₃, identiques ou différents les uns des autres, occupant des positions quelconques sur les atomes de carbone du cycle peroxydique et étant choisis parmi les atomes et groupes suivants :
      hydrogène, halogène, -OH, -CF₃, -NO₂, aryle (par exemple phényle) ou hétéroaryle (par exemple pyridinyle), alkyle ou -O-alkyle (lesdits groupes alkyles comprenant de 1 à 10 atomes de carbone et étant linéaires, ramifiés ou cycliques), ou
      structure mono-, bi- ou tricyclique en C3 à C18 pouvant en outre contenir 1 ou plusieurs (par exemple de 1 à 6) hétéroatomes choisis parmi l'oxygène, l'azote et le soufre, éventuellement substituée par un ou plusieurs groupes choisis parmi les atomes d'halogène, les groupes hydroxyles et les groupes alkyles en C1 à C6, linéaires, ramifiés ou cycliques ; des exemples de telles structures cycliques sont les groupes cycloalkyles (tels que les groupes cyclopropyle, cyclobutyle, cyclopentyle, cyclohexyle, cycloheptyle et cyclooctyle) et le groupe adamantyle (structure tricyclique comprenant 10 atomes de carbone),
   . l'un au moins des groupes R₃ étant différent d'un atome d'hydrogène,
   . deux groupes R₃ portés par des atomes de carbone adjacents sur le cycle peroxydique pouvant former ensemble une structure cyclique à 5 ou 6 chaînons, saturée ou insaturée, éventuellement substituée en positions quelconques par un ou plusieurs (par exemple de 1 à 3) substituants R₃ tels que définis ci-dessus,
   . deux groupes R₃ portés par le même atome de carbone du cycle peroxydique pouvant former ensemble une structure mono-, bi- ou tricyclique telle que définie ci-dessus (qui sera donc située en position spiro sur le cycle peroxydique).

De manière avantageuse, le résidu A draine à l'intérieur du parasite le composé de formule (I) selon l'invention, qui exerce alors un effet alkylant sur l'hème et/ou les protéines parasitaires.

Les composés de formule (I) peuvent exister à l'état de bases ou de sels d'addition à des acides. De tels sels d'addition font également partie de l'invention. Ces sels sont avantageusement préparés avec des acides pharmaceutiquement acceptables, mais les sels d'autres acides utiles, par exemple, pour la purification ou l'isolement des composés de formule (I), font également partie de l'invention.

Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

L'invention vise les mélanges racémiques, ainsi que les isomères optiquement purs des molécules de formule (I), et encore des mélanges en toutes proportions desdits isomères optiquement purs. Elle vise également les molécules achirales.

Dans la définition des composés de formule (I) ci-dessus et dans ce qui suit, sauf mention différente dans le texte, on entend par :
- atome d'halogène : un atome de F, Cl, Brou I,
- groupe alkyle : groupe monovalent provenant de l'enlèvement d'un atome d'hydrogène dans la molécule d'un hydrocarbure (tel qu'un groupe éthyle de formule -C₂H₅),
- radical ou chaîne alkylène : un groupement bivalent provenant de l'enlèvement de deux atomes d'hydrogène sur deux atomes de carbone différents d'un hydrocarbure (tel par exemple qu'une chaîne éthylène de formule -CH₂-CH₂-),
- groupe acétal : un groupe acétal cyclique ou non. A titre d'exemples on peut citer les groupes diméthoxyle, diéthoxyle, éthane-1,2-dioxyle, propane-1,3-dioxyle et 2,2-diméthyl-propane-1,3-dioxyle, répondant respectivement aux formules suivantes : 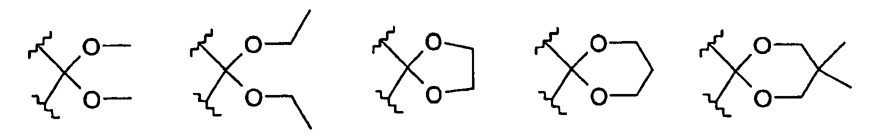
- résidu facilitant la biodisponibilité : une molécule cyclique en C6-C18 saturée ou insaturée, ou une chaîne en C1-C18 linéaire éventuellement substituée, ladite molécule et ladite chaîne comportant un ou plusieurs hétéroatomes choisis parmi N, O et S. A titre d'exemples de résidus facilitant la biodisponibilité, on peut citer les résidus guanidinium, morpholino, peptidiques ou de polyamines,
- groupe fonctionnel capable d'augmenter l'hydrosolubilité de la molécule duale : un groupe avantageusement choisi parmi -COOH, -OH ou -N(Rₐ,R_{b}) avec Rₐ et R_{b}, identiques ou différents, représentant un atome d'hydrogène ou un radical alkyle en C1 à C5, linéaire, ramifié ou cyclique,
- radical amine : un groupe de formule -NRₐ-, où Rₐ est tel que défini ci-dessus,
- radical amide : un groupe de formule -NRₐ-CO- ou -CO-NRₐ-, où Rₐ est tel que défini ci-dessus,
- radical thioamide : un groupe de formule -NRₐ-CS- ou -CS-NRₐ-, où Rₐ est tel que défini ci-dessus,
- radicaux sulfonyle et sufonate : des groupes de formules respectives -SO₂- et -SO₃-,
- radical sulfonamide : un groupe de formule -NRₐ-SO₂- ou - SO₂-NRₐ-, où Rₐ est tel que défini ci-dessus,
- radical carboxyle : un groupe de formule -CO-O- ou -O-CO-,
- radical thiocarboxyle : un groupe de formule -CS-O- ou -O-CS-,
- radicaux carbonyle et thiocarbonyle : des groupes de formules respectives -CO- et -CS-,
- radicaux éther et thioéther : des groupes de formules respectives -O- et -S-,

Dans les composés de formule (I) selon l'invention, A peut représenter un hétérocycle azoté de formule (II) : 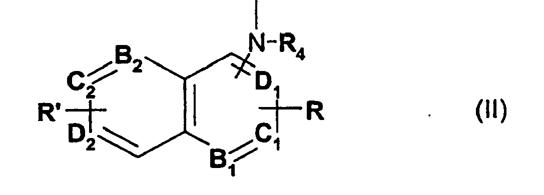 dans laquelle :
- R et R', identiques ou différents, représentent chacun un ou plusieurs (par exemple 1 à 3) substituants occupant des positions distinctes sur les cycles auxquels ils sont rattachés, choisis parmi les atomes d'hydrogène ou d'halogène et les groupes -OH, -CF₃, aryle (tel que phényle), hétéroaryle (tel que pyridinyle), alkyle ou -O-alkyle (lesdits groupes alkyles comprenant de 1 à 5 atomes de carbone et étant linéaires, ramifiés ou cycliques), -NO₂ ou -N(Rₐ,R_{b}), où Rₐ et R_{b}, identiques ou différents, représentent des atomes d'hydrogène ou des groupes alkyles comprenant de 1 à 5 atomes de carbone et étant linéaires, ramifiés ou cycliques ; l'un au moins de R ou R' étant différent d'un atome d'hydrogène ;
- R₄ représente un atome d'hydrogène ou un radical alkyle en C1 à C5, linéaire, ramifié ou cyclique, ou R₄ forme, avec un atome de carbone ou d'azote présent dans le groupement -(Y₁)ₚ-(U)ₚ-(Y₂)ₚ- de la formule (I), un cycle comportant de 5 à 8 chaînons (par exemple un cycle pipérazinyle),
- les radicaux B₁, C₁, D_{1,} B₂, C₂ et D₂ représentent des atomes d'azote ou des chaînons -CH=, étant donné que soit l'un des radicaux B₁, C₁ et D₁ représente un atome d'azote et les autres radicaux représentent des chaînons -CH=, soit l'un des radicaux B₂, C₂ et D₂ représente un atome d'azote et les autres radicaux représentent des chaînons -CH=, soit l'un des radicaux B_{1,} C₁ et D₁ et l'un des radicaux B₂, C₂ et D₂ représentent des atomes d'azote et les autres radicaux représentent des chaînons -CH=.

L'hétérocycle azoté de formule (II) peut notamment être choisi parmi une aminoquinoléine de formule (IIa) ou une 1,5-naphtyridine de formule (III) suivantes : 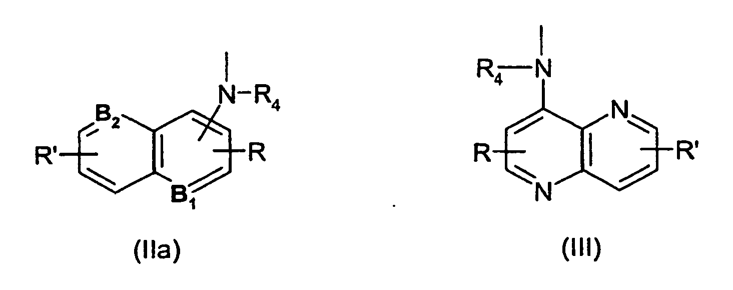 dans lesquelles R, R' et R₄ sont tels que définis ci-dessus et soit B₁ représente un atome d'azote et B₂ représente un chaînon -CH=, soit B₁ représente un chaînon -CH= et B₂ représente un atome d'azote.

Parmi les aminoquinoléines de formule (IIa), on peut encore citer les aminoquinoléines de formules (IIb) et (IIc), dans lesquelles R, R' et R₄ sont tels que définis ci-dessus :

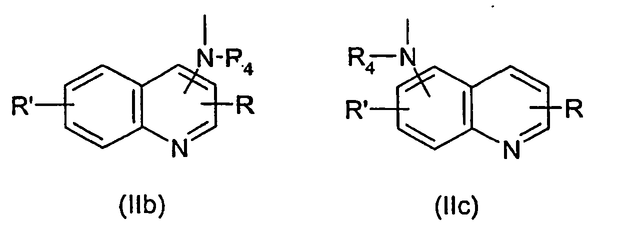

Dans les composés de formule (I) selon l'invention, A peut encore représenter un groupe de formule (IV):

R₅-CHOH- (IV)

dans laquelle R₅ représente un radical aryle (par exemple un 9-phénanthrényle) ou un résidu hétérocyclique azoté (par exemple une 4-quinoléinyle) éventuellement substitué par un ou plusieurs (par exemple de 1 à 3) groupes R tels que définis précédemment Dans les composés de formule (I) selon l'invention, A peut encore représenter un résidu phénol-2(aminométhyle) de formule (V), un résidu biguanide choisi parmi les dérivés de proguanil de formule (VI), un dérivé de cycloguanil de formule (VII), un résidu de pyrimidine, et plus particulièrement de pyriméthamine de formule (VIII) ou (IX), ou un résidu acridine de formule (X), formules dans lesquelles R, R', Rₐ et R_{b} sont tels que définis ci-dessus :

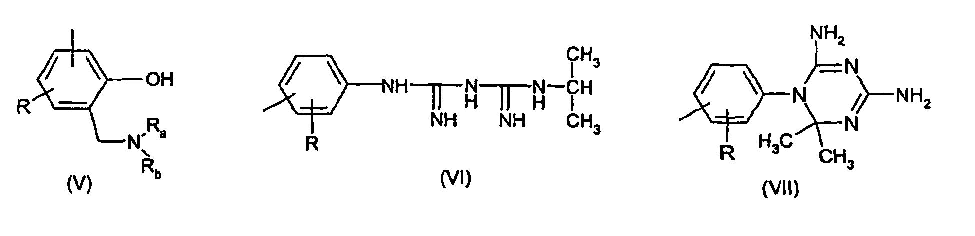

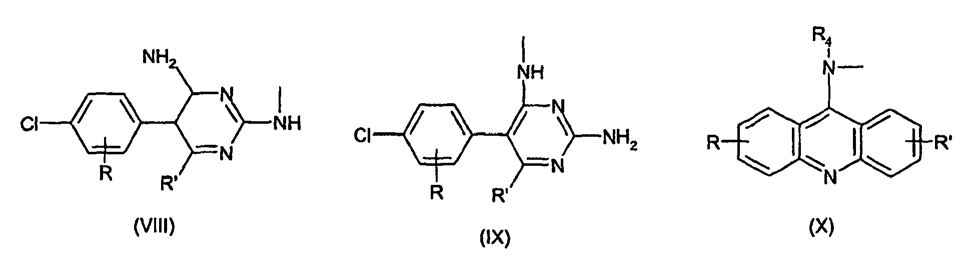

Ainsi, selon la nature du résidu A, on peut obtenir différents sous-groupes de composés de formule (I).

Par exemple, quand A représente un hétérocycle azoté de formule (II) telle que définie précédemment, alors on obtient des composés selon l'invention répondant à la formule (1 bis) :

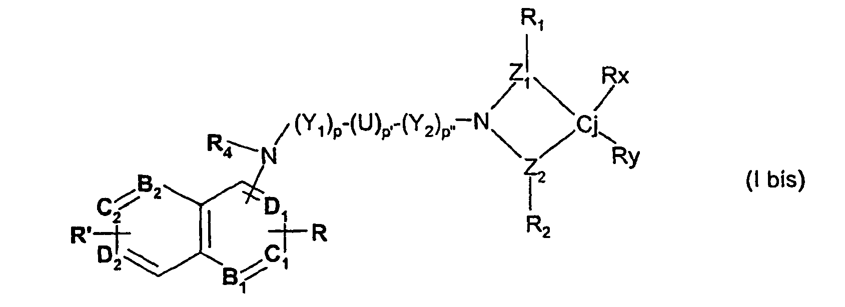

En particulier, quand A représente un hétérocycle azoté de type aminoquinoléine, selon la formule (IIa) telle que définie précédemment, alors on obtient des composés selon l'invention répondant à la formule (I ter) :

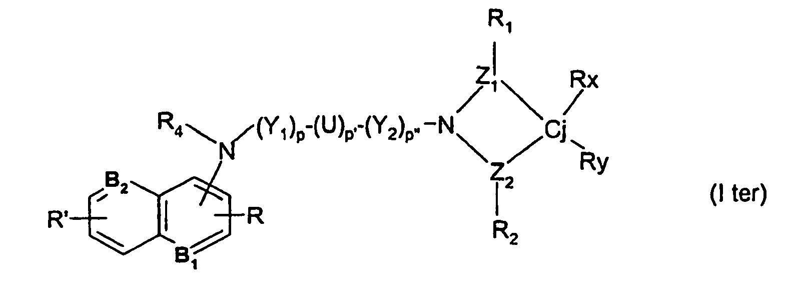

Parmi les molécules duales de formule (I) conformes à l'invention, on peut plus spécialement citer celles dans lesquelles Rₓ et R_{y} forment ensemble un peroxyde cyclique comprenant 5 ou 6 chaînons et comportant 1 atome d'oxygène supplémentaire dans la structure cyclique (soit au total 3 atomes d'oxygène dans le cycle), Cj étant l'un des sommets de ce peroxyde cyclique, ledit peroxyde cyclique étant substitué par 1 à 4 groupes R₃, identiques ou différents, tels que définis ci-avant.

De tels cycles peroxydiques peuvent en particulier consister en :
- des trioxanes de formule (XI) : 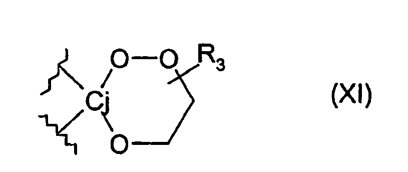 dans laquelle R₃ représente 1 à 4 groupes, identiques ou différents, tels que définis ci-avant, ou
- des trioxolanes de formule (XII) : 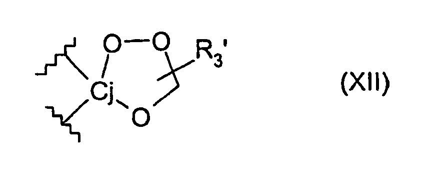 dans laquelle R_{3'} représente 1 ou 2 groupes, identiques ou différents, choisis parmi les atomes et groupes suivants :
   hydrogène, halogène, -OH, -CF₃, -NO₂, aryle (par exemple phényle) ou
   hétéroaryle (par exemple pyridinyle), alkyle ou -O-alkyle (lesdits groupes alkyles comprenant de 1 à 10 atomes de carbone et étant linéaires, ramifiés ou cycliques), ou
   structure mono-, bi- ou tricyclique en C3 à C18 pouvant en outre contenir 1 ou plusieurs (par exemple de 1 à 6) hétéroatomes choisis parmi l'oxygène, l'azote et le soufre, éventuellement substituée par un ou plusieurs groupes choisis parmi les atomes d'halogène, les groupes hydroxyles et les groupes alkyles en C1 à C6, linéaires, ramifiés ou cycliques ; des exemples de telles structures cycliques sont les groupes cycloalkyles (tels que les groupes cyclopropyle, cyclobutyle, cyclopentyle, cyclohexyle, cycloheptyle et cyclooctyle) et le groupe adamantyle (structure tricyclique comprenant 10 atomes de carbone),
   . l'un au moins des groupes R_{3'} étant différent d'un atome d'hydrogène,
   . deux groupes R_{3'} portés par le même atome de carbone du cycle peroxydique pouvant former ensemble une structure mono-, bi- ou tricyclique telle que définie ci-dessus (qui sera donc située en position spiro sur le cycle peroxydique).

Dans les formules (XI) et (XII), le carbone Cj est tel que défini dans la formule (I), c'est-à-dire correspond au carbone de jonction entre le peroxyde cyclique et l'hétérocycle formé entre l'atome d'azote, le carbone Cj et les radicaux Z₁ et Z₂.

Parmi les trioxanes de formule (XI), on peut notamment citer celles répondant à la formule (XIa) : 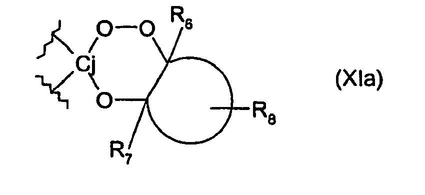 dans laquelle l'arc de cercle représente une structure cyclique à 5 ou 6 chaînons, saturée ou insaturée, Cj est tel que défini ci-avant, et R₈, R₇ et R₈, identiques ou différents les uns des autres, sont choisis parmi les atomes et groupes suivants : hydrogène, halogène, -OH, -CF₃, -NO₂, aryle (par exemple phényle) ou hétéroaryle (par exemple pyridinyle), alkyle ou -O-alkyle (lesdits groupes alkyles comprenant de 1 à 10 atomes de carbone et étant linéaires, ramifiés ou cycliques).

Dans la formule (XIa), R₆, R₇ et R₈ représentent avantageusement des atomes d'hydrogène ou des groupes alkyles en C1-C10, linéaires ou ramifiés.

Parmi les trioxanes de formule (XI), on peut encore citer celles répondant à la formule (XIb), dans laquelle Cj est tel que défini ci-avant et R₆, R₇ et R₈ sont tels que définis en rapport avec la formule (XIa) :

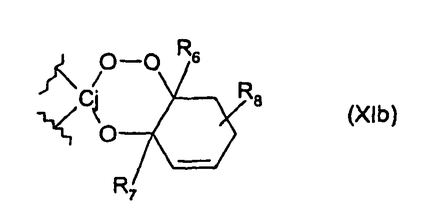

Parmi les trioxanes de formule (XI), on peut encore citer celles répondant à la formule (XIc) : 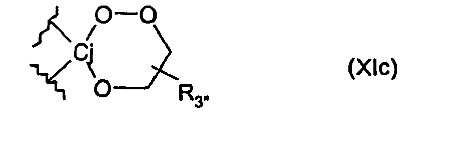 dans laquelle Cj est tel que défini ci-avant et R_{3"} représente 1 à 4 groupes, identiques ou différents les uns des autres, occupant des positions quelconques sur les atomes de carbone du cycle peroxydique et étant choisis parmi les atomes et groupes suivants :
hydrogène, halogène, -OH, -CF₃, -NO₂, aryle (par exemple phényle) ou hétéroaryle (par exemple pyridinyle), alkyle ou -O-alkyle (lesdits groupes alkyles comprenant de 1 à 10 atomes de carbone et étant linéaires, ramifiés ou cycliques), ou
structure mono-, bi- ou tricyclique en C3 à C18 pouvant en outre contenir 1 ou plusieurs (par exemple de 1 à 6) hétéroatomes choisis parmi l'oxygène, l'azote et le soufre, éventuellement substituée par un ou plusieurs groupes choisis parmi les atomes d'halogène, les groupes hydroxyles et les groupes alkyles en C1 à C6, linéaires, ramifiés ou cycliques ; des exemples de telles structures cycliques sont les groupes cycloalkyles (tels que les groupes cyclopropyle, cyclobutyle, cyclopentyle, cyclohexyle, cycloheptyle et cyclooctyle) et le groupe adamantyle (structure tricyclique comprenant 10 atomes de carbone),
   . l'un au moins des groupes R_{3"} étant différent d'un atome d'hydrogène,
   . deux groupes R_{3"} portés par le même atome de carbone du cycle peroxydique pouvant former ensemble une structure mono-, bi- ou tricyclique telle que définie ci-dessus (qui sera donc située en position spiro sur le cycle peroxydique).

Dans la formule (XIc), R_{3"} représente avantageusement 1 à 4 groupes choisis parmi les atomes d'hydrogène et les groupes alkyles en C1-C10, linéaires ou ramifiés, ou deux groupes R_{3"} portés par le même atome de carbone du cycle peroxydique forment ensemble une structure mono-, bi- ou tricyclique telle que définie ci-dessus.

L'invention vise en particulier les molécules duales correspondant au produit de couplage comprenant une aminoquinoléine de formule (IIa) et une structure peroxydique de formule (XI) ; de telles molécules répondent à la formule (XIII), dans laquelle R, R', B₁, B_{2,} Y₁, U, Y₂, p, p', p", Z₁, Z₂, Cⱼ, R₁, R₂, R₃ et R₄ sont tels que définis ci-dessus :

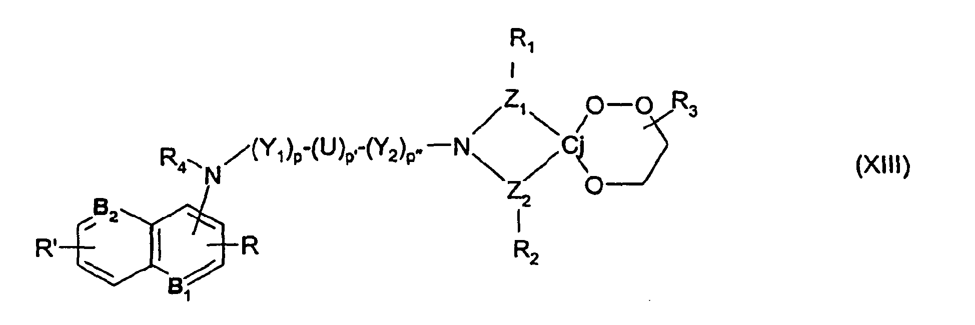

L'invention vise également les molécules duales correspondant au produit de couplage comprenant une aminoquinoléine de formule (IIa) et une structure peroxydique de formule (XIa) ; de telles molécules répondent à la formule (XIIIa), dans laquelle R, R', B_{1,} B₂, Y₁, U, Y_{2,} p, p', p", Z₁, Z₂, Cⱼ, R₁, R₂, R₄, R₆, R₇ et R₈ sont tels que définis ci-dessus :

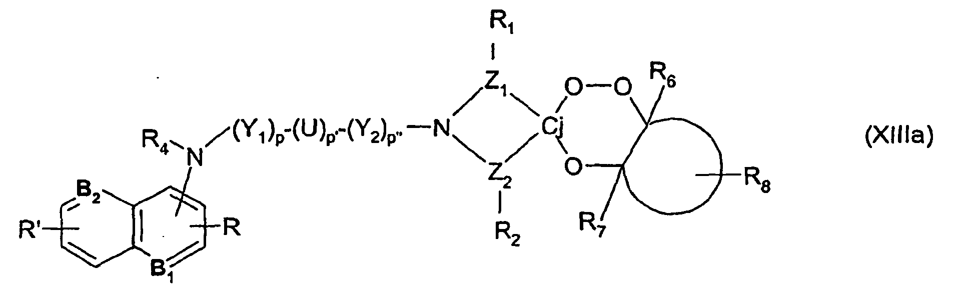

L'invention vise également les molécules duales correspondant au produit de couplage comprenant une aminoquinoléine de formule (IIa) et une structure peroxydique de formule (XIb) ; de telles molécules répondent à la formule (Xlllb), dans laquelle R, R', B₁, B₂, Y₁, U, Y₂, p, p', p", Z₁, 2₂, C_{j,} R₁, R₂, R₄, R₆, R₇ et R₈ sont tels que définis ci-dessus :

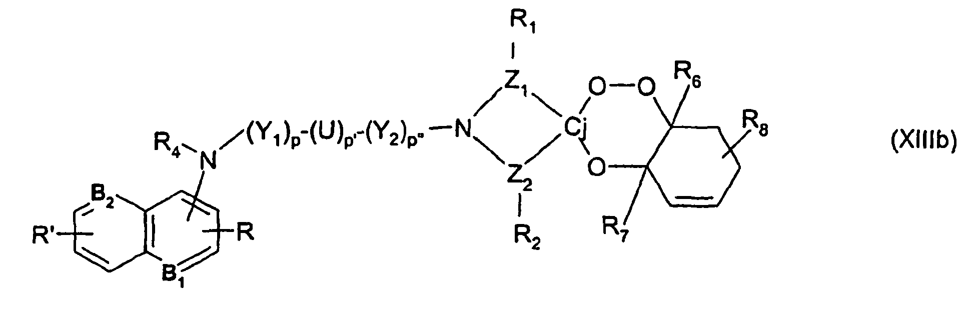

L'invention vise également les molécules duales correspondant au produit de couplage comprenant une aminoquinoléine de formule (IIa) et une structure peroxydique de formule (XIc) ; de telles molécules répondent à la formule (XIIIc), dans laquelle R, R', B₁, B₂, Y₁, U, Y₂, p, p', p", Z₁, Z₂, Cⱼ, R₁, R₂, R_{3"} et R₄ sont tels que définis ci-dessus :

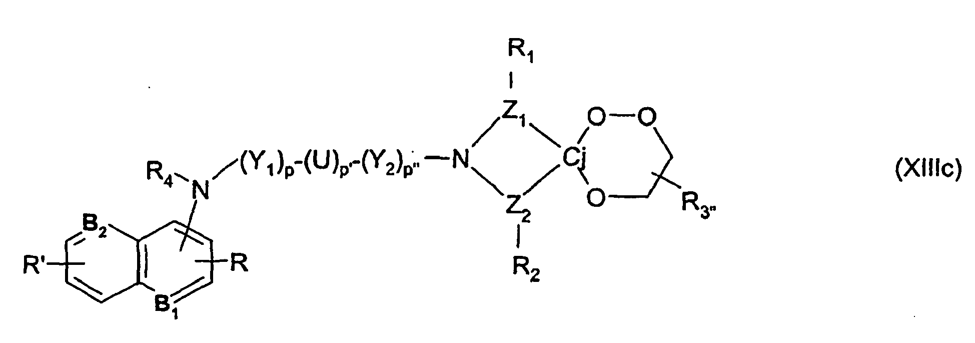

L'invention vise également les molécules duales correspondant au produit de couplage comprenant une aminoquinoléine de formule (IIa) et une structure peroxydique de formule (XII) ; de telles molécules répondent à la formule (XIV), dans laquelle R, R', B₁, B₂, Y₁, U, Y₂, p, p', p", Z₁, Z₂, Cⱼ, R₁, R₂, R_{3'} et R₄ sont tels que définis ci-dessus :

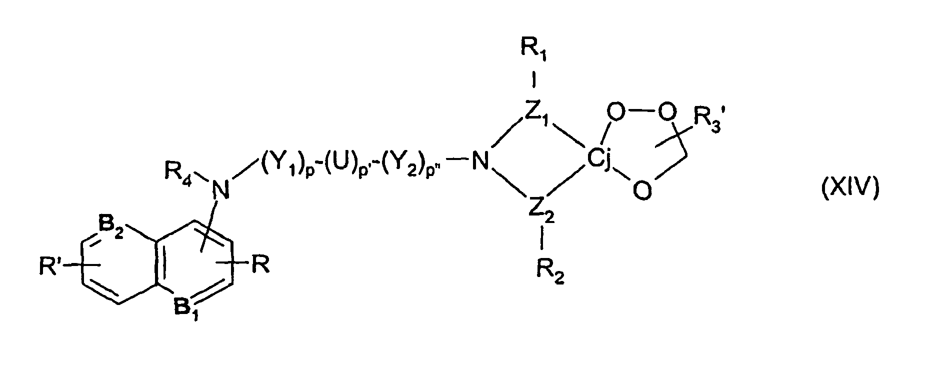

Dans les différentes formules définies ci-avant, R et R' sont avantageusement tels que :
- R et R' représentent un seul substituant, c'est-à-dire soit R, soit R' représente un atome d'hydrogène et l'autre parmi R et R' est choisi parmi les atomes d'halogène et les groupes -CF₃, -N(Rₐ,R_{b}) et -O-alkyle, où le groupe alkyle est en C1 à C5, linéaire, ramifié ou cyclique, Rₐ et R_{b} étant tels que définis ci-avant, ou
- R et R' représentent chacun un groupe choisi parmi les atomes d'halogène et les groupes -CF₃, -N(Rₑ,R_{b}) et -O-alkyle, où le groupe alkyle est en C1 à C5, linéaire, ramifié ou cyclique, Rₐ et R_{b} étant tels que définis ci-avant.

Quant au groupement de formule -(Y₁)_{P}-(U)_{P}-(Y₂)_{p"}, présent dans les molécules de formule (I) en tant que bras de couplage entre le résidu A et le peroxyde cyclique formé par Rₓ et R_{y}, il est avantageusement choisi de manière à moduler l'hydrosolubilité de la molécule, afin de lui conférer une activité optimale.

A cet égard, on peut notamment citer les molécules de formule (I) selon l'invention dans lesquelles p, p' et p" sont tels que définis ci-avant et :
- Y₁ et Y₂, identiques ou différents, représentent une chaîne alkylène en C1 à C6, linéaire, ramifiée ou cyclique, comportant éventuellement un ou plusieurs radicaux amine, amide, carbonyle ou éther, cette chaîne alkylène étant éventuellement substituée par un ou plusieurs groupes choisis parmi les atomes d'halogène, les groupes hydroxyles, les groupes acétals et les radicaux alkyles en C1 à C5, linéaires, ramifiés ou cycliques,
- U représente un radical amine, amide, carbonyle, carboxyle ou éther.

On peut également citer les molécules de formule (I) selon l'invention dans lesquelles :
- soit p' = p" = 0, p = 1 et Y₁ représente une chaîne alkylène en C1 à C6, linéaire, ramifiée ou cyclique (avantageusement linéaire ou ramifiée), éventuellement substituée comme décrit ci-dessus,
- soit p = p' = 1, p" = 0, Y₁ représente une chaîne alkylène en C1 à C6, linéaire, ramifiée ou cyclique (avantageusement linéaire ou ramifiée), éventuellement substituée comme décrit ci-dessus, et U représente un radical amine, amide, carbonyle, carboxyle ou éther,
- soit p = p' = p" = 1, Y₁ et Y₂ représentent des chaînes alkylènes en C1 à C6, linéaires, ramifiées ou cycliques (avantageusement linéaires ou ramifiées), éventuellement substituées comme décrit ci-dessus, et U représente un radical amine, amide, carbonyle, carboxyle ou éther.

D'autres molécules de formule (I) selon l'invention sont telles que Z₁ et Z₂, identiques ou différents, représentent chacun un radical alkylène en C1 à C4, linéaire ou ramifié. On peut notamment citer les molécules de formule (I) selon l'invention dans lesquelles Z₁ et Z₂ représentent chacun un radical éthylène, de façon à former un cycle pipéridinyle avec l'atome d'azote et le carbone de jonction Cj.

Les différentes définitions données ci-dessous pour chacun des groupements constitutifs de la formule (I) peuvent être pris en combinaison les uns avec les autres, de façon à former différents sous-groupes de molécules duales selon l'invention. On peut en particulier citer les molécules duales répondant aux formules (XV) ou (XVI), dans lesquelles R, R', B₁, B₂, Y_{1,} U, Y₂, p, p', p", R₁, R₂, R₃, R_{3'} et R₄ sont tels que définis ci-dessus :

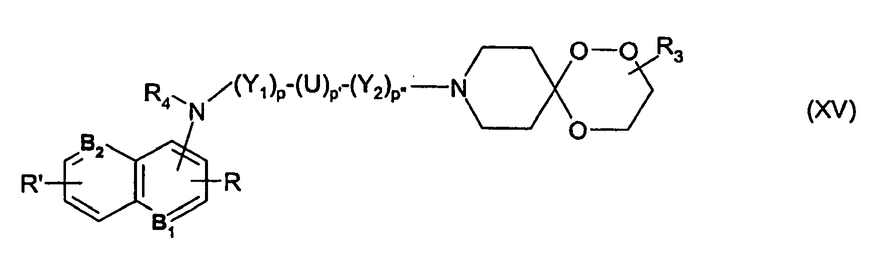

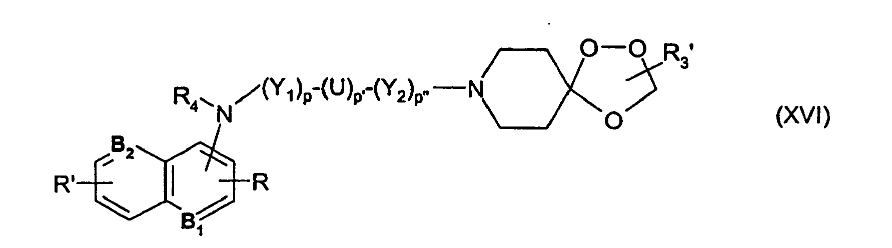

Un autre sous-groupe de composés de formule (I) conformes à l'invention peut être tel que :
- A représente un résidu de molécule à activité antipaludique et/ou un résidu facilitant la biodisponibilité, ce dernier possédant un ou plusieurs hétéroatomes choisis parmi N, O et S dans une molécule cyclique en C6-C18 saturée ou insaturée ou dans une chaîne en C1-C18 linéaire substituée ou non, tel qu'un résidu guanidinium, morpholino, peptidique ou de polyamine,
- p, p', p" représentent indépendamment les uns des autres 0 ou 1, l'un au moins de p, ou de p" étant égal à 1,
- Y₁ et Y₂, identiques ou différents, représentent une chaîne alkylène en C1 à C5, linéaire ou ramifiée, contenant le cas échéant un ou plusieurs radicaux amine, amide, sulfonamide, carboxyle, thiocarboxyle, carbonyle, éther, thioéther ou thiocarbonyle, cette chaîne alkylène en C1 à C5 étant le cas échéant substituée par un radical alkyle linéaire ou ramifié en C1 à C5,
- U est une fonction amine, amide, thioamide, sulfonyle, carbonyle, thiocarbonyle, carboxyle, thiocarboxyle (C(O)=S), éther ou thioéther, sulfonate (SO₃)
- Z₁ et Z₂, identiques ou différents, représentent un radical alkylène en C1 à C4 linéaire, ramifié ou cyclique, saturé ou insaturé, l'un de Z₁ ou Z₂ pouvant être absent, ou l'ensemble Z₁ + Z₂ représente une structure polycyclique incluant N et le carbone de jonction Cj,
- R₁ et R₂ identiques ou différents représentent un atome d'hydrogène ou un groupe fonctionnel capable d'augmenter l'hydrosolubilité de la molécule duale, avantageusement choisi parmi -COOH, -OH, -N(Rₐ, R_{b}) avec Rₐ et R_{b}, identiques ou différents, représentant un atome d'hydrogène ou un radical alkyle en C1 à C5, linéaire, ramifié ou cyclique,
- Rₓ et R_{y} forment ensemble un peroxyde cyclique comprenant de 4 à 8 chaînons et comportant 1 ou 2 atomes d'oxygène supplémentaires dans la structure cyclique (soit au total de 3 à 4 atomes d'oxygène dans le cycle), Cj étant l'un des sommets de ce peroxyde cyclique, ledit peroxyde cyclique étant substitué par 1 à 4 groupes R₃, identiques ou différents les uns des autres, occupant des positions quelconques sur les atomes de carbone du cycle peroxydique et étant choisis parmi les atomes et groupes suivants : hydrogène, halogène, -OH, -CF₃, -NO₂, aryle ou hétéroaryle (par exemple un phényle ou une pyridine), alkyle ou -O-alkyle, lesdits groupes alkyles comprenant de 1 à 6 atomes de carbone et étant linéaires, ramifiés ou cycliques, structure mono-, bi- ou tricyclique en C3 à C18 pouvant en outre contenir 1 ou plusieurs (par exemple de 1 à 6) hétéroatomes choisis parmi l'oxygène, l'azote et le soufre, éventuellement substituée par un groupe alkyle en C1 à C6, linéaire ramifié ou cyclique ; des exemples de telles structures cycliques sont les groupes cyclohexyle ou adamantyle (structure tricyclique comprenant 10 atomes de carbone),
   - l'n au moins des groupes R₃ étant différent d'un atome d'hydrogène,
   - deux groupes R₃ adjacents sur le cycle peroxydique pouvant former ensemble une structure cyclique à 5 ou 6 chaînons, saturée ou insaturée, et éventuellement substituée en positions quelconques par un ou plusieurs (par exemple de 1 à 3) substituants R₃ tels que définis ci-dessus.

L'invention a également pour objet un procédé de préparation des molécules de formule (I) définies ci-dessus. Ce procédé comprend la réaction de dérivés réactifs de A (tels que des dérivés halogénés) et de dérivés peroxydiques comportant les résidus Rₓ et R_{y} de manière à former, entre ces dérivés, un bras de couplage -(Y₁)ₚ-(U)ₚ-(Y₂)ₚ- tel que défini en rapport avec la formule (I).

Diverses voies de synthèse seront aisément accessibles à l'Homme du métier en opérant selon les techniques classiques. Pour la synthèse des dérivés peroxydiques comportant les résidus Rₓ et R_{y}, on se référera par exemple à l'ouvrage de S. Pataï : "The Chemistry of peroxides", John Wiley and Sons Ltd, 1983.

Ainsi, pour préparer des molécules duales de formule (I) renfermant, comme résidu A, une aminoquinoléine de formule (IIa) telle que définie précédemment, on peut fait réagir un composé de formule (XVII) : 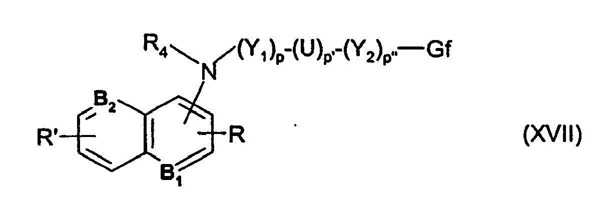 dans laquelle R, R', B₁, B₂, R₄, Y₁, Y₂, U, p, p' et p" sont tels que définis dans les formules (I) et (IIa) ci-dessus et Gf représente un groupe fonctionnel (avantageusement, un groupe hydroxyle ou un groupe partant, tel qu'un atome d'halogène), avec un composé de formule (XVIII), dans laquelle Z₁, Z₂, R₁, R₂, Cj, Rₓ et R_{y} sont tels que définis dans la formule (I) :

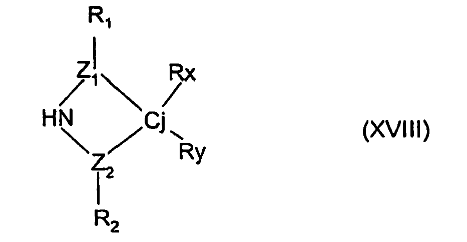

On obtient ainsi les composés selon l'invention répondant à la formule (I ter), telle que définie précédemment.

Alternativement, pour l'obtention des composés de formule (I) dans lesquels p = 1 et renfermant, comme résidu A, une aminoquinoléine de formule (IIa), on peut faire réagir un composé de formule (XIX) : 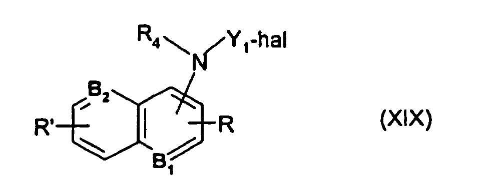 dans laquelle R, R', B_{1,} B₂, R₄ et Y₁ sont tels que définis dans les formules (I) et (IIa) ci-dessus, et « hal » représente un atome d'halogène, avec un composé de formule (XX), dans laquelle U, Y₂, p', p", Z₁, Z₂, R₁, R₂, Cj, Rₓ et R_{y} sont tels que définis dans la formule (I) (avantageusement, p' = 1 et U = -NH₂) :

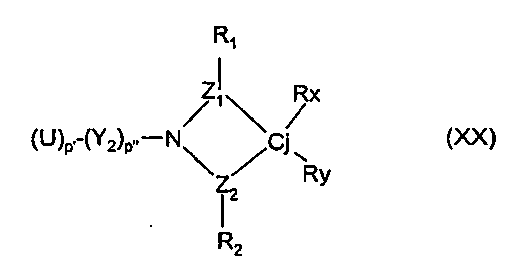

Pour l'obtention des composés de formule (I) dans lesquels p = p' = 1, p" = 0 et U représente un radical amide de formule -NH-CO-, une alternative peut consister à faire réagir un composé de formule (XVIII) telle que définie ci-dessus avec du phosgène ou un dérivé du phosgène (tel que le triphosgène), puis avec un composé de formule (XXI), dans laquelle R, R', B₁, B₂, R₄ et Y₁, sont tels que définis dans les formules (I) et (IIa) ci-dessus :

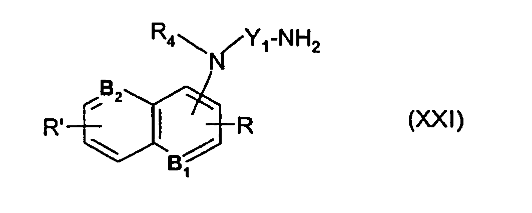

Par ailleurs, pour l'obtention des composés de formule (I) dans lesquels p = p' = 1, p" = 0 et U représente un radical carboxyle de formule -O-CO-, une alternative peut consister à faire réagir un composé de formule (XXII) : 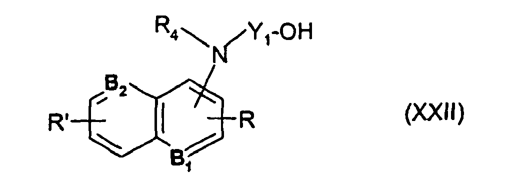 dans laquelle R, R', B₁, B₂, R₄ et Y₁ sont tels que définis dans les formules (I) et (IIa) ci-dessus, avec du phosgène ou un dérivé du phosgène (tel que le triphosgène), puis avec un composé de formule (XVIII) telle que définie ci-dessus.

Les réactions de couplage décrites ci-dessus pour l'obtention des composés de formule (I) conformes à l'invention sont avantageusement réalisées dans un solvant polaire, en présence d'une base organique ou minérale et à température ambiante.

La réaction de couplage est suivie, le cas échéant, d'une réaction avec un acide pharmaceutiquement acceptable, pour obtenir le produit de couplage sous forme de sel. Dans ce but, on procède à la protonation des azotes basiques en ajoutant un acide pharmaceutiquement acceptable organique ou minéral. A titre d'exemples de sels organiques pharmaceutiquement acceptables, on citera les sels des acides citrique, tartrique, fumarique et lactique. A titre d'exemples de sels minéraux pharmaceutiquement acceptables, on citera les sels d'acides chlorhydrique et phosphorique. La réaction peut être réalisée avec 2 équivalents d'acide. Le produit protoné est ensuite récupéré et soumis à une ou plusieurs étapes de purification si nécessaire.

Pour l'obtention des intermédiaires de formule (XIX) définis ci-dessus, on peut procéder comme suit :
a) on fait réagir un composé de formule (XXIII): 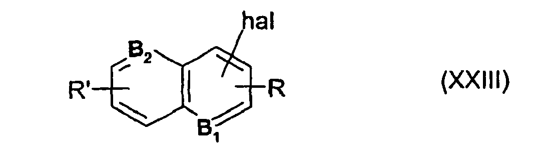 dans laquelle R, R', B₁ et B₂ sont tels que définis ci-dessus dans la formule (IIa) et « hal » représente un atome d'halogène, avec un dérivé aminé de formule R₄-NH-Y₁-U₁, dans laquelle R₄ et Y₁ sont tels que définis précédemment et U₁ représente un groupe -OH, -Cl, -Br, -l, -NH₂, -NHR₄, -COR₄ ou -COOR₄ (avantageusement, U₁ = -OH), ce qui conduit à l'obtention d'un composé de formule (XXIV) : 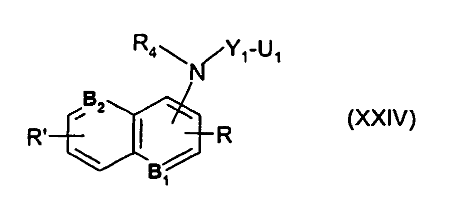
b) on substitue le groupe U₁ par un halogène (« hal »), de façon avantageuse un atome de brome, pour conduire au composé de formule (XIX).

L'étape a) est avantageusement réalisée à une température de 185°C sous agitation. Le dérivé aminé de formule R₄-NH-Y₁-U₁ peut être utilisé à raison de 5 équivalents molaires. Après refroidissement, le produit obtenu peut être précipité par addition de soude à 10% et lavé au méthanol.

L'étape b) est avantageusement réalisée par addition d'un mélange HBr/H₂SO₄ à 160°C pendant 3h30. Après neutralisation, le produit est récupéré par extraction, par exemple au toluène.

L'obtention des composés de formule (I) selon l'invention dans lesquels A est tel que défini précédemment et est différent d'un groupe de formule (IIa) peut être réalisée de façon similaire aux réactions décrites ci-dessus, mais en remplaçant de façon appropriée le composé de départ (XVII), (XIX), (XXI) ou (XXII) par un autre dérivé réactif de A, selon les techniques de chimie organique connues de l'Homme du métier.

L'invention a, en outre, pour objet des composés de formules (XVIII) et (XX) définies ci-dessus. Ces composés peuvent être utiles en tant qu'intermédiaires de synthèse des composés de formule (I) conformes à la présente invention.

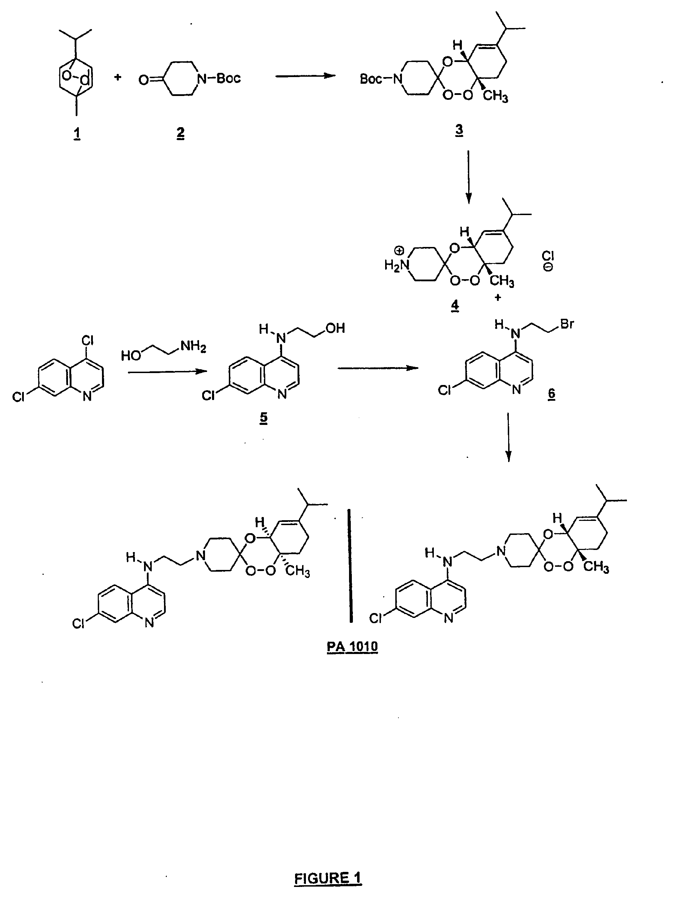

Les exemples qui suivent décrivent la préparation de certains composés conformes à l'invention. Ces exemples ne sont pas limitatifs et ne font qu'illustrer la présente invention. Ces exemples renvoient aux figures 1 à 12, qui décrivent respectivement les synthèses des composés PA 1010, PA 1011, PA 1025 (et ses sels PA 1047 et PA 1042), PA 1128, PA 1102, PA 1035, PA 1020, PA 1021 (et son sel PA 1040), PA 1026, PA 1069, PA 1080 et PA 1097.

### 1 - -Synthèse du PA 1010, mélange racémique (Figure 1) :

7-chloro-*N*-{2-[6-isopropyl-8a-methyl-4a,7,8,8a-tetrahydro-1'*H*-spiro[1,2,4-benzotrioxine-3,4'-piperidin]-1'-yl]ethyl}quinolin-4-amine

### 1-1 : Synthèse du trioxane 3

Dans un ballon sous atmosphère d'argon est mis en solution 0,66 g (3,3 mmoles) de Boc-pipéridone 2 (préparée selon la méthode décrite par J. McGuire et al., J. Med. Chem. 1998, 41, 1409-1416) dans 2 mL de dichlorométhane sec. Le mélange est refroidi à -20°C et 0,46 mL (3,6 mmoles) de BF₃OEt₂, puis 0,5 mL (0,33 mmole) d'une solution d'ascaridole **1,** préparé selon la méthode décrite par Meunier *et al*., dans la demande internationale PCT WO 01/77105, sous forme d'une solution à 0,66 M dans le dichlorométhane, sont ajoutés. A ce mélange réactionnel est ajouté toutes les 15 minutes 42 µL (0,33 mmole) de BF₃OEt₂ suivi de 0,5 mL (0,33 mmole) d'ascaridole **1.** Cette opération d'addition fractionnée est répétée 8 fois de plus. La réaction est ensuite stoppée par addition de 5 mL d'une solution aqueuse saturée en NaHCO₃ et 50 mL de dichlorométhane sont ajoutés. La phase organique est extraite par 100 mL de dichlorométhane, séchée par addition de Na₂SO₄, filtrée et les solvants sont évaporés sous vide. Le résidu est ensuite purifié par colonne de chromatographie (SiO₂ 60 ACC 6-35 µm, éluant : n-hexane/éther/dichlorométhane 70/20/10, v/v/v). La sortie des produits de colonne est contrôlée par CCM (SiO₂ 60 F 254, révélé à l'acide phosphomolybdique) et le trioxane est récupéré. Les phases contenant le trioxane **3** sont réunies et les solvants sont évaporés, conduisant à une huile identifiée comme le trioxane **3** : 0,20 g (Rdt = 16%).

### 1-2 : Synthèse du trioxane 4

0,46 g (1,2 mmoles) de trioxane **3** est solubilisé dans 3 mL d'acétate d'éthyle. A ce mélange est ajouté goutte à goutte, à température ambiante, 0,7 mL d'une solution d'HCl à 3 M dans l'acétate d'éthyle. Le mélange est laissé sous agitation toute une nuit. 50 mL d'éther sont ajoutés et le précipité est filtré. La poudre est lavée par 20 mL d'éther et séchée sous vide pendant 2 h. Une poudre blanche est obtenue, identifiée comme étant le composé **4 :** 0,28 g (Rdt = 70%).

### 1-3 : Synthèse de la 7-chloro 4-(β-hydroxy éthvlamino)quinoléine 5

Un mélange de 4,7-dichloroquinoléine (50,0 g, 252 mmoles) et de 2-aminoéthanol (46,26 g, 757 mmoles) est chauffé sous agitation magnétique à 150°C pendant 15 min, puis à 185°C pendant 30 min. Après retour à température ambiante, le solide est mis en suspension dans 250 mL d'une solution aqueuse de soude à 10 %, p/v. Le précipité obtenu est filtré sur fritté, lavé à l'eau puis porté au reflux dans 125 mL de méthanol pendant 15 min. Après retour à température ambiante, le précipité est filtré sur fritté, lavé avec 3 fois 40 mL de méthanol puis séché sous vide. Le produit **5** est obtenu sous la forme d'une poudre blanche : 52,28 g (Rdt = 93 %).

### 1-4 : Synthèse de la 7-chloro 4-(β-bromoéthylamino)quinoléine 6

L'acide bromhydrique (22,5 mL, 414 mmoles), puis l'acide sulfurique (7,5 mL, 140 mmoles) sont ajoutés goutte à goutte sur la 7-chloro 4-(β-hydroxyéthylamino)quinoléine **5** (15 g, 67 mmoles), le milieu réactionnel étant réfrigéré à l'aide d'un bain d'eau froide. Le milieu réactionnel est ensuite chauffé à 165°C durant 3 h 30, puis versé dans 300 mL d'eau froide. Le pH est ensuite ajusté par ajout de NaHCO₃ (pH 9 environ) puis le milieu est extrait au reflux du toluène (300 mL) pendant 15 min. La phase organique est ensuite recueillie et le produit **6** est obtenu par cristallisation à -18°C durant une nuit, filtration puis séchage sous vide : 10,55 g (Rdt = 55 %).

### 1-5 : Synthèse du PA 1010

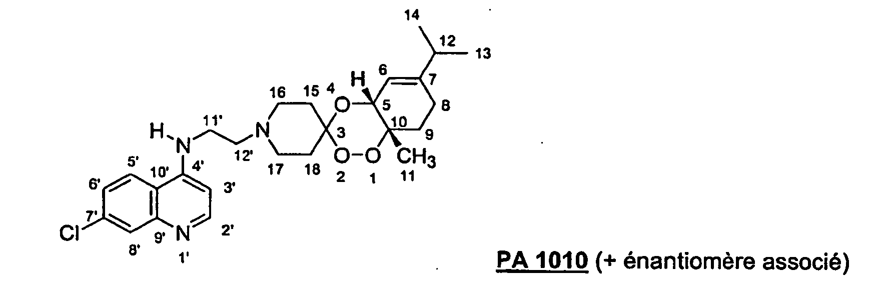

0,14 g (0,46 mmole) de trioxane **4** et 0,13 g (0,46 mmole) du composé **6** sont solubilisés dans 10 mL de DMF. Au mélange obtenu est ajouté 0,14 mL (1 mmole) de triéthylamine et le mélange est laissé sous agitation à température ambiante 96 h. 50 mL d'eau sont alors ajoutés et le composé est extrait avec 3 x 100 mL d'éther. Les solvants de la phase organique sont évaporés et le brut est ensuite purifié par colonne de chromatographie (SiO₂ 60 ACC 6-35 µm, éluant : éther/triéthylamine 99/1, v/v). La sortie des produits de colonne est contrôlée par CCM (SiO₂ 60 F 254, révélé sous UV). Quand le composé **6** n'apparaît plus la phase éluante est modifiée (dichlorométhane /triéthylamine, 80/20, v/v) et le produit de couplage est récupéré. Les solvants des phases contenant le produit de couplage sont évaporés, le brut est solubilisé dans 100 mL d'éther et lavé par 5 mL d'eau. La phase organique est séchée par addition de Na₂SO₄, filtrée et évaporée. L'huile obtenue est solubilisée dans un minimum de dichlorométhane additionné de la même quantité de n-hexane, puis évaporée sous vide pendant 24 h, conduisant à une poudre blanche identifiée comme étant le **PA 1010 :** 0,08 g (Rdt = 35%). PF (point de fusion) : 134-135°C (décomposition). RMN ¹H (300 MHz, 298 K, CDCl₃) δ, ppm : 8,55 (d, *J* = 5,1 Hz, 1H, HC2'), 7,97 (d, *J* = 2,1 Hz, 1H, *H*C8'), 7,66 (d, *J* = 9,0 Hz, 1H, *H*C5'). 7,40 (dd, *J* = 9,0 Hz, *J* = 2,1 Hz, 1H, *H*C6'), 6,38 (d, *J* = 5,4 Hz, 1H, *H*C3'), 6,01 (m, 1H, NH), 5,45 (d, *J* = 4,8 Hz, 1H, *H*C6), 4,06 (s large, 1H, *H*C5), 3,32 (td, *J* = 5,1 Hz, *J* = 5,4 Hz, 2H, *H*C11'), 2,81 (t, *J* = 6,0 Hz, 2H, *H*C12'), 2,73-2,46 (m, 4H + 1H, *H*-piperidone + *H*C9), 2,29-2,22 (m, 1H + 2H, *H*C12 + *H*C8), 2,10-1,76 (m, 4H, H-piperidone), 1,54-1,52 (m, 1H, *H*C9), 1,13 (s large, 3H, *H*C11), 1,06 (d, *J* = 4,8 Hz, 3H, *H*C13, *H*C14), 1,04 (d, *J* = 4,8 Hz, 3H, *H*C13, *H*C14). RMN ¹³C (100 MHz, 293 K, CDCl₃) δ, ppm : 152,58 (C2'), 150,58 (C7), 150,09 (C4'), 149,39 (C9'), 135,26 (C7'), 129,13 (C8'), 125,84 (C6'), 121,60 (C5'), 117,66 (C10'), 116,36 (C6), 101,31 (C3), 99,71 (C3'), 79,18 (C10), 67,60 (C5), 55,25 (C12'), 49,94, 49,62 (C15, C16, C17, C18), 39,36 (C11'), 35,50 (C15, C16, C17, C18), 35,03 (C12), 26,59 (C8), 25,68 (C9), 21,86, 21,56 (C13, C14), 19,76 (C11). SM (DCl/NH₃>0) m/z (%): 472 (MH+, 100)). Analyse élémentaire : pour C₂₆H₃₄N₃O₃Cl•0,5H₂O : % théor. C 64,92, H 7,33, N 8,73 ; % expér. C 64,53, H 7,52, N 8,41.

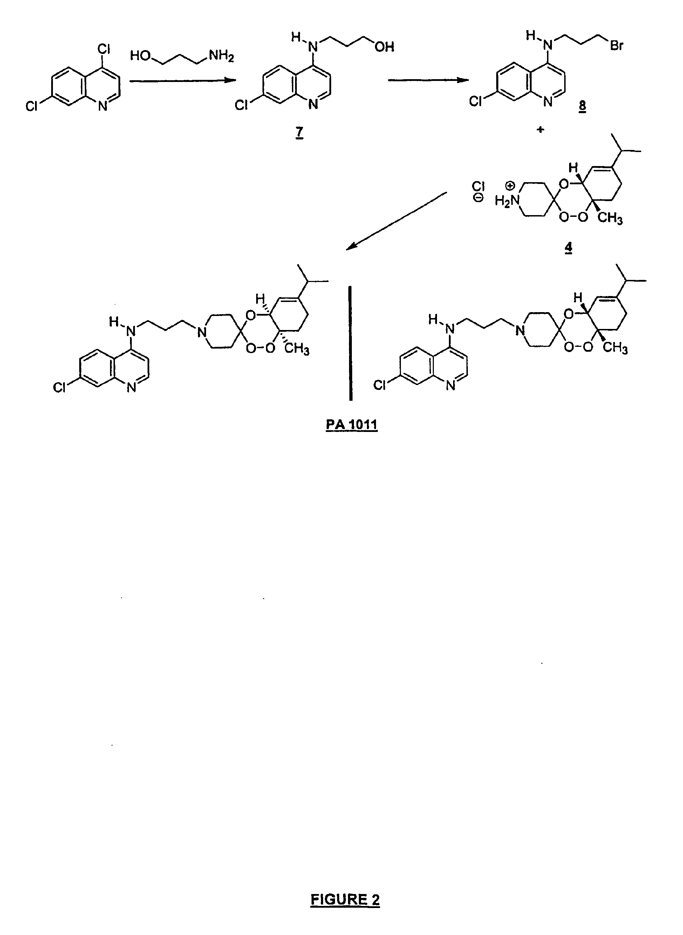

### 2 - Synthèse du PA 1011, mélange racémique (Figure 2)

7-chloro-*N*-{2-[6-isopropyl-8a-methyl-4a,7,8,8a-tetrahydro-1'*H*-spiro[1,2,4-benzotrioxine-3,4'-piperidin]-1'-yl]propyl} quinolin-4-amine

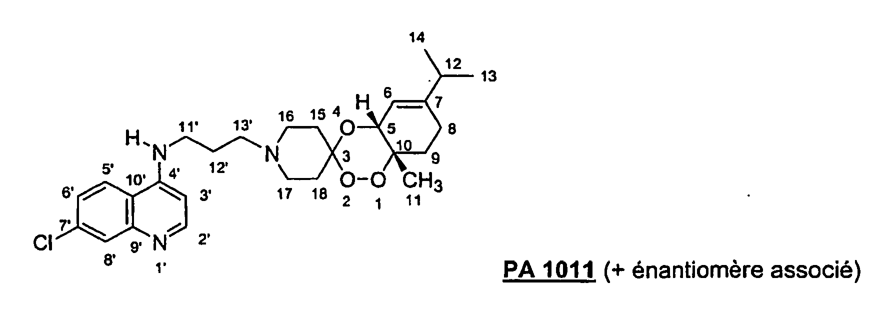

0,10 g (0,33 mmole) de trioxane **4** et 0,10 g (0,33 mmole) de composé **8** (la 4-(3-bromopropylamino)-7-chloroquine, préparée selon la méthode décrite par J. Lhomme et al., J. Med. Chem. 1977, 20, 106-113) sont solubilisés dans 10 mL de DMF. On procède ensuite comme décrit dans l'exemple 1-5 ci-dessus, en utilisant 0,10 mL (0,74 mmole) de triéthylamine. On obtient une poudre blanche identifiée comme étant le **PA 1011 :** 0,05 g (Rdt = 33%). PF : 140-141°C (déc.). RMN ¹H (300MHz, 298K, CDCl₃) δ, ppm : 8,52 (d, *J* = 5,4 Hz, 1H, *H*C2'), 7,95 (d, *J* = 2,1 Hz, 1H, *H*C8'), 7,77 (d, *J* = 9,0 Hz, 1H, *H*C5'), 7,56 (m, 1H, NH), 7,39 (dd, *J* = 9,0 Hz, *J* = 2,1 Hz, 1H, *H*C6'), 6,34 (d, *J* = 5,4 Hz, 1H, *H*C3'), 5,50 (d, *J* = 4,8 Hz, 1H, *H*C6), 4,08 (s large, 1H, *H*C5). 3,40 (td, *J* = 4,8 Hz, *J* = 4,8 Hz, 2H, *H*C11'), 2,74 (m, 2H, *H*C12'), 2,66 (t, *J* = 5,4 Hz, 2H, *H*C13'), 2,34-1,71 (m, 8H + 2H + 1H, *H*-plperidone + HC8 + *H*C9), 1,58-1,54 (m, 1H, *H*C9), 1,20- 1,09 (s large, 3H, *H*C11), 1,08 (d, *J* = 6,6 Hz, 3H, *H*C13, *H*C14), 1,06 (d, *J* = 6,6 Hz, 3H, *H*C13_{,} *H*C14). SM (DCl/NH₃>0) m/z (%): 486 (MH+, 100)). Analyse élémentaire : pour C₂₇H₃₆N₃O₃Cl•0,5H₂O : % théor. C 65,50, H 7,53, N 8,48 ; % expér. C 65,78, H 7,36, N 8,12.

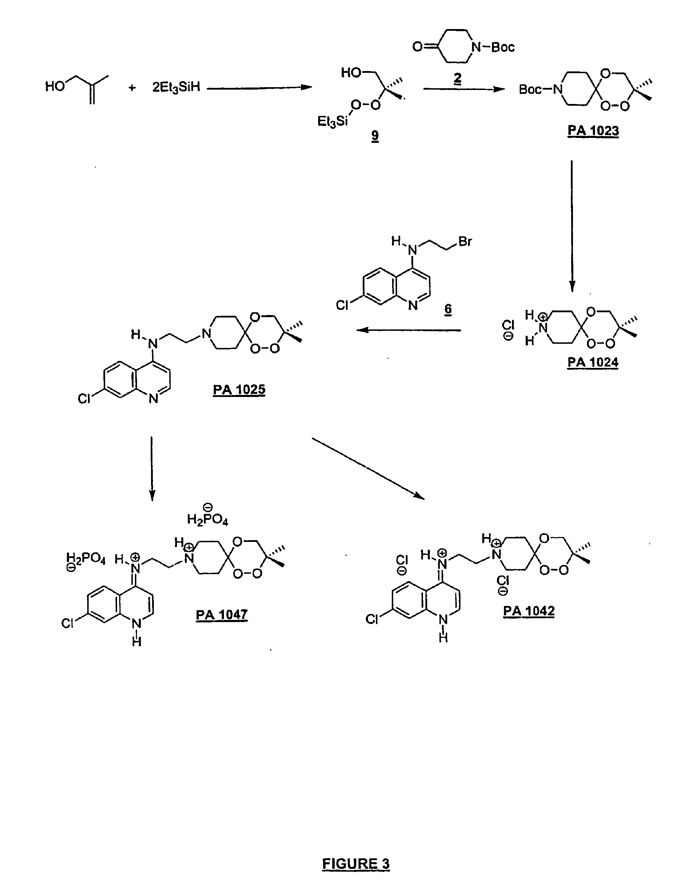

### 3 - Synthèse du PA 1025, molécule achirale, et ses sels PA 1047 et PA 1042 Figure 3)

### 7-chloro-N-[1,2,5-trioxa-9-azaspiro[5.5]undéc-9-yl)ethyl]quinolin-4-amine

### 3-1 : Synthèse du 2-méthyl-2-[(triéthylsilyl)dioxy]-propanol 9

On procède selon la méthode décrite par P.M. O'Neill et al. (Tetrahedron Letters, 42, 2001, 4569-4571).

### 3-2 : Synthèse du PA 1023

10,72 g (48 mmoles) de 2-méthyl-2-[(triéthylsilyl)dioxy]-propanol **9** et 29,05 g (145 mmoles) de N-(butoxycarbonyl)-4-piperidone **2** sont solubilisés dans 250 mL de chloroforme. 6,47 g (34 mmoles) d'acide paratoluènesulfonique sont ajoutés à température ambiante sous argon, et le mélange est laissé sous agitation pendant 30 minutes. Le milieu réactionnel est alors directement purifié par chromatographie (SiO₂ 60 ACC 70-200 µm, éluant : éther/pentane , 20/80, v/v). La sortie des produits de colonne est contrôlée par CCM (SiO₂ 60 F 254, révélé par l'acide sulfurique). Les solvants des phases contenant le produit sont évaporés conduisant à un solide blanc identifié comme étant le **PA 1023 :** 4,03 g (Rdt = 30%). PF : 69-70°C.

### 3-3 : Synthèse du PA 1024

0,27 g (0,94 mmole) de **PA 1023** est solubilisé dans 3 mL d'acétate d'éthyle. A ce mélange est ajouté goutte à goutte, à température ambiante, 0,5 mL d'une solution d'HCl 3 M dans l'acétate d'éthyle. Le mélange est laissé sous agitation pendant 1 h. Les solvants sont ensuite évaporés- au 4/5^{ième} sous vide et 30 mL d'éther sont ajoutés. Le précipité apparu est filtré, lavé par 20 mL d'éther et séché sous vide pendant 2 h. Une poudre blanche est obtenue, identifiée comme étant le **PA 1024** : 0,12 g (Rdt = 56%). PF : 155°C (déc.).

### 3-4 : Synthèse du PA 1025

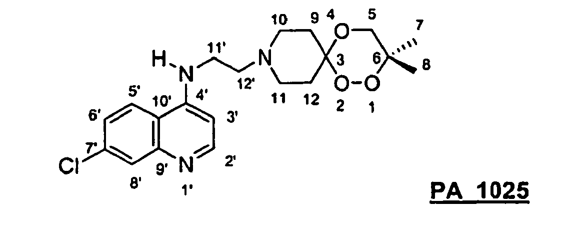

0,47 g (2,11 mmoles) de **PA 1024** et 0,60 g (2,11 mmoles) de composé **6** sont solubilisés dans 25 mL de DMF. Au mélange obtenu est ajouté 0,65 mL (4,6 mmoles) de triéthylamine et le mélange est laissé sous agitation à température ambiante pendant 72 h. Le DMF est évaporé sous vide et le résidu est purifié par colonne de chromatographie (SiO₂ 60 ACC 70-200 µm, éluant : éther/méthanol/triéthylamine, 80/19/1, v/v/v). La sortie des produits de colonne est contrôlée par CCM (SiO₂ 60 F 254). Les solvants des phases contenant le produit de couplage sont évaporés, le brut est solubilisé dans 300 mL d'éther et lavé par 100 mL d'eau. La phase organique est séchée par addition de Na₂SO₄, filtrée et évaporée sous vide pendant 24 h, conduisant à une poudre blanche identifiée comme étant le **PA 1025** : 0,25 g (Rdt = 30%). PF : 156°C (déc.). RMN ¹H (400 MHz, 233 K, CDCl₃) δ, ppm **:** 8,54 (d, *J* = 5,2 Hz, 1H, *H*C2'), 7,93 (d, *J* = 2,0 Hz, 1H, *H*C8'), 7,68 (d, *J* = 8,8 Hz, 1H, *H*C5'), 7,40 (d, *J* = 7,6 Hz, *H*C6'), 6,38 (d, *J* = 5,6 Hz, 1H, *H*C3'), 6,15 (s, 1H, *H*N), 3,91 (s large, 1H, *H*C5), 3,49 (d, *J* = 11,6 Hz, 1H, *H*C5), 3,31 (s, 2H, *H*C11'), 3,12-1,60 (m, 8H, *H*-pipéridone), 2,82 (s, 2H, *H*C12'), 1,55 (s, 3H, *H*C7, *H*C8), 1,14 (s, 3H, *H*C7, *H*C8). RMN ¹³C (100 MHz, 233 K, CDCl₃) δ, ppm : 152,70 (C2'), 150,05 (C4'), 149,15 (C9'), 135,26 (C7'), 128,94 (C8'), 125,91 (C6'), 121,91 (C5'), 117,54 (C10'), 100,82 (C3), 99,78 (C3'), 77,87 (C6), 67,15 (C5), 55,15 (C12'), 49,83 et 49,51 (C9, C12), 35,23 (C11'), 28,80 (C10, C11), 23,36, 22,33 (C7, C8). SM (DCl/NH₃>0) m/z (%): 392 (MH+, 100). Analyse élémentaire : pour C₂₀H₂₆N₃O₃Cl : % théor. C 61,30, H 6,69, N 10,72 ; % expér. C 61,31, H 6,59, N 10,44.

### 3-5 : Synthèse du PA 1042

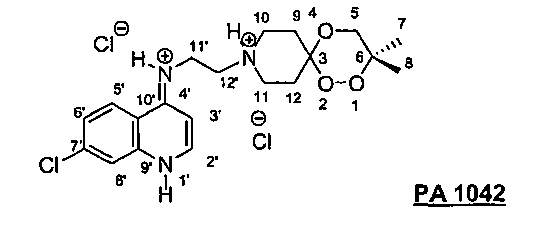

0,18 mL d'acide chlorhydrique 12 M est dilué dans 2 mL d'acétone. Cette solution

est versée goutte à goutte à une solution de 0,40 g (1,01 mmoles) de **PA 1025** dans 18 mL d'acétone. La suspension obtenue est versée dans 200 mL d'éther sous agitation. Le mélange est filtré, le précipité lavé avec 100 mL d'éther puis séché sous vide conduisant à une poudre blanche identifiée comme étant le **PA 1042** : 0,44 g (Rdt = 95%). PF : 174°C (déc.). RMN ¹H (300 MHz, 298 K, DMSOd₆) δ, ppm : 14,61 (s, 1H, *H*N), 11,41 (s, 1H, *H*N), 9,76 (s, 1H, *H*N), 8,82 (d, *J* = 9,0 Hz, *H*C5'), 8,66 (d, *J* = 6,9 Hz, *H*C2'), 8,13 (s, 1H, *H*C8'), 7,79 (dd, *J* = 9,0 Hz , *J* = 1,8 Hz, 1H, *H*C6'), 7,05 (d, *J* = 7,2 Hz, *H*C3'), 4,02 (m, 2H, *H*C11'), 3,80-3,40 (m, 2H + 2H + 2H, *H*C5 + *H*C12' + *H*-pipéridone), 3,07 (m, 3H, *H*-pipéridone), 2,08 (m, 3H, *H*-pipéridone), 1,37-1,06 (m, 6H, *H*C7, *H*C8). SM (ES/MS>0, MeOH) m/z (%) : 392,25 (MH+, 100). Analyse élémentaire : pour C₂₀H₂₆N₃O₃Cl•2HCl•1,4H₂O : % théor. C 49,02, H 6,33, N 8,57 ; % expér. C 48,73, H 5,93, N 8,50.

### 3-6 : Synthèse du PA 1047

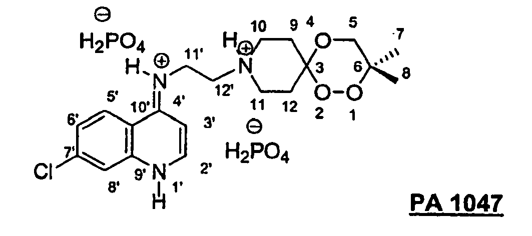

On procède selon le mode opératoire décrit en 3-5 ci-dessus, à l'aide de 0,14 mL d'acide phosphorique 15M dilué dans 2 mL d'acétone. On obtient 0,54 g de **PA 1047** (Rdt = 90%). PF : 173°C (déc.). RMN ¹H (300 MHz, 298 K, DMSOd₆) δ, ppm : 8,98 (m, 6H, HN + *H*₂PO₄), 8,44 (m, 2H, *H*C2'+ *H*C5'), 7,87 (s, 1H, *H*C8'), 7,58 (d , *J* = 7,2 Hz, 1H, *H*C6'), 6,72 (d, *J* = 7,2 Hz, *H*C3'), 3,70-3,50 (m, 4H, *H*C11' + *H*C5), 2,89 (s, 2H, *H*C12'), 2,72 (s, 4H, *H*-pipéridone), 2,26-1,76 (m, 4H, *H-*pipéridone), 1,36-0.93 (m, 6H, *H*C7, *H*C8). SM (DCl/NH₃>0) m/z (%) : 392 (MH+, 9).

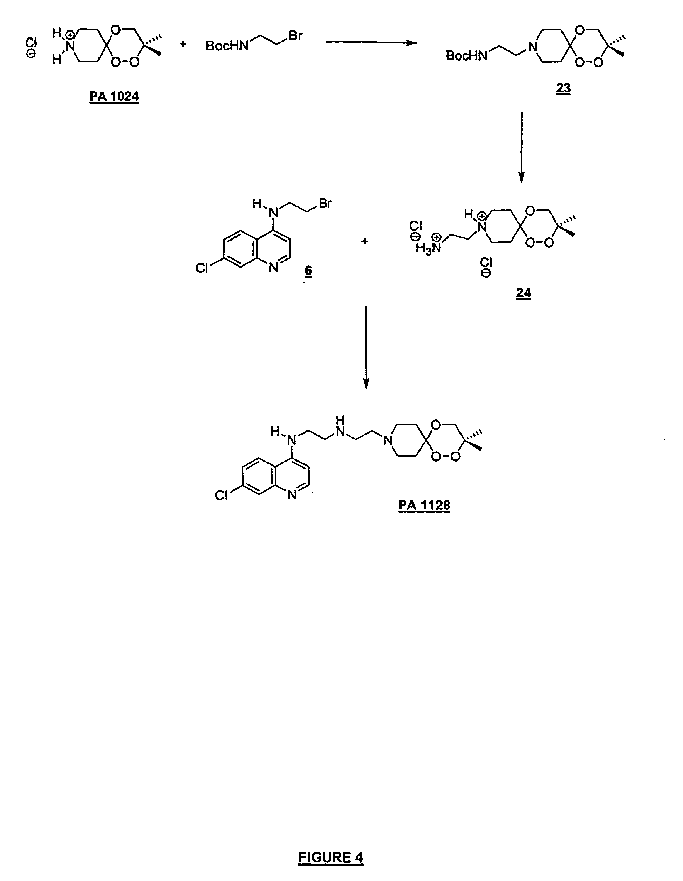

### 4 - Synthèse du PA 1128, molécule achirale (Figure 4)

*N*-(7-chloroquinolin-4-yl)-*N'*-[2-(3,3-dimethyl-1,2,5-trioxa-9-azaspiro[5.5]undec-9-yl)ethyl]ethane-1,2-diamine

### 4-1 : Synthèse du [2-(3,3-diméthyl-1,2,5-trioxa-9-aza-spiro[5.5]undec-9-yl)-éthyl]-carbamic acid tert-butyl ester 23

0,50 g (2,23 mmoles) de **PA 1024** et 0,50 g (2,23 mmoles) de bromure de 2-(boc-amino)éthyle sont solubilisés dans 40 mL de DMF. Au mélange obtenu est ajouté 0,69 mL (4,91 mmoles) de triéthylamine et le mélange est laissé sous agitation à température ambiante 72 h. Le DMF est évaporé et le résidu est ensuite purifié par colonne de chromatographie (SiO₂ 60ACC 70-200 µm, éluant : acétate d'éthyle, MeOH, triéthylamine (90/9/1, v/v/v)). Les solvants des phases contenant le produit de couplage sont évaporés, puis le résidu repris dans 50 mL d'éther et lavé par 50 mL d'eau. La phase organique est séchée par addition de Na₂SO₄, filtrée et évaporée sous vide, conduisant à une poudre blanche identifiée comme étant le produit **23** : 0,21 g (Rdt = 28%).

### 4-2 : Synthèse du composé 24

0,21 g (0,63 mmole) de composé **23** est solubilisé dans 4 mL d'acétate d'éthyle et 3 mL de méthanol. A ce mélange est ajouté goutte à goutte à température ambiante 5 mL d'une solution d'HCl à 3 M dans l'acétate d'éthyle. Le mélange est laissé sous agitation 24 h. Les solvants sont ensuite évaporés au 4/5^{ième} sous vide et 30 mL d'éther sont ajoutés. Le précipité apparu est filtré, lavé par 20 mL d'éther et séché sous vide 2 h. Une poudre blanche est obtenue, identifiée comme étant le composé 24 **:** 0,17 g (Rdt = quantitatif).

### 4-3 : Synthèse du PA 1128

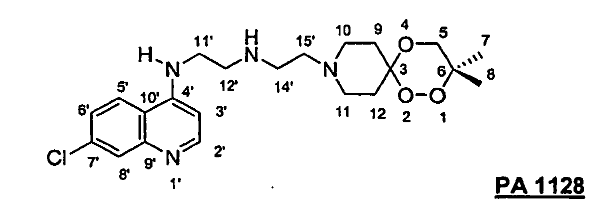

0,10 g (0,31 mmole) de **24** et 0,89 g (0,31 mmole) de **6** sont solubilisés dans 5 mL de DMF. Au mélange obtenu est ajouté 0,14 mL (1,02 mmoles) de triéthylamine et le mélange est laissé sous agitation à température ambiante 72 h. Le DMF est évaporé et le résidu est ensuite purifié par colonne de chromatographie (SiO₂ 60ACC 70-200 µm, éluant : CH₂Cl₂, MeOH, triéthylamine (24,9/75/0,1, v/v/v)). Les solvants des phases contenant le produit de couplage sont évaporés, puis le résidu repris dans 50 mL d'éther et lavé par 50 mL d'eau. La phase organique est séchée par addition de Na₂SO₄, filtrée et évaporée sous vide conduisant à une huile identifiée comme étant le **PA 1128** : 0,004 g (Rdt = 3%). RMN ¹H (300 MHz, 298 K, CDCl₃) δ, ppm : 8,53 (d, *J* = 5,4 Hz, 1H, *H*C2'), 7,96 (d, *J* = 2,1 Hz, 1H, *H*C8'), 7,78 (d, *J* = 9,0 Hz, 1H, *H*C5'), 7,38 (dd, *J* = 9,0 Hz, *J* = 2,2 Hz, *H*C6'), 6,39 (d, *J* = 5,4 Hz, 1H, *H*C3'), 6,01 (s, 1H, NH), 3,75 (s large, 1H; *H*C5), 3,45 (m, 1H + 2H, *H*C5 +, *H*C11'), 3,07 (t, *J* = 6,3 Hz, *H*C12'), 2,77 (m, 2H, *H*C14'), 2,56 (m, 2H + 2H, *H*C15' + *H*-pipéridone), 2,45 (m, 2H, *H*-pipéridone), 1,79 (m, 4H, *H*-pipéridone), 1,50 (s, 3H, *H*C7, *H*C8), 1,12 (s, 3H, *H*C7, *H*C8). SM (DCl/NH₃>0) m/z (%) : 435 (MH+, 5).

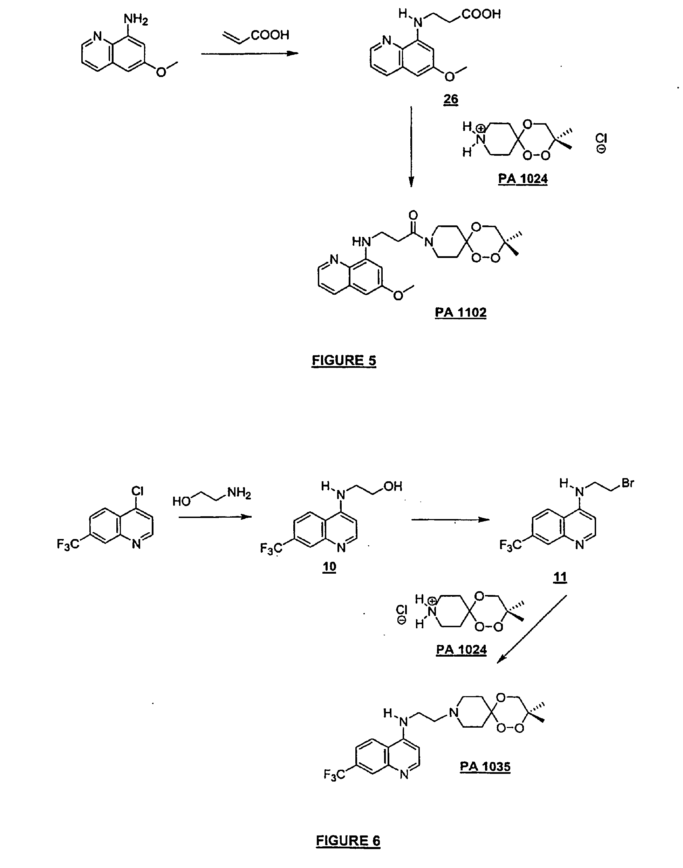

### 5 - Synthèse du PA 1102, molécule achirale (Figure 5)

*N*-[3-(3,3-dimethyl-1,2,5-trioxa-9-azaspiro[5.5]undec-9-yl)-3-oxopropyl]-6-methoxyquinolin-8-amine

### 5-1 : Synthèse du 3-(6-methoxy-quinolin-8-ylamino)-propionic acid 26

Un mélange de 5,0 g (19,56 mmoles) d'hydrobromure de 6-méthoxy 8-aminoquinoléine, 1,48 mL (21,51 mmoles) d'acide acrylique et 5,45 mL (39,12 mmoles) de triéthylamine est dissous dans 25 mL de toluène et porté sous agitation magnétique à 110°C pendant 16 h. 10 mL d'une solution aqueuse de soude 10 % (p/v) sont ensuite ajoutés et le milieu réactionnel est maintenu 20 min à 100°C sous agitation. Après retour à température ambiante, la phase contenant le toluène est éliminée et la phase aqueuse est extraite par 100 mL de chloroforme. La phase aqueuse est ensuite acidifiée par ajout d'acide acétique jusqu'à pH 5-6. Le précipité obtenu est filtré puis lavé à l'eau. Après séchage sous vide, le composé **26** est obtenu sous la forme d'une poudre verte : 1,77 g (Rdt = 37 %).

### 5-2: Synthèse du PA 1102

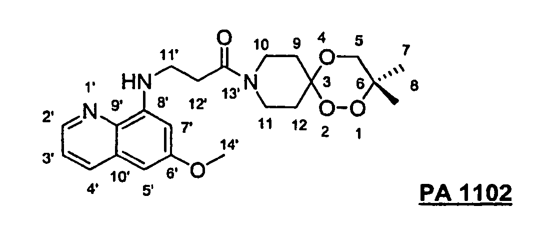

0,50 g (2,23 mmoles) de **PA 1024** et 0,55 g (2,23 mmoles) de composé **26** sont mis en solution dans 20 mL de DMF. Au mélange obtenu sont successivement ajoutés 1,23 mL (11,17 mmoles) de N-méthylmorpholine et 1,16 g (2,23 mmoles) de PYBOP^{®} (benzotriazol-1-yl-oxy-trispyrrolidinophosphonium hexafluorophosphate). Le mélange est laissé sous agitation à température ambiante 24 h. Après ajout de 80 mL de dichlorométhane, la phase organique est lavée avec 300 mL d'une solution saturée de NaHCO₃ puis par deux fois 300 mL d'eau. Les solvants de la phase organique sont évaporés sous vide conduisant à une huile. Le résidu est ensuite purifié par colonne de chromatographie (SiO₂ 60ACC 70-200 µm, éluant : acétate d'éthyle, triéthylamine (90/10, v/v)). Les phases contenant le produit de couplage sont réunies puis lavées avec 1 L d'eau. La phase organique est séchée par addition de Na₂SO₄, filtrée et évaporée sous vide conduisant à une poudre blanche identifiée comme étant le **PA 1102** : 0,63 g (Rdt = 71%). PF : 138°C (déc.). RMN ¹H (300 MHz, 298 K, CDCl₃) : δ, ppm : 8,55 (d, *J* = 4,2 Hz, 1H, *H*C2'), 7,94 (dd, *J* = 8,1 Hz, *J* = 2,1 Hz, 1H, *H*C4'), 7,32 (dd, *J* = 8,1 Hz, *J* = 4,2 Hz, *H*C3'), 6,42 (m, 1H, *H*N), 6,38 (m, 1H + 1H, HC5' + *H*C7'), 3,91 (s, 3H, *H*C14'), 3,67 (q, *J* = 5,7 Hz, *H*C11'), 3,65 (m, 1H, *H*C5), 3,57-3,35 (m, 1H + 4H, *H*C5 + *H*-pipéridone), 2,77 (t, *J* = 6,6 Hz, , 2H, *H*C12'), 2,34 (m, 1H, *H*-pipéridone), 2,06 (m, 3H, *H*-pipéridone), 1,49 (s, 3H, *H*C7, *H*C8), 1,13 (s, 3H, *H*C7, *H*C8). SM (DCl/NH₃>0) m/z (%) : 416 (MH+, 62). Analyse élémentaire : pour C₂₂H₂₉N₃O₅ : % théor. C 63,60, H 7,04, N 10,11 ; % expér. C 63,63, H 6,76, N 10,11.

### 6 - Synthèse du PA 1035, molécules achirale (Figure 6)

7-trifluorométhyl-N-[1,2,5-trioxa-9-azaspiro[5.5]undéc-9-yl)éthyl]quinolin-4-amine

### 6-1 : Synthèse de la 7-trifluorométhyl-4-(β-hydroxy éthylamino)quinoléine 10

Un mélange de 4-chloro-7-(trifluorométhyl)quinoléine (1,0 g, 4,7 mmoles) et de 2-aminoéthanol (0,86 g, 14,1 mmoles) est chauffé sous agitation magnétique à 150°C pendant 15 min, puis à 185°C pendant 30 min. Après retour à température ambiante, le solide est mis en suspension dans 5 mL d'une solution aqueuse de soude à 10 %, p/v. Le précipité obtenu est filtré sur fritté, lavé à l'eau puis porté au reflux dans 10 mL d'éthanol pendant 15 min. Après retour à température ambiante, de l'eau est rajoutée jusqu'à apparition d'un précipité. Le précipité est filtré sur fritté, lavé avec 10 mL d'eau puis séché sous vide. Le produit **10** est obtenu sous la forme d'une poudre blanche : 0,82 g (Rdt = 68 %). PF : 181-182°C.

### 6-2 : Synthèse de la 7-trifluorométhyl-4-(β-bromo éthlamino)quinoléine 11

L'acide bromhydrique (0,88 mL, 16,2 mmoles), puis l'acide sulfurique (0,29 mL, 5,5 mmoles) sont ajoutés goutte à goutte sur la 7-trifluorométhyl-4-(β-hydroxyéthylamino)quinoléine **10** (0,67 g, 2,6 mmoles), le milieu réactionnel étant réfrigéré à l'aide d'un bain d'eau froide. Le milieu réactionnel est ensuite chauffé à 165°C durant 3 h 30, puis versé dans 10 mL d'eau froide. Le pH est ensuite ajusté par ajout de NaHCO₃ (pH 9 environ) puis le milieu est extrait au reflux du toluène (10 mL) pendant 15 min. La phase organique est ensuite recueillie et le produit **11** est obtenu par cristallisation à -18°C durant une nuit, filtration puis séchage sous vide : 0,48 g (Rdt = 58 %). PF : 106°C (déc.).

### 6-3 : Synthèse du PA 1035

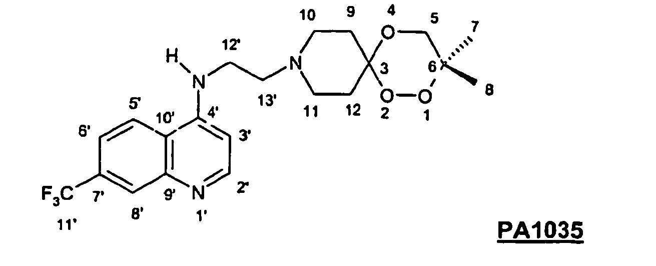

0,18 g (0,8 mmole) de **PA 1024** et 0,24 g (0,7 mmole) du composé **11** sont solubilisés dans 5 mL d'acétonitrile. Au mélange obtenu est ajouté 0,34 g (3,2 mmoles) de Na₂CO₃ et le mélange est laissé sous agitation à température ambiante pendant 45 min. Le mélange est ensuite chauffé 45 min à 40°C, 45 min à 50°C, 45 min à 60°C et 1 h 30 à 70°C. Le mélange réactionnel est ensuite filtré, les solvants évaporés et le résidu est purifié par colonne de chromatographie (SiO₂ 60 ACC 70-200 µm, éluant: dichlorométhane / triéthylamine, 80/20, v/v). La sortie des produits de colonne est contrôlée par CCM (SiO₂ 60 F 254). Les solvants des phases contenant le produit de couplage sont évaporés, le brut est solubilisé dans 50 mL d'éther et lavé par 10 mL d'eau. La phase organique est séchée par addition de Na₂SO₄, filtrée et évaporée sous vide pendant 24 h, conduisant à une poudre blanche identifiée comme étant le **PA 1035** : 0,46 g (Rdt = 13 %). RMN ¹H (300 MHz, 298 K, CDCl₃) δ, ppm : 8,63 (d, *J* = 5,1 Hz, 1H, *H*C2'), 8,30 (s, 1H, *H*C8'), 7,92 (d, *J* = 8,7 Hz, 1H, *H*C5'), 7,64 (dd, *J* = 8,7 Hz, *J* = 1,5 Hz, *H*C6'), 6,48 (d, *J* = 5,4 Hz, 1H, *H*C3'), 6,23 (s, 1H, *H*N), 3,79 (s large, 1H, *H*C5), 3,59 (s large, 1H, *H*C5), 3,38 (s, 2H, *H*C12'), 2,87 (t, *J* = 5 ,7 Hz, 2H, *H*C13'), 2,74-1,90 (m, 8H, *H*-pipéridone), 1,37 (s, 3H, *H*C7, *H*C8), 1,04 (s, 3H, *H*C7, *H*C8) ; PF : 154°C (déc) ; SM (DCI/NH₃>0) m/z(%) : 426 (MH+, 100%).

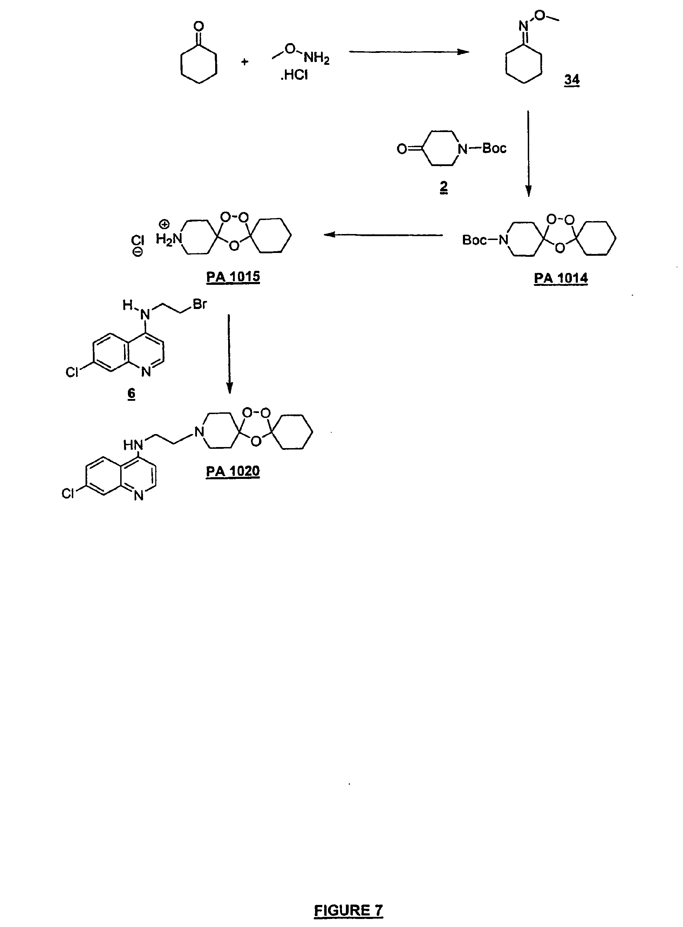

### 7 - Synthèse de PA 1020, molécule achirale (Figure 7)

7-chloro-*N*-[2-(7,14,15-trioxa-3-azadispiro[5.1.5.2]pentadéc-3-yl)éthyl]quinolin-4-amine

### 7-1 : Procédure générale pour la préparation des 1,2,4-trioxolanes

Un ozoneur (Trailigaz^{®} LI, 1981) est utilisé pour générer l'ozone à partir de l'oxygène. Le dérivé O-méthyl-oxime cétone, ainsi que la N-Boc-pipéridone **2** sont dissous dans un mélange dichlorométhane/pentane 20/80, v/v puis placés à 0°C. Le milieu réactionnel est tout d'abord purgé par bullage d'oxygène pendant 5 minutes, puis soumis au bullage d'ozone pendant 10 minutes (débit : 200 Uh, puissance : 0,6 A). La solution est ensuite purgée 5 minutes par bullage d'oxygène puis 10 minutes par bullage d'argon. Le brut réactionnel est ensuite concentré à 28°C à l'aide d'un évaporateur rotatif. L'huile ainsi obtenue est ensuite chromatographiée sur colonne de silice.

### 7-2 : Synthèse du trioxolane PA 1014

Une solution de O-méthyl-oxime cyclohexanone **34** (0,636 g, 5 mmoles), préparée selon la méthode décrite par Donaruma et al., J. Org. Chem., 1957, 22, 1024, et du composé **2** (1,99 g, 10 mmoles) dans un mélange dichlorométhane (20 mL) / pentane (80 mL) est traitée par bullage d'ozone selon la procédure générale détaillée en 7-1 ci-dessus. Le brut réactionnel obtenu est ensuite chromatographié sur colonne de silice (SiO₂ 60 AAC 6-35 µm, éluants : hexane/éther 65/35, v/v). Le **PA 1014** est obtenu, après recristallisation dans 15 ml d'un mélange éthanol/eau (2/1, v/v), sous la forme d'une poudre blanche : 0,22 g (Rdt = 14 %).

### 7-3 : Synthèse du trioxolane PA 1015

Le **PA 1014** (0,610 g, 1,95 mmoles) est dissous dans 3 mL d'acétate d'éthyle, puis l'acide chlorhydrique 12 M (290 µL, 3,48 mmoles), préalablement dilué dans 870 µl d'acétate d'éthyle, est versé. Le mélange est maintenu sous agitation magnétique à température ambiante durant 17 h. Le produit est ensuite précipité par ajout de 25 mL de diéthyléther, puis filtré sur fritté. Après lavage à l'éther et séchage sous vide, le produit **PA 1015** est obtenu sous la forme d'une poudre blanche : 0,25 g (Rdt = 51 %).

### 7-4 : Synthèse du PA 1020

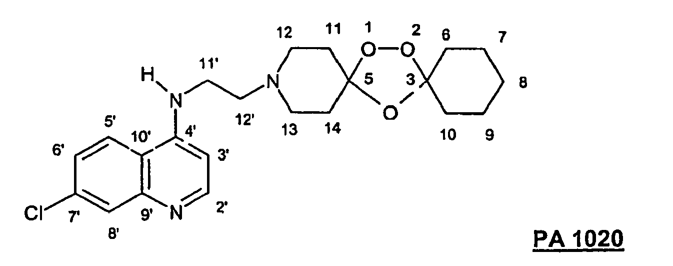

Le **PA 1015** (0,18 g, 0,73 mmole) et le composé **6** (0,21 g, 0,73 mmole) sont mis en suspension dans 5 mL de diméthylformamide puis 226 µL (1,62 mmol) de triéthylamine sont ajoutés goutte à goutte. Le milieu est maintenu sous agitation magnétique et à température ambiante pendant 89 h. Après ajout de 70 mL d'eau, la phase aqueuse est extraite par 100 mL de diéthyléther, puis saturée en NaCl et extraite 2 fois avec 100 mL d'éther. Les phases éthérées sont regroupées, séchées avec Na₂SO₄, concentrées à l'évaporateur rotatif puis chromatographiées sur silice (SiO₂ 60AAC 6-35 µm, éluants : éther / triéthylamine (9,9/0,1 v/v)). Les phases contenant le **PA 1020** sont rassemblées, puis les solvants évaporés, conduisant à une poudre blanche : 0,09 g (Rdt = 29 %). PF : 153 °C (déc.). RMN ¹H (400 MHz, 298 K, CDCl₃) δ, ppm : 8,53 (d, *J* = 5,6 Hz, 1H, *H*C2'), 7,99 (d, *J* = 1,5 Hz, 1H, *H*C8'), 7,68 (d, *J* = 6,9 Hz, 1H, *H*C5'), 7,40 (dd, *J* = 5,6 Hz et *J* = 1.5 Hz, 1H, *H*C6'), 6,39 (d, *J* = 5,6 Hz, 1H, *H*C3'), 6,10 (s large, 1H, *H*N), 3,33 (m, 2H, *H*C11'), 2,82 (t, 2H, *H*C12'), 2,72 et 2,57 (2m, 2H et 2H, *H*C12 et *H*C13), 1,94 (m, 4H, *H*C11 + *H*C14), 1,81-1,57 (m, 14H, *H*C6 + *H*C7 + *H*C9 + *H*C10), 1,47 (m, 1H, *H*C8), 1,40 (m, 1H, *H*C8). RMN ¹³C (100 MHz, 298 K, CDCl₃) δ, ppm : 152,02 (C2'), 150,33 (C4'), 148,92 (C9'), 135,54 (C7'), 128,77 (C8'), 125,97 (C6'), 121,55 (C5'), 117,59 (C10'), 109,93 (C3), 107,20 (C5), 99,64 (C3'), 55,29 (C12'), 51,04 (C12, C13), 39,57 (C11'), 34,83 (C11, C14), 34,96 et 24,19 (C6, C7, C9, C10), 25,24 (C8). SM (ES/MS>0) m/z (%) : 418,25 (MH+). Analyse élémentaire : pour C₂₂H₂₈ClN₃O₃: % théor. C 63,22, H 6,75, N 10,05 ; % expér. C 63,26, H 6,74, N 9,76.

La pureté du composé a été déterminée par HPLC selon les conditions suivantes : colonne Beckman Coulter ODS C18, 5 µm, 4,6 x 250 mm ; éluants : A : H₂O 0.1 % TFA, B : CH₃CN/H₂O 90/10 0,1 % TFA, gradient : de 10 % à 100 % de B en 40 min, puis 100 % de B pendant 20 min. Débit : 1 mL/min. µ = 254 nm. Volume injecté : 10 µL. Echantillon à 1 mg/mL dans le méthanol. Pureté : 98%.

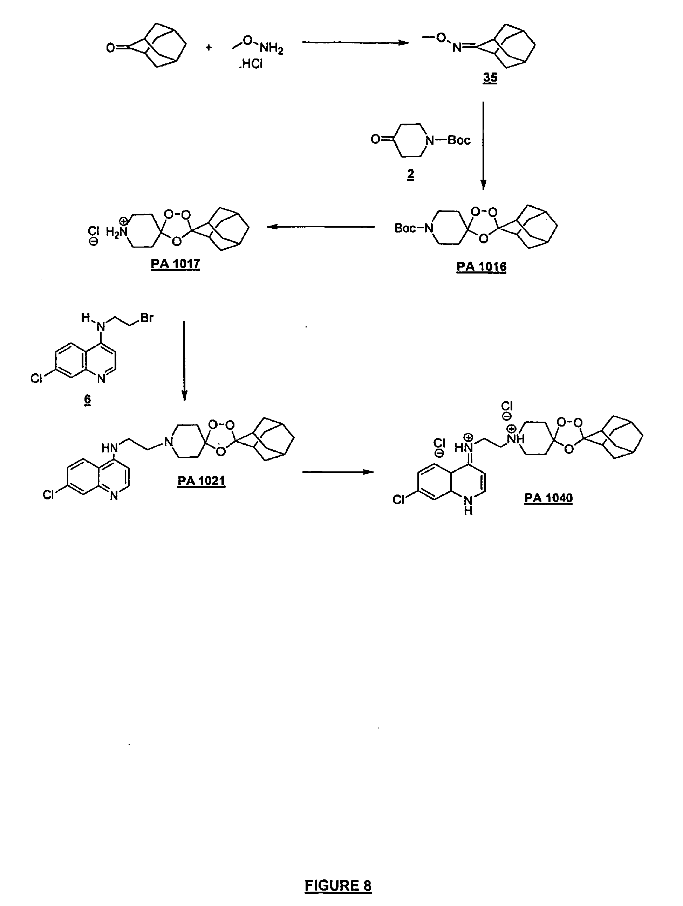

### 8 - Synthèse de PA 1021, molécule achirale, et son sel PA 1040 (Figure 8)

7-chloro-N-[2-(1*H*-dispiro[pipéridine-4,3'-[1,2,4]trioxolane-5',2"-tricyclo[3.3.1.1^{3,7}] décan]-1-yl)éthyl]quinolin-4-amine

### 8-1 : Synthèse de la O-méthyl-oxime 2-adamantanone 35

A 4,51 g (30 mmoles) de 2-adamantanone dans 30 mL de méthanol sont ajoutés 3,76 g (45 mmoles) de méthoxylamine, sous forme de chlorhydrate, ainsi que 4,36 g (55,1 mmoles) de pyridine. Le milieu réactionnel est agité à température ambiante pendant 66 h, les solvants sont évaporés sous vide, puis le résidu est dilué avec 50 mL de dichlorométhane et 50 mL d'eau. La phase organique est séparée puis la phase aqueuse est extraite à nouveau avec 30 mL de dichlorométhane. Les phases organiques sont regroupées, lavées avec deux fois 30 mL d'acide chlorhydrique dilué (1 M), 30 mL de saumure, puis séchées sur sulfate de magnésium. Après filtration et évaporation à l'évaporateur rotatif, le produit **35** est obtenu sous la forme d'une poudre blanche : 3,57 g (Rdt = 66 %).

### 8-2 : Synthèse du trioxolane PA 1016

Le composé est synthétisé selon le mode opératoire décrit dans le brevet US 6,486,199 (Vennerstrom et coll.). Une solution de O-méthyl-oxime 2-adamantanone **35** (0,9 g, 5 mmoles) et de 2 (1,99 g, 10 mmoles) dans un mélange dichlorométhane (20 mL) / pentane (80 mL) est traitée par bullage d'ozone selon la procédure générale décrite en 7-1 ci-dessus. Le brut réactionnel obtenu est ensuite chromatographié par colonne de silice (SiO₂ 60 AAC 6-35 µm, éluants : hexane/éther 80/20, v/v). Le N-Boc-trioxolane **PA 1016** est obtenu, après recristallisation dans 15 ml d'éthanol/eau (2/1, v/v), sous la forme d'une poudre blanche : 0,37 g (Rdt = 21 %).

### 8-3 : Synthèse du trioxolane PA 1017

A 0,65 g (1,78 mmoles) de **PA 1016** dans 3 mL d'acétate d'éthyle sont ajoutés 266 µl (3,20 mmoles) d'acide chlorhydrique 12 M, préalablement dilué dans 800 µL d'acétate d'éthyle. Le mélange est maintenu sous agitation à température ambiante durant 20 h. Le produit est ensuite précipité par ajout de 25 mL de diéthyléther, puis filtré sur fritté. Après lavage à l'éther et séchage sous vide, le produit **PA 1017** est obtenu sous la forme d'une poudre blanche : 0,42 g (Rdt = 78 %).

### 8-4 : Synthèse du PA 1021

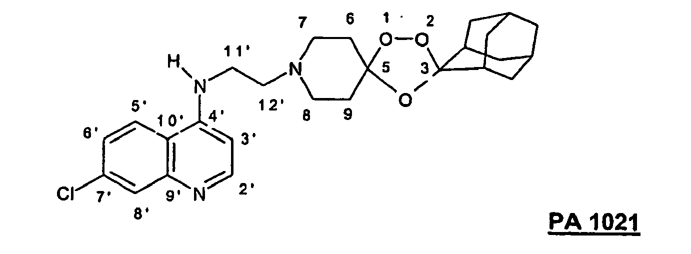

Le **PA 1017** (0,32 g, 1,06 mmoles) et le composé **6** (0,30 g, 1,06 mmoles) sont mis en suspension dans 10 mL de diméthylformamide, puis 326 µL (2,33 mmoles) de triéthylamine sont ajoutés goutte à goutte. Le milieu est maintenu sous agitation magnétique à température ambiante pendant 112 h. Après ajout de 100 mL d'eau, la phase aqueuse est extraite par 100 mL de diéthyléther, puis saturée en NaCl et extraite avec 2 fois 100 mL d'éther. Les phases éthérées sont regroupées, séchées avec Na₂SO₄, concentrées à l'évaporateur rotatif puis chromatographiées sur silice (SiO₂ 60 AAC 6-35 µm, éluants : acétate d'éthyle / triéthylamine 97/3, v/v). Les phases contenant le **PA 1021** sont rassemblées, puis les solvants évaporés, conduisant à une poudre blanche : 0,25 g

(Rdt = 52 %). PF : 130°C. RMN ¹H (400 MHz, 298 K, CDCl₃) δ, ppm : 8,53 (d, *J* = 5,6 Hz, 1H, *H*C₂'), 7,98 (d, *J* = 1,5 Hz, 1H, *H*C8'), 7,69 (d, *J* = 6,9 Hz, 1H, *H*C5'), 7,40 (dd, *J* = 5,6 Hz et *J* = 1.5 Hz, 1H, *H*C6'), 6,38 (d, *J* = 5,6 Hz, 1H, *H*C3'), 6,10 (s large, 1H, *H*N), 3,33 (m, 2H, *H*C11'), 2,82 (m, 2H, *H*C12'), 2,73 et 2,57 (2m, 2H et 2H, *H*C7 et *H*C8), 1,94 (m, 2H + 2H, *H*C6 + *H*C9), 2,04-1,70 (m, 14H, *H*-adamantane). RMN ¹³C (100 MHz, 298 K, CDCl₃) δ, ppm : 152,95 (C2'), 150,30 (C4'), 149,00 (C9'), 135,49 (C7'), 128,81 (C8'), 125,95 (C6'), 121,57 (C5'), 117,60 (C10'), 112,34 (C3), 107,27 (C5), 99,64 (C3'), 55,32 (C12'), 51,12 (C7, C8), 39,56 (C11'), 34,95 (C6, C9), 37,11, 36,76, 35,24, 35,15, 27,21, 26,81 (C-adamantane). SM (ES/MS>0, MeOH) m/z (%) : 470,05 (MH+, 38). Analyse élémentaire : pour C₂₆H₃₂ClN₃O₃ : % théor. C 66,44, H 6,86, N 8,94 ; % expér. C 66,66, H 6,75, N 8,60. Pureté : 98% (déterminée par HPLC selon le protocole décrit ci-dessus en 7-4).

### 8-5 : Synthèse du PA 1040

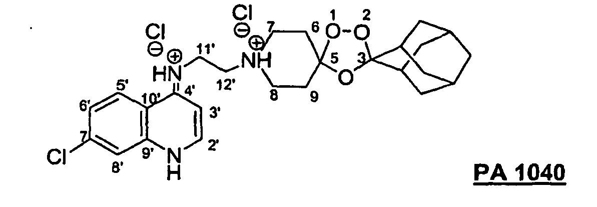

A 0,11 g (0,24 mmole) de **PA 1021** dissous dans 2,5 mL de chloroforme sont ajoutés 500 µL (0,50 mmole) d'acide chlorhydrique 1 M dans l'éther. Après addition de 15 mL d'éther, la suspension est centrifugée, puis le culot obtenu est successivement lavé avec de l'éther (deux fois 15 mL) puis avec 15 mL de pentane. Après séchage sous vide, le produit **PA 1040** est obtenu sous la forme d'une poudre blanche : 0,13 g (Rdt = 98 %). PF: 189°C (déc.). RMN ¹H (300 MHz, 298 K, DMSOd₆) δ, ppm: 14,60 (s large, 1H, HNC4'), 11,50 (s large, 1H, *H*NC14'), 9,76 (s large, 1H, HNC2'), 8,81 (d, *J* = 9,3 Hz, 1H, *H*C5'), 8,66 (d, *J* = 6,9 Hz, 1H, *H*C2'), 8,13 (d, *J* = 1,8 Hz, 1H, *H*C8'), 7,79 (dd, *J* = 9,3 Hz et *J* = 1,8 Hz, 1H, *H*C6'), 7,05 (d, *J* = 6,9 Hz, 1H, *H*C3'), 4,04 (s large, 2H, *H*C11'), 3,67-3,15 (m, 2H + 2H, *H*C7 + *H*C8), 3,41 (s large, 2H, *H*C12'), 2,50-1,66 (m, 2H + 2H + 14H, HC6 + HC9 + *H*-adamantane). SM (ES/MS>0, MeOH) m/z (%) : 470,15 (MH+). Analyse élémentaire : pour C₂₆H₃₂ClN₃O₃•2HCl•3H₂O: % théor. C 52,29, H 6,75, N 7,03 ; % expér. C 52,22, H 6,68, N 7,10.

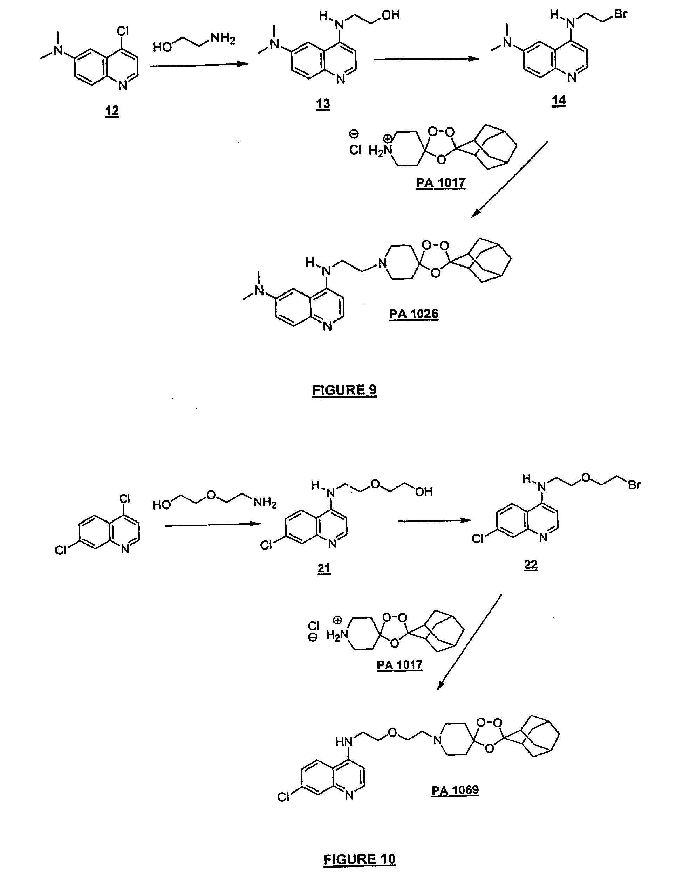

### 9 - Synthèse du PA 1026, molécule achirale (Figure 9)

*N*⁴-[2-(1*H*-dispiro[pipéridine-4,3'-[1,2,4]trioxolane-5',2"-tricyclo[3.3.1.1^{3.7}]décan]-1-yl)éthyl]-*N*⁶,*N*⁶-diméthylquinoline-4,6-diamine

### 9-1 : Synthèse de la 6-diméthylamino 4-(β-hydroxy éthylamino)quinoléine 13

Un mélange de 4-chloro-6-diméthylamino quinoléine **12** (4,92 g, 24 mmoles), préparé selon la méthode décrite par Riegel et al., J. Am. Chem. Soc., 1946, 68, 1264, et de 2-aminoéthanol (43,99 g, 720 mmoles) est chauffé sous agitation magnétique à 150°C pendant 15 min, puis à 185°C pendant 30 min. Après retour à température ambiante, le solide est mis en suspension dans 25 mL d'une solution aqueuse de soude à 10 % p/v et 165 mL d'eau. Le précipité obtenu est filtré sur fritté, lavé à l'eau puis séché sous vide. Le produit est obtenu sous la forme d'une poudre blanche : 4,86 g (Rdt = 88 %).

### 9-2 : Synthèse de la 6-diméthylamino-4-(β-bromo éthvlamino)quinoléine 14

L'acide bromhydrique (6,6 mL, 122 mmoles), puis l'acide sulfurique (2,2 mL, 41,4 mmoles) sont ajoutés goutte à goutte sur la 6-diméthylamino-4-(β-hydroxyéthylamino)quinoléine **13** (4,56 g, 19,7 mmoles), le milieu réactionnel étant réfrigéré à l'aide d'un bain d'eau froide. Le milieu réactionnel est ensuite chauffé à 165°C durant 3 h 30 puis versé dans 71 mL d'eau froide. Le pH est alors ajusté par ajout de NaHCO₃ (pH 9 environ) puis le milieu est extrait au reflux du toluène (71 mL) pendant 15 min. La phase organique est ensuite recueillie et le produit est obtenu par cristallisation à -18°C durant une nuit, filtration puis lavage à l'hexane et séchage sous vide : 3,28 g (Rdt = 57 %).

### 9-3 : Synthèse du PA 1026

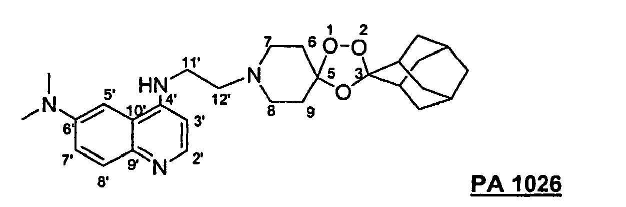

0,30 g (0,99 mmole) de **PA 1017** ainsi que 0,29 g (0,99 mmole) de 6-diméthylamino-4-(β-bromoéthylamino)quinoléine **14** sont mis en suspension dans 10 mL de diméthylformamide, puis 305 µL (2,18 mmoles) de triéthylamine sont ajoutés goutte à goutte. Le milieu est maintenu sous agitation magnétique à température ambiante pendant 113 h. Après ajout de 100 mL d'eau, la phase aqueuse est extraite par 100 mL de diéthyléther, puis saturée en NaCl et extraite avec deux fois 100 mL d'éther. Les phases éthérées sont regroupées, séchées avec Na₂SO₄, concentrées à l'évaporateur rotatif puis chromatographiées sur silice (SiO₂ 60AAC 6-35 µm, éluants : dichlorométhane

/ triéthylamine (9/1, v/v)). La sortie du produit est suivie par CCM (SiO₂ 60F254). Après précipitation au dichlorométhane / hexane, le produit **PA 1026** est obtenu sous la forme d'une poudre blanche : 0,14 g (Rdt = 30 %). RMN ¹H (300 MHz, 298 K, CDCl₃) δ, ppm : 8,36 (d, *J* = 5,1 Hz, 1H, *H*C2'), 7,96 (d, *J* = 9,3 Hz, 1H, *H*C8'), 7,32 (dd, *J* = 9,3 Hz et *J* = 2,4 Hz, 1H, *H*C7'), 6,66 (d, *J* = 2,4 Hz, 1H, *H*C5'), 6,36 (d, *J* = 5,1 Hz, 1H, *H*C3'), 5,99 (s large, 1H, *H*N), 3,35 (m, 2H, *H*C11'), 2,85 (m, 2H, *H*C12'), 2,69 et 2,63 (2m, 2H et 2H, *H*C7 et *H*C8), 2,04-1,71 (m, 2H + 2H + 14H, *H*C6 + *H*C9 + *H*-adamantane). SM (ES/MS>0, MeOH ) m/z (%) : 479,45 (MH+). Analyse élémentaire : pour C₂₈H₃₈N₄O₃•H₂O : % théor. C 67,71, H 8,12, N 11,28; % expér. C 67,78, H 8,02, N 11,13. Pureté: 99 % (déterminée par HPLC selon le protocole décrit en 7-4 ci-dessus).

### 10 - Synthèse du PA 1069, molécule achirale (Figure 10)

7-chloro-*N*-{2-[2-(1*H*-dispiro[piperidine-4,3'-[1,2,4]trioxolane-5',2"-tricyclo[3.3.1.1^{3,7}] decan]-1-yl)ethoxy]ethyl}quinolin-4-amine

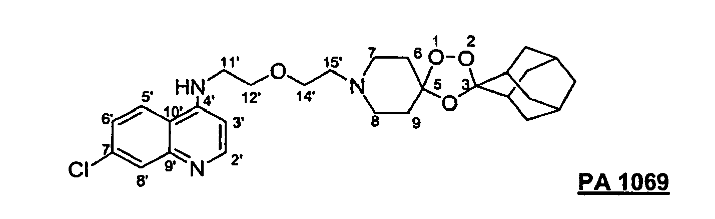

### 10-1 : Synthèse du 2-[2-(7-chloro-quinolin-4-ylamino)-ethoxy]-ethanol 21

Un mélange de 4,7-dichloroquinoléine (5,0 g, 25,0 mmoles) et de 2-(2-aminoéthoxy)éthanol (10,51 g, 100,0 mmoles) est chauffé à 135°C sous agitation magnétique durant 5 h. Après refroidissement, le milieu réactionnel est repris dans 200 mL d'éther diéthylique et l'agitation est maintenue une nuit à température ambiante. Le résidu solide formé est isolé, puis dissous dans un mélange de 500 mL de dichlorométhane/triéthylamine (9/1, v/v) et filtré sur fritté contenant un lit de silice (SiO₂ 60 AAC 70-200 µm). Le produit est ensuite élué de la silice par de l'éthanol (2 L). Les phases éthanoliques sont regroupées et évaporées. 50 mL de dichlorométhane sont ajoutés au résidu et la phase organique est lavée avec deux fois 50 mL d'eau. La phase organique est récupérée, séchée sur Na₂SO₄, puis évaporée à l'évaporateur rotatif. Après séchage sous vide, le composé **21** est obtenu sous la forme d'une poudre beige : 5,03 g (Rdt = 75 %).

### 10-2 : Synthèse de la [2-(2-bromo-ethoxy)-ethyl]-(7-chloro-quinolin-4-yl)-amine 22

L'acide bromhydrique (5,9 mL, 109,1 mmoles), puis l'acide sulfurique (2,0 mL, 37,0 mmoles) sont ajoutés goutte à goutte sur 4,7 g de composé **21** (17,6 mmoles), le milieu réactionnel étant réfrigéré à l'aide d'un bain d'eau froide. Le milieu réactionnel est ensuite chauffé à 155°C durant 2 h 30, puis versé dans 70 mL d'eau froide. Le pH est ensuite ajusté par ajout de NaHCO₃ (pH 9 environ), puis le milieu est extrait au reflux du toluène (70 mL) pendant 20 min. La phase organique est ensuite recueillie et le composé **22** est obtenu après cristallisation à -18°C durant une nuit, filtration, lavage à l'hexane, puis séchage sous vide : 1,77 g (Rdt = 31 %).

### 10-3 : Synthèse du PA 1069

0,50 g (1,66 mmoles) de **PA 1017** et 0,77 g (1,66 mmoles) de composé **22** sont mis en solution dans 25 mL de diméthylformamide puis 508 µL (3,64 mmoles) de triéthylamine sont ajoutés goutte à goutte. Le milieu est maintenu sous agitation magnétique à température ambiante pendant 101 h. Après ajout de 100 mL de dichlorométhane, la phase organique est lavée 1 fois avec 200 mL d'eau saturée en NaHCO₃, puis 3 fois avec 200 mL d'eau. La phase organique est ensuite séchée avec Na₂SO₄, concentrée à l'évaporateur rotatif puis chromatographiée sur silice (SiO₂ 60 AAC 6-35 µm, éluants : acétate d'éthyle/triéthylamine (90/10, v/v)). Après recristallisation dichlorométhane/n-hexane et séchage sous vide, le produit **PA 1069** est obtenu sous la forme d'une poudre blanche : 0,29 g (Rdt = 35 %). PF : 144°C (déc.). RMN ¹H (300 MHz, 298 K, CDCl₃) δ, ppm : 8,53 (d, *J* = 5,4 Hz, 1H, *H*C2'), 7,99 (d, *J* = 2,1 Hz, 1H, *H*C8'), 7,86 (d, *J* = 9,0 Hz, 1H, *H*C5'), 7,44 (dd, *J* = 9,0 Hz et *J* = 2,1 Hz, 1H, *H*C6'), 6,42 (d, *J* = 5,4 Hz, 1H, *H*C3'), 5,86 (s large, 1H, *H*N), 3,83 (m, 2H, *H*C12'), 3,70 (m, 2H, *H*C14'), 3,51 (m, 2H, *H*C11'), 2,78-2,60 (m, 2H + 2H + 2H, *H*C15' + *H*C7 + *H*C8), 2,05-1,70 (m, 2H + 2H + 14H, *H*C6 + *H*C9 + *H*-adamantane). SM (ES/MS>0, MeOH) m/z (%): 514,25 (MH+). Analyse élémentaire : pour C₂₈H₃₆ClN₃O₄ : % théor. C 65,42, H 7,06, N 8,17 ; % expér. C 65,32, H 7,02, N 8,08.

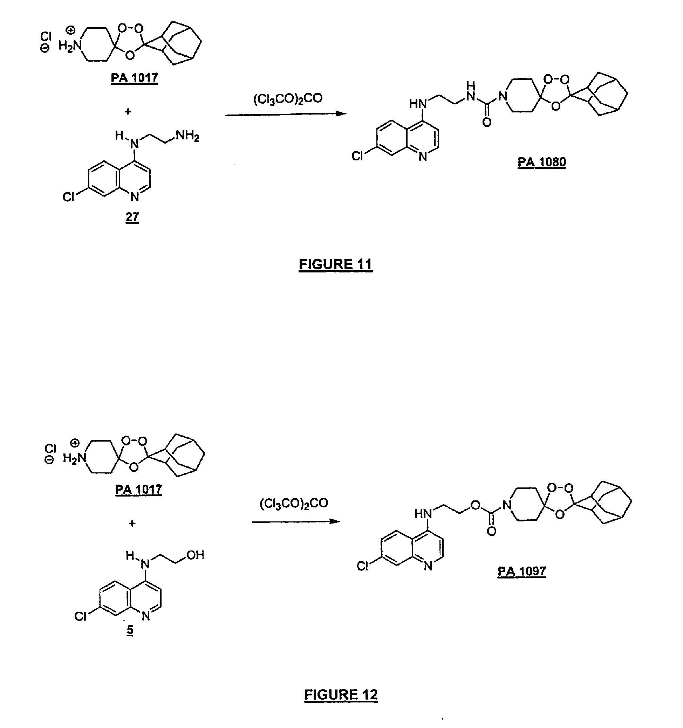

### 11 - Synthèse du PA 1080, molécule achirale (Figure 11)

*N*-{2-[(7-chloroquinolin-4-yl)amino]ethyl}-1*H*-dispiro[piperidine-4,3'-[1,2,4] trioxolane-5',2"-tricydo[3.3.1.1^{3,7}]decane]-1-carboxamide

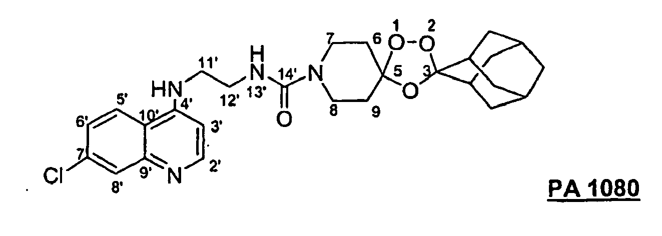

A 0,53 g (1,76 mmoles) de **PA 1017** préalablement séché sous vide dans 15 mL de dichlorométhane sec, 544 µL (3,87 mmoles) de triéthylamine anhydre sont ajoutés. Cette solution est alors ajoutée goutte à goutte à une solution de 0,19 g (0,65 mmole) de triphosgène dans 5 mL de dichlorométhane sec. Le milieu réactionnel est maintenu sous agitation magnétique à température ambiante durant 1 h. Par ailleurs, à une solution de 0,39 g (1,76 mmoles) de **27** (préparé selon la méthode décrite par Meunier *et al.* dans la demande internationale PCT WO 01/77105) dans 15 mL de dichlorométhane sec est ajouté 272 µL (1,94 mmoles) de triéthylamine anhydre. Cette solution est ensuite transférée sur le milieu réactionnel initial et l'ensemble est maintenu sous agitation magnétique et à température ambiante durant 16 h. Après ajout de 100 mL de dichlorométhane, la phase organique est lavée avec 200 mL d'eau saturée en NaHCO₃ puis avec deux fois 200 mL d'eau. La phase organique est ensuite séchée avec Na₂SO₄, concentrée puis chromatographiée sur silice (SiO₂ 60 AAC 6-35 µm, éluants: dichlorométhane/triéthylamine (90/10, v/v)). Après recristallisation avec un mélange dichlorométhane/diéthyléther, le composé obtenu est repris dans 75 mL de chloroforme. La phase organique est successivement lavée avec 150 mL d'eau, 150 mL de saumure, et avec trois fois 150 mL d'eau. La phase organique est ensuite séchée avec Na₂SO₄, concentrée à l'évaporateur rotatif et séchée sous vide. Le produit **PA 1080** est obtenu sous la forme d'une poudre blanche : 0,20 g (Rdt = 23 %). PF : 192°C (déc.). RMN ¹H (250 MHz, 298 K, CDCl₃) δ, ppm : 8,41 (d, *J* = 5,6 Hz, 1H, *H*C2'), 7,89 (d, *J* = 2,0 Hz, 1H, *H*C8'), 7,86 (d, *J* = 8,8 Hz, 1H, *H*C5'), 7,47 (s large, 1H, *H*NC12'), 7,34 (dd, *J* = 8,8 Hz et *J* = 2,0 Hz, 1H, *H*C6'), 6,21 (d, *J* = 5,6 Hz, 1H, *H*C3'), 5,30 (t, *J* = 6,4 Hz, 1H, *H*NC13'), 3,69 et 3,32 (2m, 2H et 2H, *H*C11' et *H*C12'), 3,52 (m, 2H + 2H, *H*C7 + *H*C8), 1,93-1,66 (m, 2H + 2H + 14H, *H*C6 + *H*C9 +*H*-adamantane). SM (ES/MS>0, MeOH) m/z (%) : 513,30 (MH+). Analyse élémentaire : pour C₂₇H₃₃ClN₄O₄•0,7H₂O : % théor. C 61,69, H 6,59, N 10,66; % expér. C 61,69, H 6,09, N 10,35.

### 12 - Synthèse du PA 1097, molécule achirale (Figure 12)

2-[(7-chloroquinolin-4-yl)amino]ethyl-1*H*-dispiro[piperidine-4,3'-[1,2,4]trioxolane-5',2"-tricyclo[3.3.1.1^{3,7}]decane]-1-carboxylate

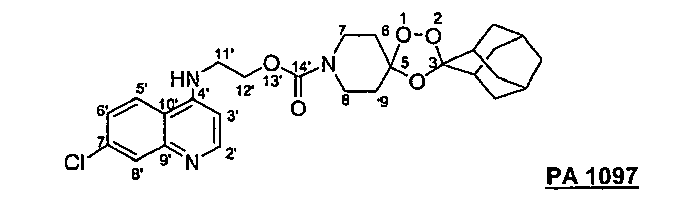

A 0,42 g (1,92 mmoles) de composé **5** (préalablement séché sous vide) dans 15 mL de dichlorométhane sec sont ajoutés 269 µL (1,92 mmoles) de triéthylamine anhydre. La solution ainsi préparée est ensuite ajoutée sur 0,21 g (0,71 mmole) de triphosgène dans 5 mL de dichlorométhane sec. Le milieu réactionnel est maintenu sous agitation magnétique à température ambiante durant 3 h 30. Par ailleurs, au **PA 1017** sec (0,58 g, 1,90 mmoles) dans 15 mL de dichlorométhane sec sont ajoutés 594 µL (4,22 mmoles) de triéthylamine anhydre. Cette solution est alors transférée sur le milieu réactionnel initial et l'ensemble est maintenu sous agitation magnétique à température ambiante durant 1 h 45. Après ajout de 100 mL de dichlorométhane, la phase organique est lavée avec 200 mL d'eau saturée en NaHCO₃ puis avec deux fois 150 mL d'eau. La phase organique est ensuite séchée avec Na₂SO₄, concentrée à l'évaporateur rotatif puis chromatographiée sur silice (SiO₂ 60 AAC 6-35 µm, éluants : acétate d'éthyle/triéthylamine (90/10, v/v)). Les fractions contenant le produit sont regroupées, les solvants évaporés et 70 mL de chloroforme sont ajoutés. La phase organique est lavée avec deux fois 150 mL d'eau, séchée sur MgSO₄. Un volume d'hexane est ajouté puis l'ensemble est ensuite concentré à l'évaporateur rotatif puis séché sous vide. Le produit **PA 1097** est obtenu sous la forme d'une poudre blanche : 0,07 g (Rdt = 7 %). PF : 159°C (déc.). RMN ¹H (300 MHz, 298 K, CDCl₃) δ, ppm : 8,55 (d, *J* = 5,4 Hz, 1H, *H*C2'), 7,97 (d, *J* = 2,1 Hz, 1H, *H*C8'), 7,72 (d, *J* = 8,7 Hz, 1H, *H*C5'), 7,40 (dd, *J* = 8,7 Hz, *J* = 2,1 Hz, 1H, *H*C6'), 6,37 (d, *J* = 5,4 Hz, 1H, *H*C3'), 6,31 (s large, 1H, *H*N), 4,56 (m, 2H, *H*C12'), 3,60-3,52 (m, 2H + 2H + 2H, *H*C7 + *H*C8 + *H*C12'), 2,05-1,63 (m, 2H + 2H + 14H, *H*C6 + *H*C9 + *H*-adamantane). SM (DCl/NH₃>0) m/z (%): 514 (MH+). Analyse élémentaire : pour C₂₇H₃₂ClN₃O₅•1H₂O : % théor. C 60,95, H 6,44, N 7,90; % expér. C 60,88, H 5,75, N 7,73.

Outre les composés dont les protocoles d'obtention ont été détaillés dans ce qui précède, d'autres composés de formule (I) selon l'invention sont rassemblés dans le tableau suivant ; ces exemples ne sont pas limitatifs et ne font qu'illustrer la présente invention.

**Tableau**

| **Composé** | **Point de fusion** **(°C)** | **RMN¹H** **(**δ**, ppm)** |
|---|---|---|
| 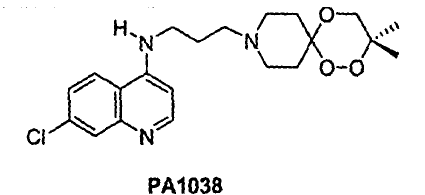 | 144 | 300 MHz, 298 K, CDCl₃: 8,52 (d, *J* = 5,1 Hz, 1H), 7,95 (s, 1H), 7,76 (d, *J* = 9,0 Hz, 1H). 7,57 (s, 1H). 7,37 (dd, *J* = 8,7 Hz, *J* = 1,8 Hz), 6,34 (d, *J* = 5,1 Hz, 1H), 3,85 (s large, 1H), 3,53 (s large, 1H), 3,40 (m, 2H), 2,73-2,51 (m, 2H + 4H), 2,18-1,87 (m, 2H + 4H), 1,55 (s, 3H), 1,17 (s, 3H) |
| 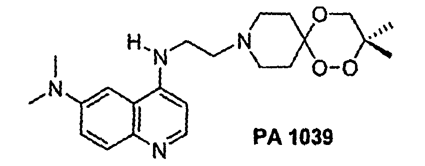 | 157 (déc.) | 300 MHz, 298 K, CDCl₃: 8,38 (d, *J* = 5,1 Hz, 1H), 7,88 (d, *J* = 9,3 Hz, 1H), 7,31 (dd, *J* = 2,7 Hz, *J* = 9,3 Hz, 1H), 6,66 (s, 1H), 6,35 (d, *J* = 5,1 Hz, 1H), 5,82 (s. 1H), 3,79 (s large, 1H), 3,47 (s large, 1H), 3,33 (q, *J* = 5,4 Hz, 2H), 3,07 (s, 6H), 2,83 (t, *J* = 5,4 Hz, 2H), 2,66-2,50 (m, 4H), 2,42-1,80 (m, 4H), 1,51 (s, 3H), 1,12 (s, 3H) |
| 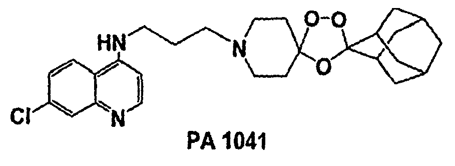 | 159 (déc.) | 300 MHz, 298 K, CDCl₃: 8,50 (d, *J* = 5,4 Hz, 1H), 7,99 (d, *J* = 2,1 Hz, 1H), 7,78 (d, *J* = 9,0 Hz, 1H), 7,60 (s large, 1H), 7,38 (dd, *J* = 9,0 Hz et *J* = 2,1 Hz, 1H), 6,35 (d, *J* = 5,4 Hz, 1H), 3,41 (m, 2H), 2,77-2,58 (m, 2H + 2H + 2H), 2,09-1,72 (m, 2H + 2H + 2H + 14H) |
| 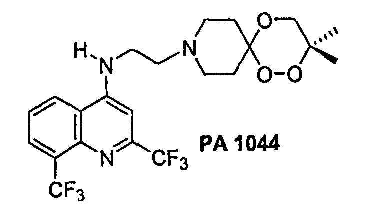 | 144 (déc.) | 8,08 (d, *J* = 7,5 Hz, 1H), 7,98 (d, *J* = 8,4 Hz), 7,58 (dd, *J* = 7,8 Hz, *J* = 7,8 Hz, 1H), 6,75 (s, 1H), 6,40 (s, 1H), 3,81 (s large, 1H), 3,49 (s large, 1H), 3,39 (q, *J* = 5,1 Hz, 2H), 2,86 (t, *J* = 6,0 Hz, 2H), 2,71-2,52 (m, 4H), 2,18-1,70 (m, 4H), 1,52 (s, 3H), 1,14 (s, 3H) |
| 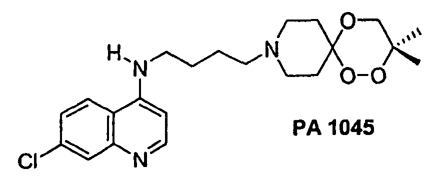 | 145 | 300MHz, 298K, CDCl₃: 8,54 (d, *J* = 5,4 Hz, 1H), 7,96 (d, *J* = 2,1 Hz, 1H), 7,67 (d, *J* = 9,0 Hz, 1H), 7,37 (dd, *J* = 9,0 Hz, *J* = 2,4 Hz), 6,42 (d, *J* = 5,4 Hz, 1H), 5,33 (s, 1H), 3,80 (s large, 1H), 3,46 (s large, 1H), 3,34 (q, *J* = 6,3 Hz), 2,68-2,38 (m, 2H + 4H), 2,10-1,78 (m, 2H + 4H), 1,70 (q, *J* = 6,9 Hz, 2H), 1,51 (s, 3H), 1,15 (s, 3H) |
| 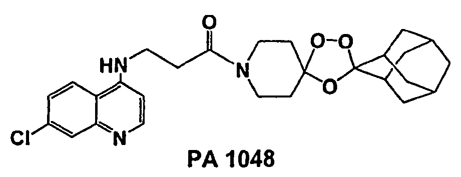 | 137 | 300 MHz, 298 K, CDCl₃: 8,49 (d, *J* = 5,4 Hz, 1H), 7,97 (d, *J* = 2,1 Hz, 1H), 7,76 (dd, *J* = 9,0 Hz et *J* = 2,1 Hz, 1H), 7,36 (d, *J* = 9,0 Hz, 1H), 6,44 (d, *J* = 5,4 Hz, 1H), 6,43 (s large, 1H), 3,87 et 3,63 (2m, 1H et 1H), 3,70 (m, 2H), 3,54 (m, 1H + 1H), 2,76 (m, 2H), 2,00-1,70 (m, 2H + 2H + 14H) |
| 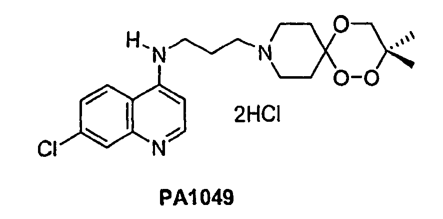 | 142 (déc.) | 300 MHz, 298 K, DMSOd₆: 14,30 (s, 1H), 11,20 (s, 1H), 9,71 (s, 1H), 8,73 (d, *J* = 9,0 Hz), 8,59 (d, *J* = 6,9 Hz), 8,08 (d, *J* = 2,1 Hz, 1H), 7,79 (dd, *J* = 9,3 Hz , *J* = 2,1 Hz, 1H), 6,95 (d, *J* = 6,9 Hz), 3,75 (m, 1H), 3,67-3,40 (m, 2H + 1H + 2H), 3,22 (m, 2H), 2,93 (m, 2H), 2,25-1,80 (m, 2H + 4H), 1,39-1,06 (m, 6H) |
| 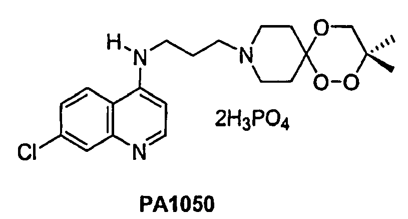 | 166 (déc.) | 300 MHz, 298 K, D₂O: 8,22 (d, *J* = 7,2 Hz, 1H), 8,02 (m, 1H), 7,73 (m, 1H), 7,53 (m, 1H), 6,72 (d, *J* = 7,2 Hz), 3,80 (m, 1H), 3,70-3,40 (m, 2H + 1H + 2H), 3,23 (m, 2H), 3,13-2,97 (m, 2H), 2,19-1,87 (m, 2H + 4H), 1,43-1,05 (m, 6H) |
| 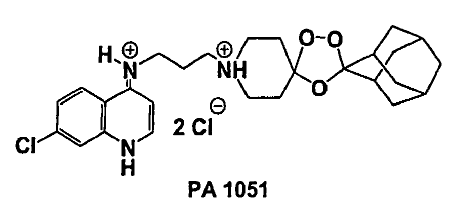 | 120 (déc.) | 300 MHz, 298 K, DMSO-d₆: 14,25 (s large, 1H), 11,39 (s large, 1H). 9,63 (s large, 1H), 8,70 (d, *J* = 9,0 Hz, 1H), 8,59 (d, *J* = 7,2 Hz, 1H), 8,06 (d, *J* = 2,1 Hz, 1H), 7,78 (dd, *J* = 9,0 Hz et *J* = 2,1 Hz, 1H), 6,94 (d, *J* = 7,2 Hz, 1H), 3,65 (s large, 2H), 3,60-2,90 (m, 2H + 2H), 3,20 (s large, 2H), 2,27-1,66 (m, 2H + 2H + 2H + 14H) |
| 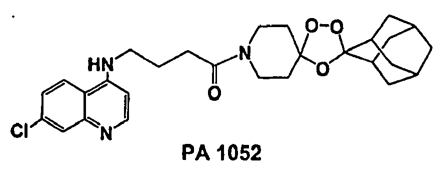 | 149(déc.) | 300 MHz, 298 K, CDCl₃: 8,48 (d, *J* = 5,4 Hz, 1H), 7,96 (d, *J* = 2,1 Hz, 1H), 7,85 (d, *J* = 9,0 Hz, 1H), 7,37 (dd, *J* = 9,0 Hz, *J* = 2,1 Hz, 1H), 6,96 (s large, 1H), 6,32 (d, *J* = 5,4 Hz, 1H), 3,85 et 3,71 (2m, 1H et 1H), 3,55 (m, 1H + 1H), 3,33 (m, 2H), 2,61 (m, 2H), 2,20 (m, 2H), 2,00-1,70 (m, 2H + 2H + 14H) |
| 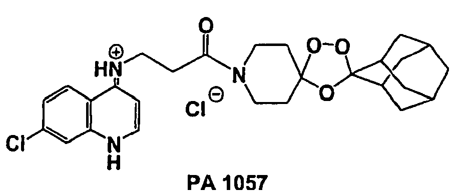 | 90 (déc.) | 300 MHz, 298 K, DMSOd₆: 14,28 (s large, 1H), 9,52 (s large, 1H), 8,65 (d, *J* = 9,0 Hz, 1H), 8,54 (d, *J* = 7,2 Hz, 1H), 8,06 (d, *J* = 2,1 Hz, 1H), 7,77 (dd, *J* = 9,0 Hz, *J* = 2,1 Hz, 1H), 6,90 (d, *J* = 7,2 Hz, 1H), 3,76 (s large, 2H), 3,64-3,44 (m, 4H), 1,91-1.65 (m, 2H + 2H + 14H) |
| 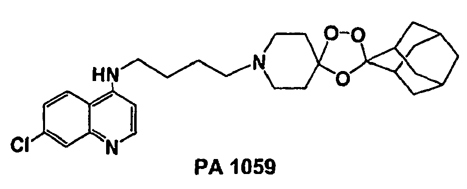 | 138 (déc.) | 300 MHz, 298 K, CDCl₃: 8,54 (d, *J* = 5,4 Hz, 1H), 7,97 (d, *J* = 2,1 Hz, 1H), 7,68 (d, *J* = 8,7 Hz, 1H), 7,37 (dd, *J* = 8,7 Hz et *J* = 2,1 Hz, 1H), 6,42 (d, *J* = 5,4 Hz, 1H), 5,31 (s large, 1H), 3,34 (m, 2H), 2,62 et 2,45 (2m, 2H et 4H), 2,05-1,69 (m, 2H + 2H + 2H + 2H + 14H) |
| 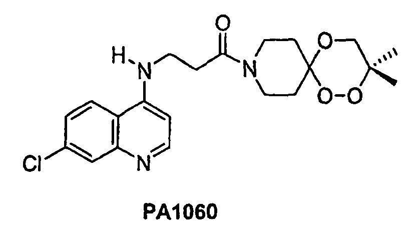 | 184 (déc.) | 300 MHz, 298 K, CDCl₃: 8,54 (d, *J* = 5,4 Hz, 1H), 7,96 (d, *J* = 2,1 Hz, 1H), 7,66 (d, *J* = 9,0 Hz, 1H), 7,37 (dd, *J* = 9,0 Hz, *J* = 2,1 Hz), 6,44 (d, *J* = 5,7 Hz, 1H), 6,15 (t, *J* = 5,4 Hz, 1H), 3,88 (m, 1H), 3,69 (m, 1H + 2H), 3,58-3,37 (m, 4H), 2,74 (t, *J* = 5,7 Hz, 2H), 2,40 (m,1H),1,90 (m, 3H), 1,51 (s, 3H), 1,13 (s, 3H) |
| 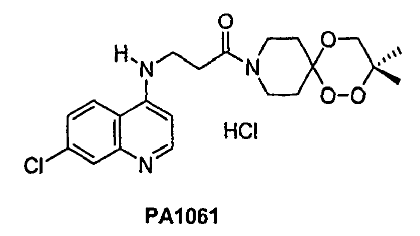 | 68 (déc.) | 300 MHz, 298 K, DMSOd₆: 14,26 (s, 1H), 9,49 (s, 1H), 8,65 (d, *J* = 9,0 Hz), 8,54 (d, *J* = 7,2 Hz), 8,06 (d, *J* = 1,8 Hz, 1H), 7,77 (dd, *J* = 9,3 Hz , *J* = 1,8 Hz, 1H), 6,91 (d, *J* = 7,2 Hz), 3,77-3,71 (m, 1H + 2H), 3,56-3,27 (m, 1H + 4H), 2,85 (t, J= 6,6 Hz, 2H), 2,27-1,59 (m, 4H), 1,37 (s, 3H), 1,08 (s, 3H) |
| 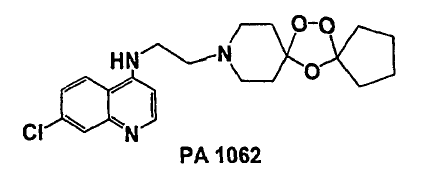 | 148 (déc.) | 300 MHz, 298 K, CDCl₃: 8,52 (d, *J* = 5,4 Hz, 1H), 8,00 (d, *J* = 2,1 Hz, 1H), 7,68 (d, *J* = 9,0 Hz, 1H), 7,40 (dd, *J* = 9,0 Hz, *J* = 2,1 Hz, 1H), 6,39 (d, *J* = 5,4 Hz, 1H), 6,11 (s large, 1H), 3,35 (s large, 2H), 2,83 (m, 2H), 2,72 et 2,67 (2m, 2H et 2H), 1,93 (s large, 2H + 2H + 2H + 2H), 1,67 (m, 2H + 2H) |
| 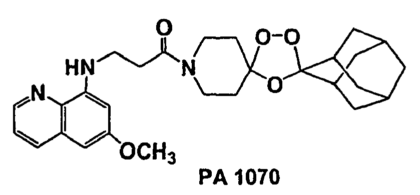 | 69 (déc.) | 300 MHz, 298 K, CDCl₃: 8,56 (dd, *J* = 4,2 Hz, *J* = 1,8 Hz, 1H), 8,00 (dd, *J* = 8,4 Hz, *J* = 1,8 Hz, 1H), 7,35 (dd, *J* = 8,4 Hz, *J* = 4,2 Hz, 1H), 6,54 (s large, 1H), 6,40 (2s, 1H + 1H), 3,91 (s, 3H), 3,87-3,47 (m, 2H + 2H + 2H), 2,80 (t, *J* = 6,6 Hz, 2H), 2,00-1,70 (m, 2H + 2H + 14H) |
| 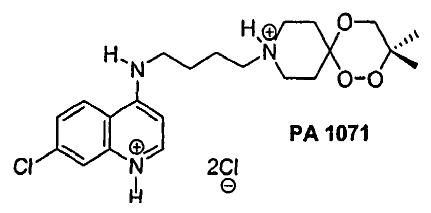 | 125 (déc.) | 300 MHz, 298 K, DMSOd₆: 14,30 (s, 1H), 11,20 (s, 1H), 9,79 (s, 1H), 8,81 (d, *J* = 9,0 Hz), 8,54 (d, *J* = 6,9 Hz), 8,11 (s, 1H), 7,74 (dd, *J* = 8,7 Hz, *J* = 1,5 Hz, 1H), 6,88 (d, *J* = 7,5 Hz), 3,75 (m, 1H), 3,65-3,40 (m, 2H + 1H + 2H), 3,14 (m, 2H), 2,92 (m, 2H), 2,25-1,79 (m, 4H + 2H), 1,75 (m, 2H), 1,38-1,06 (m, 6H) |
| 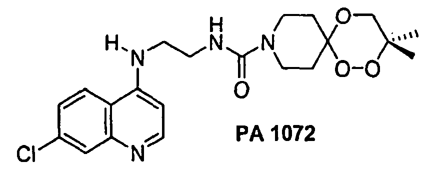 | 192 (déc.) | 300 MHz, 298 K, CDCl₃: 8,48 (d, *J* = 5,4 Hz, 1H), 7,92 (s, 1H), 7,86 (d, *J* = 9,0 Hz, 1H), 7,37 (dd, *J* = 8,7 Hz, *J* = 1,8 Hz), 7,32 (s, 1H), 6,25 (d, *J* = 5,4 Hz, 1H), 5,12 (t, *J* = 5,7 Hz, 1H), 3,74 (m, 1H + 2H), 3,62-3,34 (m, 1H + 2H + 4H), 2,50-1,70 (m, 4H), 1,48 (s, 3H), 1,14 (s, 3H) |
| 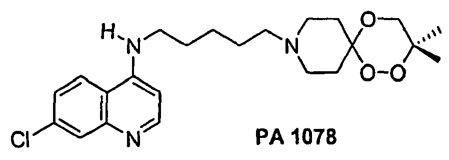 | 134 | 300 MHz, 298 K, CDCl₃: 8,55 (d, J = 5,4 Hz, 1H), 7,97 (d, J = 2,1 Hz, 1H), 7,66 (d, J = 9,0 Hz, 1H), 7,38 (dd, J = 8,7 Hz, J = 2,1 Hz), 6,42 (d, J = 5,4 Hz, 1H), 4,96 (s, 1H), 3,74 (s large, 1H), 3,45 (s large, 1H), 3,33 (q, J = 7,2 Hz), 2,56 (m, 2H), 2,41 (m, 4H), 1,85-1,26 (m, 2H + 2H + 2H + 4H + 3H), 1,13 (s, 3H) |
| 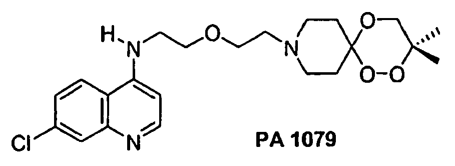 | 95 (déc.) | 300 MHz, 298 K, CDCl₃: 8,55 (d, *J* = 5,4 Hz, 1H), 7,97 (d, *J* = 1,5 Hz, 1H), 7,72 (d, *J* = 9,0 Hz, 1H), 7,40 (dd, *J* = 9,0 Hz, *J* = 1,2 Hz), 6,42 (d, *J* = 5,1 Hz, 1H), 5,50 (s, 1H), 3,80 (m, 1H + 2H), 3,67 (t, *J* = 5,4 Hz), 3,49 (m, 1H + 2H), 2,67 (m, 2H + 2H), 2,48 (m, 2H), 1,85 (m, 4H), 1,49 (s, 3H), 1,12 (s, 3H) |
| 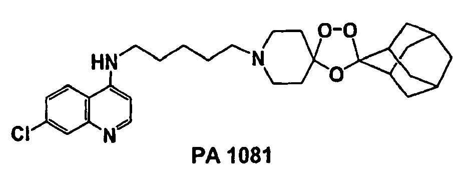 | 146 (déc.) | 250 MHz, 298 K, CDCl₃: 8,52 (d, *J* = 5,4 Hz, 1H), 7,96 (d, *J* = 2,1 Hz, 1H), 7,69 (d, *J* = 9,0 Hz, 1H), 7,37 (dd, *J* = 9,0 Hz, *J* = 2,1 Hz, 1H), 6,40 (d, *J* = 5,4 Hz, 1H), 5,10 (s large, 1H), 3,32 (m, 2H), 2,61-2,42 (m, 2H + 2H + 2H), 2,02-1,49 (m, 2H + 2H + 2H + 2H + 2H + 14H) |
| 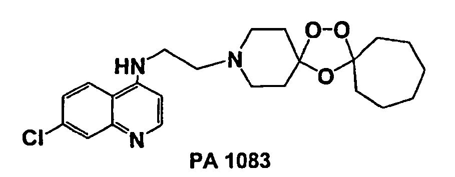 | 147 (déc.) | 250 MHz, 298 K, CDCl₃: 8,52 (d, *J* = 5,4 Hz, 1H), 7,99 (d, *J* = 2,1 Hz, 1H), 7,64 (d, *J* = 8,9 Hz, 1H), 7,39 (dd, *J* = 8,9 Hz, *J* = 2,1 Hz, 1H), 6,38 (d, *J* = 5,4 Hz, 1H), 6,06 (s large, 1H), 3,32 (m, 2H), 2,80 (m, 2H), 2,68 et 2,54 (2m, 2H et 2H), 1,91 (m, 2H + 2H + 2H + 2H), 1,67 (s large, 2H + 2H + 2H + 2H) |
| 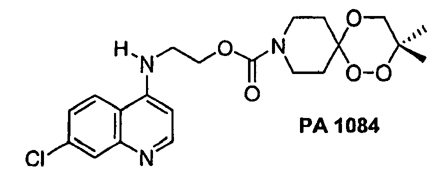 | 166 (déc.) | 300 MHz, 298 K, CDCl₃: 8,55 (d, *J* = 5,1 Hz, 1H), 7,96 (d, *J* = 2,1 Hz, 1H), 7,73 (d, *J* = 9.0 Hz, 1H), 7,39 (dd, *J* = 9,0 Hz, *J* = 2,1 Hz), 6,37 (d, *J* = 5,4 Hz, 1H), 6,33 (s, 1H), 4,56 (m, 2H), 3,79-3,62 (m, 1H + 2H), 3,54 (q, *J* = 4,5 Hz), 3,50-3,46 (m, 1H + 2H), 2,35 (m, 1H), 1,83-1,65 (m, 3H), 1,51 (s, 3H), 1,13 (s, 3H) |
| 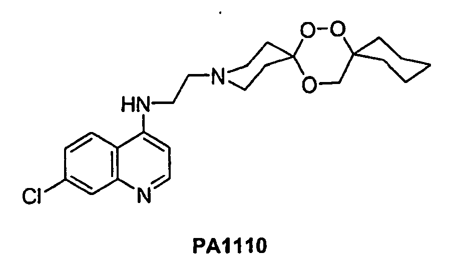 | 151 (déc.) | 300 MHz, 298 K, CDCl₃: 8,54 (d, *J* = 5,4 Hz, 1H), 7,93 (d, *J* = 2,1 Hz, 1H), 7,68 (d, *J* = 9,0 Hz, 1H), 7,40 (dd, *J* = 2,1 Hz, *J* = 8,7 Hz), 6,39 (d, *J* = 5,6 Hz, 1H), 5,97 (s, 1H), 3,78-3,48 (m, 2H), 3,33 (q, *J* = 5,4 Hz, 2H), 2,81 (t, *J* = 6,3 Hz), 2,70-2,48 (m, 4H), 2,20-1,20 (m, 4H + 10H + 3H + 3H) |
| 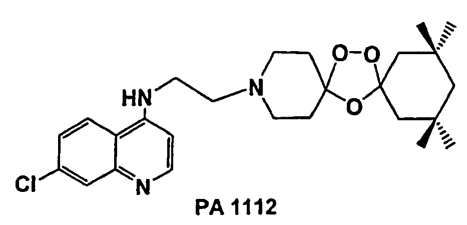 | 179 (déc.) | 300 MHz, 298 K, CDCl₃: 8,55 (d, *J* = 5,4 Hz, 1H), 8,00 (d, *J* = 2,4 Hz, 1H), 7,67 (d, *J* = 9,0 Hz, 1H), 7,41 (dd, *J* = 9,0 Hz, *J* = 2,4 Hz, 1H), 6,39 (d, *J* = 5,4 Hz, 1H), 5,99 (s large, 1H), 3,33 (m, 2H), 2,82 (m, 2H), 2,64 (m, 2H + 2H), 1,94 (t, *J* = 5,7 Hz, 4H), 1,68 et 1,56 (2d, *J* = 13,5 Hz, 2H et 2H), 1,32 (d, *J* = 13,5 Hz, 1H), 1,22 (d, *J* = 13,5 Hz, 1H), 1,06 (s, 6H), 1,04 (s, 6H) |
| 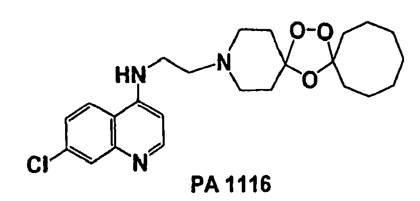 | 155 (déc.) | 300 MHz, 298 K, CDCl₃: 8,55 (d, *J* = 5,1 Hz, 1H), 7,99 (d, *J* = 2,1 Hz, 1H), 7,65 (d, *J* = 9,0 Hz, 1H), 7,40 (dd, *J* = 9,0 Hz, *J* = 2,1 Hz, 1H), 6,39 (d, *J* = 5,1 Hz, 1H), 5,98 (s large, 1H), 3,32 (m, 2H), 2,82 (m, 2H), 2,72 et 2,58 (2m, 2H et 2H), 1,98 (m, 2H + 2H + 2H + 2H), 1,60 (m, 2H + 2H + 2H + 2H + 2H) |
| 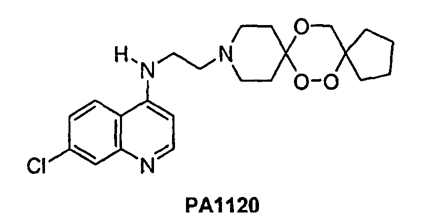 | 145 | 300 MHz, 298 K, CDCl₃: 8,55 (d, *J* = 5,4 Hz, 1H), 7,98 (d, *J* = 2,1 Hz), 7,67 (d, *J* = 9,0 Hz, 1H), 7,40 (dd, *J* = 9,0 Hz, *J* = 2,4 Hz), 6,39 (d, *J* = 5,4 Hz, 1H), 5,99 (s, 1H), 4,02 (s large, 1H), 3,53 (s large, 1H), 3,33 (q, *J* = 5,4 Hz, 2H), 2,81 (t, *J* = 6,3 Hz, 2H), 2,70-2,40 (m, 4H), 2,13 (m, 4H + 8H) |
| 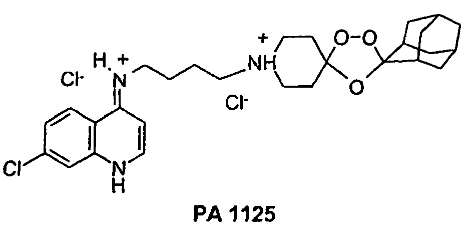 | 114(déc.) | 300 MHz, 298 K, DMSOd₆: 14,33 (s large, 1H), 11,17 (s large, 1H), 9,72 (s large, 1H), 8,77 (d, *J* = 9,0 Hz, 1H), 8,56 (d, *J* = 7,2 Hz, 1H), 8,07 (d, *J* = 1,8 Hz, 1H), 7,77 (dd, *J* = 9,0 Hz, *J* = 1,8 Hz, 1H), 6,91 (d, *J* = 7,2 Hz, 1H), 3,56 (s large, 2H), 3,45 et 3,00 (2m, 2H et 2H), 3,16 (s large, 2H), 2,36-1,66 (m, 2H + 2H + 2H + 2H + 14H) |
| 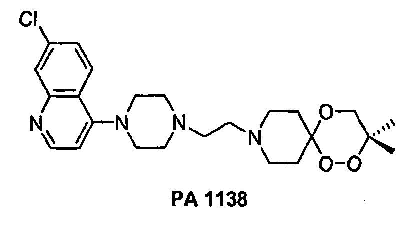 | n.d. | 300MHz, 298 K, CDCl₃: 8,72 (d, *J* = 5,1 Hz, 1H), 8,05 (d, *J* = 2,1 Hz, 1H), 7,95 (d, *J* = 9,0 Hz, 1H), 7,43 (dd, *J* = 9,0 Hz, *J* = 2,1 Hz, 1H), 6,84 (d, *J* = 5,1 Hz, 1H), 3,81 (s large, 1H), 3,46 (s large, 1H), 3,27 (m, 2H), 2,88-2,80 (m, 4H), 2,72-2,63 (m, 6H), 2,53-2,40 (m, 4H), 2,19-1,80 (m, 4H), 1,52 (s large, 3H), 1,13 (s large, 3H) |
| 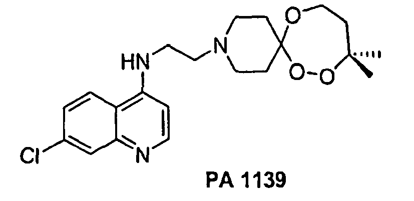 | 141 | 300MHz, 298 K, CDCl₃: 8,52 (d, *J* = 5,7 Hz, 1H), 8,03 (d, *J* = 2,1 Hz, 1H), 7,72 (d, *J* = 8,7 Hz, 1H), 7,41 (dd, *J* = 9,0 Hz, *J* = 2,1 Hz, 1H), 6,39 (d, *J* = 5,7 Hz, 1H), 6,32 (s large, 1H), 4,09-3,93 (m, 1H), 3,78-3,71 (m, 1H), 3,36 (t, *J* = 5,7 Hz, 2H), 2,84 (t, *J* = 6,3 Hz, 2H), 2,67-2,51 (m, 4H), 2,17 (m, 1H), 2,03-1,87 (m, 2H), 1,79-1,70 (m, 3H), 1,42 (s, 3H), 1,17 (s, 3H) |
| 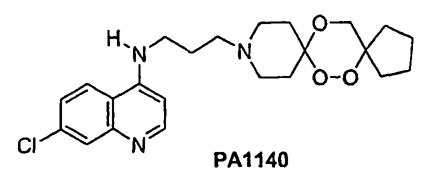 | 151 | 300MHz, 298 K, CDCl₃: 8,50 (d, *J* = 5,4 Hz, 1H), 7,98 (d, *J* = 2,1 Hz, 1H), 7,78 (d, *J* = 9,0 Hz, 1H), 7,73 (s large, 1H), 7,38 (dd, *J* = 9,0 Hz, *J* = 2,1 Hz, 1H), 6,34 (d, *J* = 5,4 Hz, 1H), 4,00 (m, 1H), 3,55 (m, 1H), 3,41 (q, *J* = 5,7 Hz, 2H), 2,77-2,40 (m, 6H + 2H), 2,20-1,44 (m, 8H + 2H + 2H) |
| 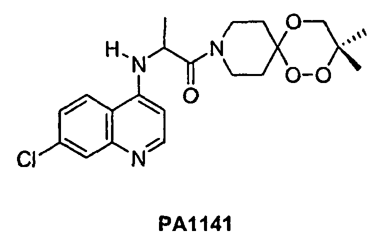 | 176 | 300MHz, 298 K, CDCl₃: 8,52 (d, *J* = 5,4 Hz, 1H), 7,97 (d, *J* = 2,1 Hz, 1H), 7,82 (d, *J* = 9,0 Hz, 1H), 7,40 (dd, *J* = 8,7 Hz, *J* = 2,1 Hz, 1H), 6,56 (d, *J* = 6,6 Hz, 1H), 6,34 (d, *J* = 5,4 Hz, 1H), 4,59 (qd, *J* = 6,6 Hz, 1H), 4,04-3,47 (m, 2H + 4H), 2,50 (m, 1H), 2,18-1,86 (m, 3H), 1,56 (s large, 3H), 1,51 (d, *J* = 6,3 Hz, 3H), 1,23 (s large, 3H) |
| 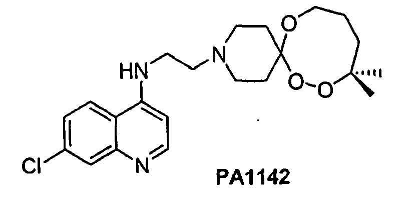 | 112 | 300MHz, 298 K, CDCl₃: 8,52 (d, *J* = 5,4 Hz, 1H), 8,03 (m, 1H), 7,73 (dd, *J* = 3,0 Hz, *J* = 9,0 Hz, 1H), 7,42 (dd, *J* = 9,0 Hz, *J* = 2,1 Hz, 1H), 6,40 (d, *J* = 5,7 Hz, 1H), 6,40 (s large, 1H), 3,95-3,90 (m, 1H), 3,72-3,62 (m, 1H), 3,37 (s, 2H), 2,86 (t, *J* = 5,1 Hz, 2H), 2,69-2,60 (m, 4H), 2,15-2,00 (m, 3H), 1,82 (t, *J* = 5,7 Hz, 2H), 1,73 (t, *J* = 4,2 Hz, 2H), 1,64-1,58 (m, 1H), 1,34 (s, 3H), 1,14 (s, 3H) |
| 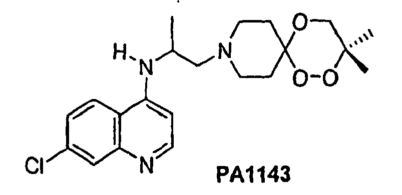 | 149 (déc.) | 300MHz, 298 K, CDCl₃: 8,52 (d, *J* = 5,4 Hz, 1H), 8,04 (s, 1H), 7,77 (d, *J* = 9,0 Hz, 1H), 7,42 (dd, *J* = 9,0 Hz, *J* = 2,4 Hz, 1H), 6,47 (d, *J* = 5,7 Hz, 1H), 6,24 (s large, 1H), 3,81-3,68 (m, 1H + 1H), 3,50 (m, 1H), 2,74-2,38 (m, 6H + 2H), 2,00-1,70 (m, 2H), 1,50 (s large, 3H), 1,35 (d, *J* = 6,0 Hz, 3H), 1,12 (s large, 3H) |

L'étude des propriétés pharmacologiques des produits de couplage de formule (I) selon l'invention a montré qu'ils présentent une activité antipaludique.

L'obtention d'un tel effet est d'autant plus avantageux que les phénomènes de résistance de souches de *Plasmodium falciparum,* l'espèce mortelle, vis-à-vis des médicaments antimalariques usuels se développent et que, de plus, la protection vaccinale, pour laquelle d'importantes recherches sont effectuées, ne pourra être réalisée avant plusieurs années.

### Etude de l'activité antipaludique des molécules duales selon l'invention sur P. falciparum

Sont rapportés ci-après les résultats obtenus *in vitro* sur *P. falciparum* cultivé dans des hématies humaines.

### 1. Culture de P. falciparum

Les souches de *P. falciparum* sont cultivées en continu selon la méthode de Trager et Jensen (Science, 1976, 193, 673-675) : les parasites sont maintenus dans des globules rouges humains (O±), dilués à 2 % de parasitémie dans un milieu RPMI 1640 supplémenté avec 25 mM d'Hépes + 24 mM NaHCO₃ + 2 mM L-glutamine et complémenté avec 5 % de sérum humain de tous groupes. Les parasites sont incubés à 37°C, en atmosphère humide et à 5 % de CO₂. Les souches FcB1-Columbia et FcM29-Cameroon sont respectivement moyennement (CL₅₀ : 66 nM) et très fortement (CI₅₀ : 258 nM) chloroquino-résistantes. Les CI₅₀ de l'artémisinine sur ces 2 souches sont respectivement de 11 nM et 5 nM.

### 2. Test de chimiosensibilité

Les tests d'activité antipaludique sont effectués selon la microméthode radioactive de Desjardins et al. (Antimicrob. Agents Chemother., 1979, 16, 710-718). Chaque molécule est testée en triple exemplaire. Les essais sont réalisés dans des microplaques de 96 puits. Les souches de *P. falciparum* sont mises en culture dans des solutions de RPMI 1640 complémenté avec 5 % de sérum humain avec un hématocrite à 2 % et une parasitémie à 1,5 %. Pour chaque essai, les parasites sont incubés avec des concentrations décroissantes des composés à tester pendant 48 h à 37°C, en atmosphère humide et à 5 % de CO₂. L'artémisinine et la chloroquine di-phosphate sont utilisées comme molécules de référence. La première dilution des composés à tester est réalisée à 1 mg/mL dans du diméthylsulfoxyde. La gamme de dilution des solutions filles successives est également réalisée dans du diméthylsulfoxyde. Chaque dilution fille est ensuite diluée au 1/50^{ème} dans du RPMI 1640 complémenté avec 5 % de sérum humain, l'ensemble des dilutions étant réalisé à 37°C. Ces dilutions sont ensuite ajoutées aux parasites en culture dans les microplaques. Après ajout du composé à tester, les parasites sont mis en culture dans du RPMI 1640 à 5 % de sérum humain et à 1 % de diméthylsulfoxyde. La croissance des parasites est mesurée par l'incorporation d'hypoxanthine tritiée (ajoutée 24 h après le début de l'exposition au composé à tester) et comparée à l'incorporation en l'absence du composé à tester (prise comme 100 %). Les valeurs de CI₅₀ (concentrations nécessaires pour inhiber de 50% la croissance du parasite) sont déterminées en traçant le pourcentage d'inhibition en fonction du logarithme de la dose à l'aide du logiciel de traitement GraphPad Prism 4® (GraphPad software, Inc., 5755 Oberlin Drive, #110, San Diego, CA 92121, USA).

### 3. Résultats

Les CI₅₀ des composés de formule (I) selon l'invention sont inférieures à 1 µM. Sur les souches utilisées, ces CI₅₀ sont, pour la plupart des composés de formule (I) testés, comparables à celles de l'artémisinine, voire meilleures.

On ne mesure pas de différence notable entre les CI₅₀ des composés testés sur l'une ou l'autre des souches, à savoir sur la souche FcB1-Colombia (souche moyennement résistante à la chloroquine) et sur la souche FcM29-Cameroon (souche fortement résistante à la chloroquine).

A titre d'exemples, les CI₅₀ des composés PA1011, PA1021 et PA1026 sur la souche FcM29-Cameroon sont respectivement égales à 13 nM, 6 nM et 4,4 nM.

L'invention vise la mise à profit des propriétés de ces produits de couplage pour leur utilisation en tant que médicament et pour l'élaboration de compositions pharmaceutiques à propriétés antipaludiques.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, dans la prévention et le traitement du paludisme.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé de formule (I) selon l'invention, ou un sel pharmaceutiquement acceptable, un hydrate ou solvat dudit composé, ainsi qu'au moins un excipient pharmaceutiquement acceptable. Lesdits excipients sont choisis selon la forme pharmaceutique et le mode d'administration souhaité, parmi les excipients habituels qui sont connus de l'Homme de l'art.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, pour la prévention ou le traitement du paludisme.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions. Des voies d'administration préférées sont les voies orale, rectale et injectable.

A titre d'exemple, une forme unitaire d'administration d'un composé selon l'invention sous forme de comprimé peut comprendre les composants suivants :

| Composé selon l'invention | 50,0 mg |
|---|---|
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

II peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement ou de prévention du paludisme qui comprend l'administration, à un patient, d'une dose efficace d'un composé de formule (I) selon l'invention, ou un de ses sels pharmaceutiquement acceptables ou hydrates ou solvats.

L'invention vise encore les réactifs biologiques dont les principes actifs sont constitués par les composés selon l'invention. Ces réactifs peuvent être utilisés comme références ou étalons dans des études d'éventuelles activités antipaludiques.

## Revendications

1. Composé répondant à la formule (I) : 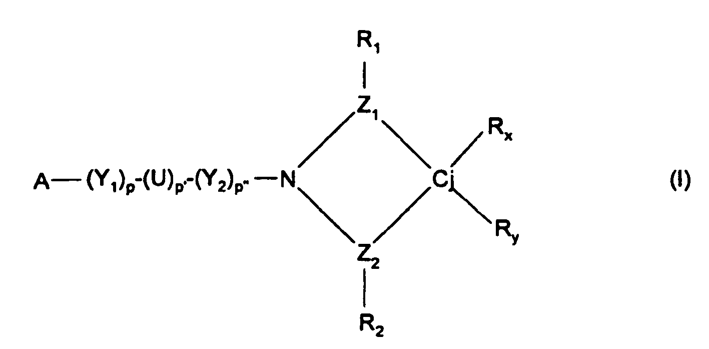 dans laquelle:
- A représente un résidu de molécule à activité antipaludique et/ou un résidu facilitant la biodisponibilité, choisi parmi :
■ Un hétérocycle azoté de formule (II) : 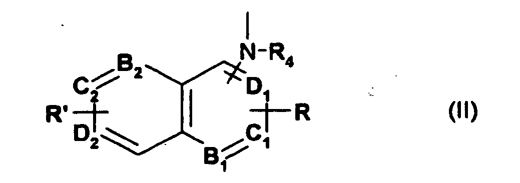
dans laquelle :
- R et R', identiques ou différents, représentent chacun un ou plusieurs substituants occupant des positions distinctes sur les cycles auxquels ils sont rattachés, choisis parmi les atomes d'hydrogène ou d'halogène et les groupes -OH, -CF₃, aryle, hétéroaryle, alkyle ou -O-alkyle, -NO₂ ou -N(Rₐ,R_{b}), où Rₐ et R_{b}, identiques ou différents, représentent des atomes d'hydrogène ou des groupes alkyles comprenant de 1 à 5 atomes de carbone et étant linéaires, ramifiés ou cycliques; l'un au moins de R ou R' étant différent d'un atome d'hydrogène ;
- R₄ représente un atome d'hydrogène ou un radical alkyle en C1 à C5, linéaire, ramifié ou cyclique, ou R₄ forme, avec un atome de carbone ou d'azote présent dans le groupement -(Y₁)ₚ-(U)_{p'}-(Y₂)_{p"}- de la formule (I), un cycle comportant de 5 à 8 chaînons,
- les radicaux B₁, C₁, D₁, B₂, C₂ et D₂ représentent des atomes d'azote ou des chaînons -CH=, étant donné que soit l'un des radicaux B₁, C₁ et D₁ représente un atome d'azote et les autres radicaux représentent des chaînons -CH=, soit l'un des radicaux B₂, C₂ et D₂ représente un atome d'azote et les autres radicaux représentent des chaînons
- CH=, soit l'un des radicaux B₁, C₁ et D₁ et l'un des radicaux B₂, C₂ et D₂ représentent des atomes d'azote et les autres radicaux représentent des chaînons -CH=,
■ un résidu de formule (IV), (V), (VI), (VII), (VIII), (IX) ou (X), dans lesquelles R, R', Rₐ et R_{b} sont tels que définis dans la revendication pour le composé de formule (II) et R₅ représente un radical aryle ou un résidu hétérocyclique azoté éventuellement substitué par un ou plusieurs groupes R :
R₅-CHOH- (IV) 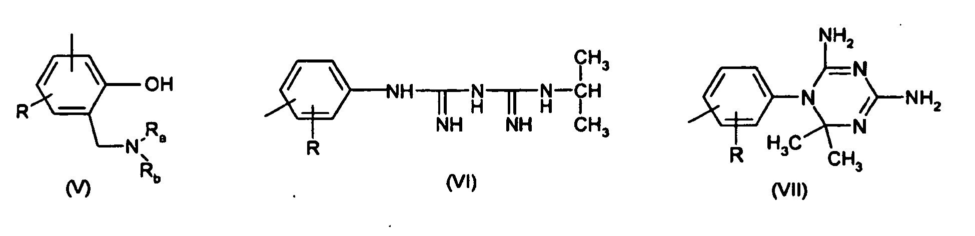 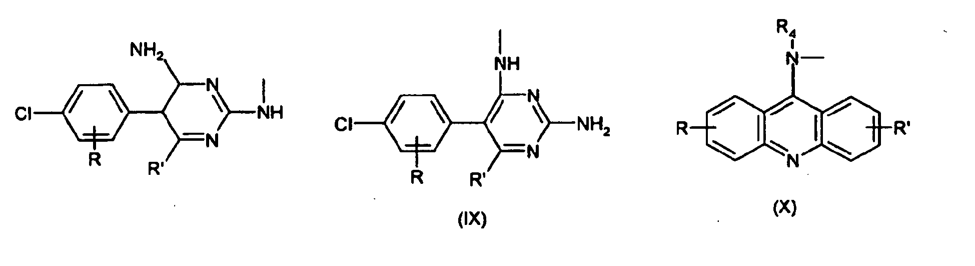
- p, p', p" représentent indépendamment les uns des autres 0 ou 1, l'un au moins de p ou de p" étant égal à 1,
- Y₁ et Y₂, identiques ou différents, représentent une chaîne alkylène en C1 à C6, linéaire, ramifiée ou cyclique, comportant éventuellement un ou plusieurs radicaux amine, amide, thioamide, sulfonyle, sulfonate, sulfonamide, carbonyle, thiocarbonyle, carboxyle, thiocarboxyle, éther ou thioéther, cette chaîne alkylène étant éventuellement substituée par un ou plusieurs groupes choisis parmi les atomes d'halogène, les groupes hydroxyles, les groupes acétals et les radicaux alkyles en C1 à C5, linéaires, ramifiés ou cycliques,
- U représente un radical amine, amide, thioamide, sulfonyle, sulfonate, sulfonamide, carbonyle, thiocarbonyte, carboxyle, thiocarboxyle, éther ou thioéther,
- Z₁ et Z₂, identiques ou différents, représentent un radical alkylène en C1 à C4 linéaire, ramifié ou cyclique, saturé ou insaturé, l'un de Z₁ ou Z₂ pouvant être absent, ou l'ensemble Z₁ + Z₂ représente une structure polycyclique incluant N et le carbone de jonction Cj,
- R₁ et R₂, identiques ou différents, représentent un atome d'hydrogène ou un groupe fonctionnel capable d'augmenter l'hydrosolubilité de la molécule duale,
- Rₓ et R_{y} forment ensemble un peroxyde cyclique comprenant de 4 à 8 chaînons et comportant 1 ou 2 atomes d'oxygène supplémentaires dans la structure cyclique, Cj étant l'un des sommets de ce peroxyde cyclique,
. ledit peroxyde cyclique étant substitué par 1 à 8 groupes R₃, identiques ou différents les uns des autres, occupant des positions quelconques sur les atomes de carbone du cycle peroxydique et étant choisis parmi les atomes et groupes suivants :
hydrogène, halogène, -OH, -CF₃, -NO₂, aryle ou hétéroaryle, alkyle ou -O-alkyle, lesdits groupes alkyles comprenant de 1 à 10 atomes de carbone et étant linéaires, ramifiés ou cycliques, ou
structure mono-, bi- ou tricyclique en C3 à C18 pouvant en outre contenir 1 ou plusieurs hétéroatomes choisis parmi l'oxygène, l'azote et le soufre, éventuellement substituée par un ou plusieurs groupes choisis parmi les atomes d'halogène, les groupes hydroxyles et les groupes alkyles en C1 à C6, linéaires, ramifiés ou cycliques,
. l'un au moins des groupes R₃ étant différent d'un atome d'hydrogène,
. deux groupes R₃ portés par des atomes de carbone adjacents sur le cycle peroxydique pouvant former ensemble une structure cyclique à 5 ou 6 chaînons, saturée ou insaturée, éventuellement substituée en positions quelconques par un ou plusieurs substituants R₃ tels que définis ci-dessus,
. deux groupes R₃ portés par le même atome de carbone du cycle peroxydique pouvant former ensemble une structure mono-, bi- ou tricyclique telle que définie ci-dessus,
à l'état de base ou de sel d'addition à un acide, ainsi qu'à l'état d'hydrate ou de solvat.

2. Composé de formule (1) selon la revendication 1, **caractérisé en ce que** A représente une aminoquinoléine de formule (IIa) ou une 1,5-naphtyridine de formule (III) : 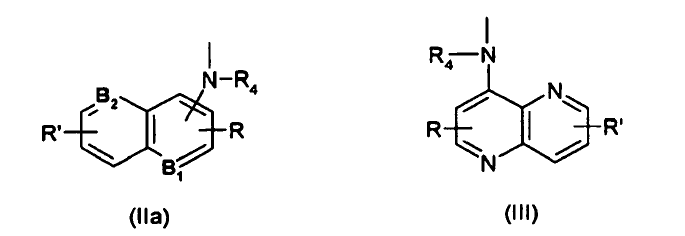 dans lesquelles R, R' et R₄ sont tels que définis dans la revendication 3 et soit B₁ représente un atome d'azote et B₂ représente un chaînon -CH=, soit B₁ représente un chaînon -CH= et B₂ représente un atome d'azote,
à l'état de base ou de sel d'addition à un acide, ainsi qu'à l'état d'hydrate ou de solvat.

3. Composé de formule (I) selon l'une quelconque des revendications précédentes, **caractérisé en ce que** A représente une aminoquinoléine de formule (IIb) ou (IIc) : 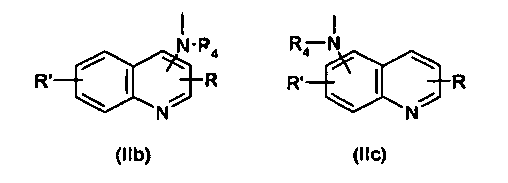 dans lesquelles R, R' et R₄ sont tels que définis dans la revendication 1,
à l'état de base ou de sel d'addition à un acide, ainsi qu'à l'état d'hydrate ou de solvat.

4. Composé de formule (I) selon l'une quelconque des revendications précédentes, **caractérisé en ce que** Rₓ et R_{y} forment ensemble un peroxyde cyclique comprenant 5 ou 6 chaînons et comportant 1 atome d'oxygène supplémentaire dans la structure cyclique, Cj étant l'un des sommets de ce peroxyde cyclique, ledit peroxyde cyclique étant substitué par 1 à 4 groupes R₃, identiques ou différents, tels que définis dans la revendication 1,
à l'état de base ou de sel d'addition à un acide, ainsi qu'à l'état d'hydrate ou de solvat.

5. Composé de formule (I) selon la revendication 4, **caractérisé en ce que** Rₓ et R_{y} forment ensemble un peroxyde cyclique répondant à la formule (XI), dans laquelle R₃ représente 1 à 4 groupes, identiques ou différents, tels que définis dans la revendication 1 : 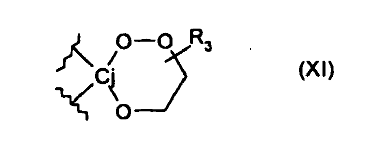 à l'état de base ou de sel d'addition à un acide, ainsi qu'à l'état d'hydrate ou de solvat.

6. Composé de formule (I) selon la revendication 5, **caractérisé en ce que** Rₓ et R_{y} forment ensemble un peroxyde cyclique répondant à la formule (XIa) : 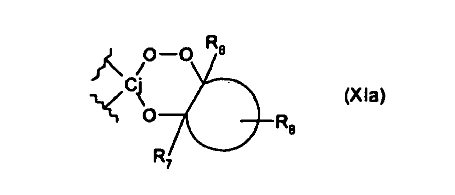 dans laquelle l'arc de cercle représente une structure cyclique à 5 ou 6 chaînons,
saturée ou insaturée, et R₆, R₇ et R₈, identiques ou différents les uns des autres, sont choisis parmi les atomes et groupes suivants : hydrogène, halogène, -OH, -CF₃, -NO₂,
aryle ou hétéroaryle, alkyle ou -O-alkyle, lesdits groupes alkyles comprenant de 1 à 10 atomes de carbone et étant linéaires, ramifiés ou cycliques,
à l'état de base ou de sel d'addition à un acide, ainsi qu'à l'état d'hydrate ou de solvat.

7. Composé de formule (I) selon la revendication 5 ou la revendication 6, **caractérisé en ce que** Rₓ et R_{y} forment ensemble un peroxyde cyclique répondant à la formule (XIb) : 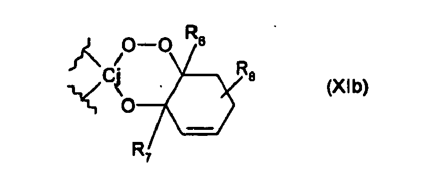 dans laquelle R₆, R₇ et R₈ sont tels que définis dans la revendication 6,
à l'état de base ou de sel d'addition à un acide, ainsi qu'à l'état d'hydrate ou de solvat.

8. Composé de formule (I) selon la revendication 5, **caractérisé en ce que** Rₓ et R_{y} forment ensemble un peroxyde cyclique répondant à la formule (XIc) : 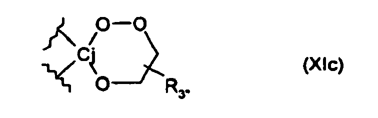 dans laquelle R_{3"} représente 1 à 4 groupes, identiques ou différents les uns des autres, occupant des positions quelconques sur les atomes de carbone du cycle peroxydique et étant choisis parmi les atomes et groupes suivants :
hydrogène, halogène, -OH, -CF₃, -NO₂, aryle ou hétéroaryle, alkyle ou -O-alkyle, lesdits groupes alkyles comprenant de 1 à 10 atomes de carbone et étant linéaires, ramifiés ou cycliques, ou
structure mono-, bi- ou tricyclique en C3 à C18 pouvant en outre contenir 1 ou plusieurs hétéroatomes choisis parmi l'oxygène, l'azote et le soufre, éventuellement substituée par un ou plusieurs groupes choisis parmi les atomes d'halogène, les groupes hydroxyles et les groupes alkyles en C1 à C6, linéaires, ramifiés ou cycliques,
. l'un au moins des groupes R_{3"} étant différent d'un atome d'hydrogène,
. deux groupes R_{3"} portés par le même atome de carbone du cycle peroxydique pouvant former ensemble une structure mono-, bi- ou tricyclique telle que définie ci-dessus,
à l'état de base ou de sel d'addition à un acide, ainsi qu'à l'état d'hydrate ou de solvat.

9. Composé de formule (I) selon la revendication 4, **caractérisé en ce que** Rₓ et R_{y} forment ensemble un peroxyde cyclique répondant à la formule (XII) : 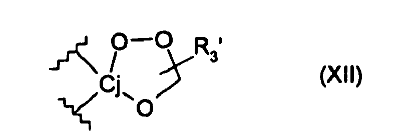 dans laquelle R₃. représente 1 ou 2 groupes, identiques ou différents, choisis parmi les atomes et groupes suivants :
hydrogène, halogène, -OH, -CF₃, -NO₂, aryle ou hétéroaryle, alkyle ou -O-alkyle, lesdits groupes alkyles comprenant de 1 à 10 atomes de carbone et étant linéaires, ramifiés ou cycliques, ou
structure mono-, bi- ou tricyclique en C3 à C18 pouvant en outre contenir 1 ou plusieurs hétéroatomes choisis parmi l'oxygène, l'azote et le soufre, éventuellement substituée par un ou plusieurs groupes choisis parmi les atomes d'halogène, les groupes hydroxyles et les groupes alkyles en C1 à C6, linéaires, ramifiés ou cycliques,
. l'un au moins des groupes R_{3'} étant différent d'un atome d'hydrogène,
. deux groupes R_{3'} portés par le même atome de carbone du cycle peroxydique pouvant former ensemble une structure mono-, bi- ou tricyclique telle que définie ci-dessus,
à l'état de base ou de sel d'addition à un acide, ainsi qu'à l'état d'hydrate ou de solvat.

10. Composé de formule (I) selon l'une quelconque des revendications 1, 2, 4 et 5, **caractérisé en ce qu'**il répond à la formule (XIII) : 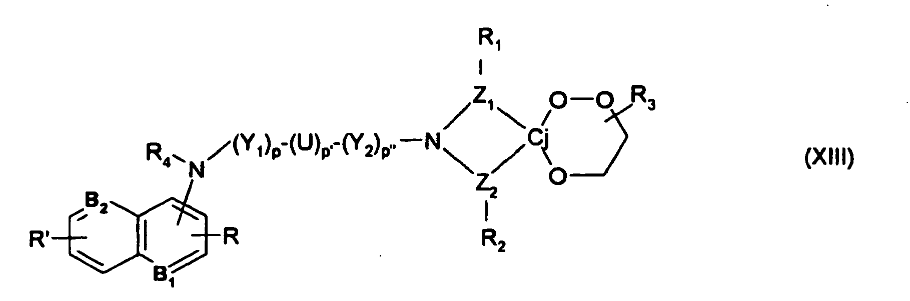 dans laquelle R, R', B₁, B₂, Y₁, U, Y₂, P, P', p", Z₁, Z₂, Cⱼ, R₁, R₂, R₃ et R₄ sont tels que définis dans l'une quelconque des revendications 1, 2, 4 et 5,
à l'état de base ou de sel d'addition à un acide, ainsi qu'à l'état d'hydrate ou de solvat.

11. Composé de formule (I) selon l'une quelconque des revendications 1, 2, 4 et 9, **caractérisé en ce qu'**il répond à la formule (XIV) : 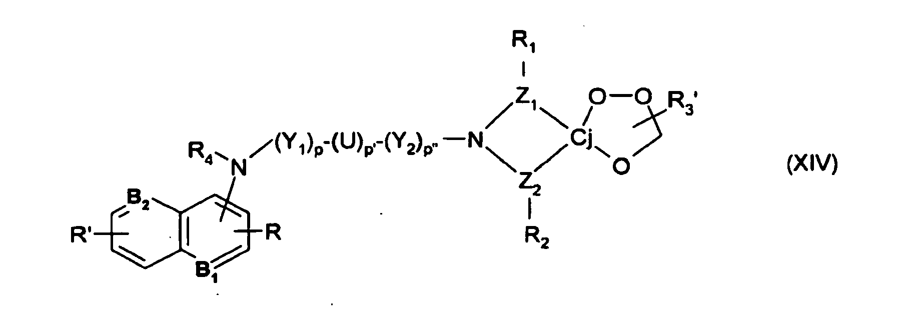 dans laquelle R, R', B₁, B₂, Y₁, U, Y₂, p, p', p", Z₁, Z₂, Cⱼ, R₁, R₂, R₃, et R₄ sont tels que définis dans l'une quelconque des revendications 1, 2, 4 et 9,
à l'état de base ou de sel d'addition à un acide, ainsi qu'à l'état d'hydrate ou de solvat.

12. Composé de formule (I) selon l'une quelconque des revendications précédentes, **caractérisé en ce que** :
- Y₁ et Y₂, identiques ou différents, représentent une chaîne alkylène en C1 à C6, linéaire, ramifiée ou cyclique, comportant éventuellement un ou plusieurs radicaux amine, amide, carbonyle ou éther, cette chaîne alkylène en C1 à C6 étant éventuellement substituée par un ou plusieurs groupes choisis parmi les atomes d'halogène, les groupes hydroxyles, les groupes acétals et les radicaux alkyles en C1 à C5, linéaires, ramifiés ou cycliques,
- U représente un radical amine, amide, carbonyle, carboxyle ou éther,
à l'état de base ou de sel d'addition à un acide, ainsi qu'à l'état d'hydrate ou de solvat.

13. Composé de formule (I) selon la revendication 12, **caractérisé en ce que** :
- soit p' = p" = 0, p = 1 et Y₁ représente une chaîne alkylène en C1 à C6, linéaire ou ramifiée, éventuellement substituée par un ou plusieurs groupes choisis parmi les atomes d'halogène, les groupes hydroxyles, les groupes acétals et les radicaux alkyles en C1 à C5, linéaires, ramifiés ou cycliques,
- soit p = p' = 1, p" = 0, Y₁ représente une chaîne alkylène en C1 à C6, linéaire ou ramifiée, éventuellement substituée par un ou plusieurs groupes choisis parmi les atomes d'halogène, les groupes hydroxyles, les groupes acétals et les radicaux alkyles en C1 à C5, linéaires, ramifiés ou cycliques, et U représente un radical amine, amide, carbonyle, carboxyle ou éther,
- soit p = p' = p" = 1, Y₁ et Y₂ représentent des chaînes alkylènes en C1 à C6, linéaires ou ramifiées, éventuellement substituées par un ou plusieurs groupes choisis parmi les atomes d'halogène, les groupes hydroxyles, les groupes acétals et les radicaux alkyles en C1 à C5, linéaires, ramifiés ou cycliques, et U représente un radical amine, amide, carbonyle, carboxyle ou éther,
à l'état de base ou de sel d'addition à un acide, ainsi qu'à l'état d'hydrate ou de solvat.

14. Composé de formule (I) selon l'une quelconque des revendications précédentes, **caractérisé en ce que** Z₁ et Z₂, identiques ou différents, représentent chacun un radical alkylène en C1 à C4, linéaire ou ramifié,
à l'état de base ou de sel d'addition à un acide, ainsi qu'à l'état d'hydrate ou de solvat.

15. Composé de formule (I) selon l'une quelconque des revendications 1, 2, 4, 5 et 10, **caractérisé en ce qu'**il répond à la formule (XV) : 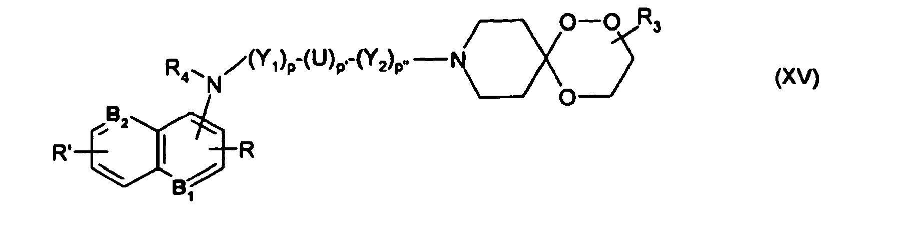 dans laquelle R, R', B₁, B₂, Y₁, U, Y₂, p, p', p", R₁, R₂, R₃ et R₄ sont tels que définis dans l'une quelconque des revendications 1, 2, 4, 5 et 10,
à l'état de base ou de sel d'addition à un acide, ainsi qu'à l'état d'hydrate ou de solvat.

16. Composé de formule (I) selon l'une quelconque des revendications 1, 2, 4, 9, et 11, **caractérisé en ce qu'**il répond à la formule (XVI) : 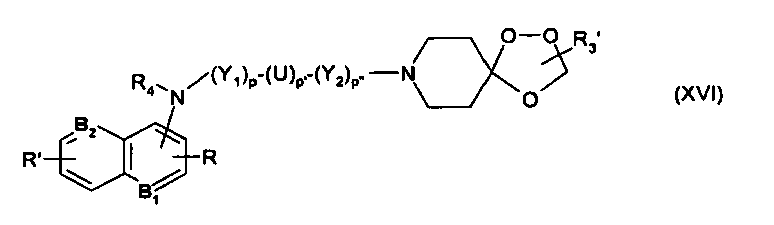 dans laquelle R, R', B₁, B₂, Y₁, U, Y₂, p, p', p", R₁, R₂, R₃, et R₄ sont tels que définis dans l'une quelconque des revendications 1, 2, 4, 9, et 11,
à l'état de base ou de sel d'addition à un acide, ainsi qu'à l'état d'hydrate ou de solvat.

17. Composé de formule (I) selon l'une quelconque des revendications précédentes, **caractérisé en ce qu'**il est choisi parmi les composés suivants :

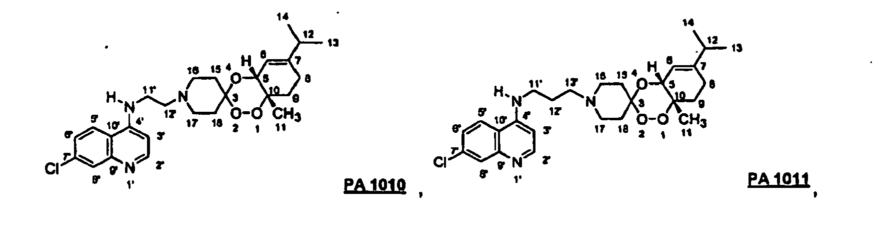

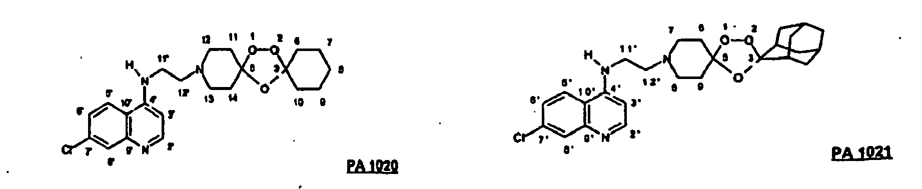

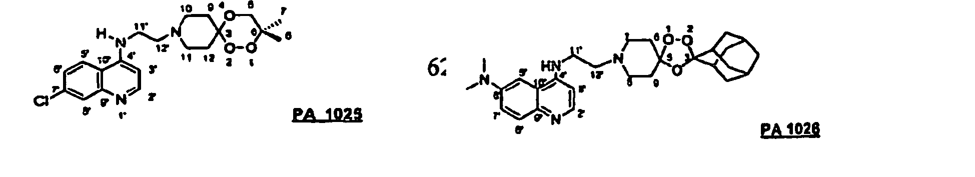

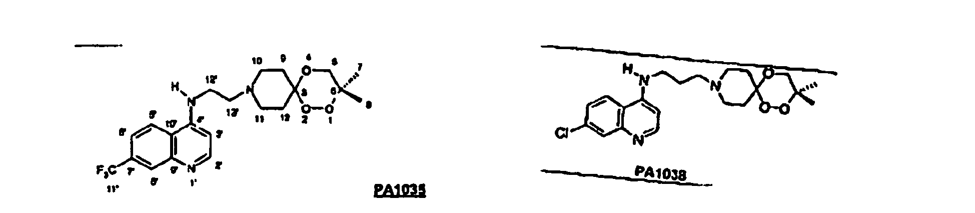

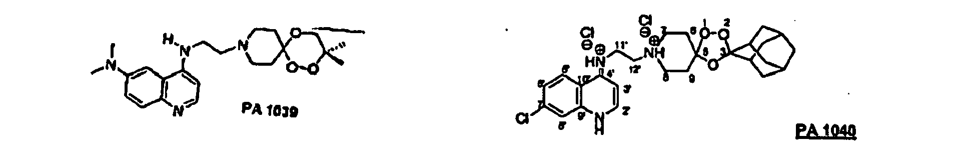

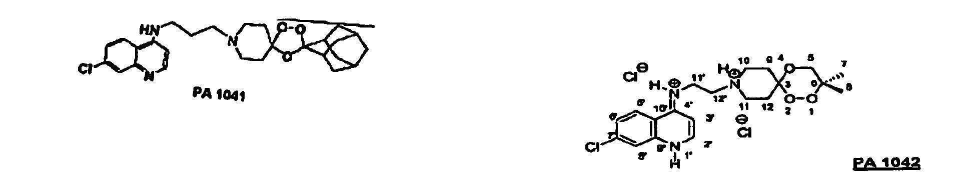

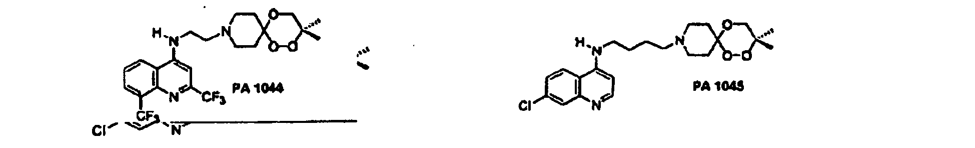

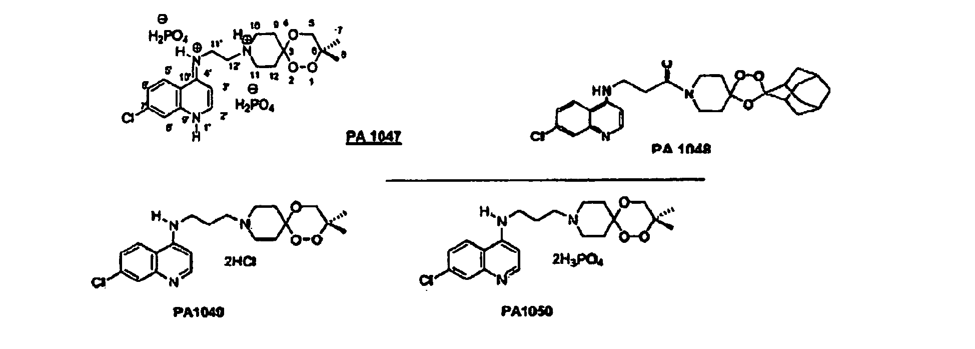

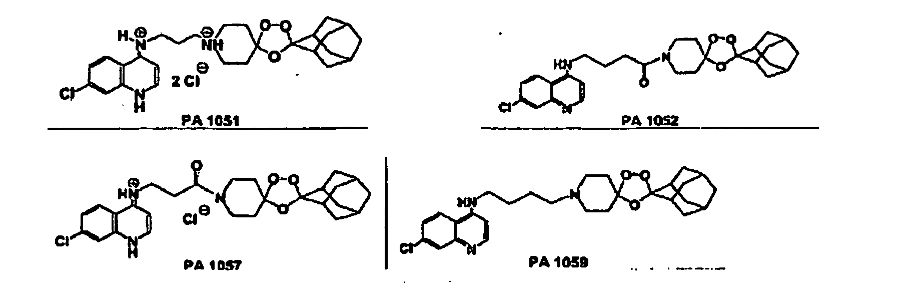

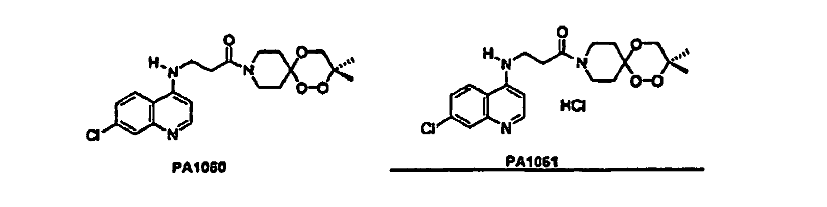

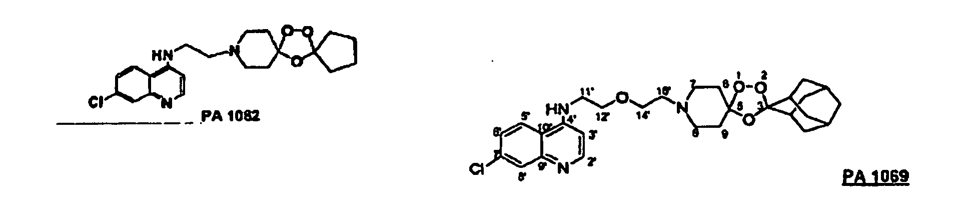

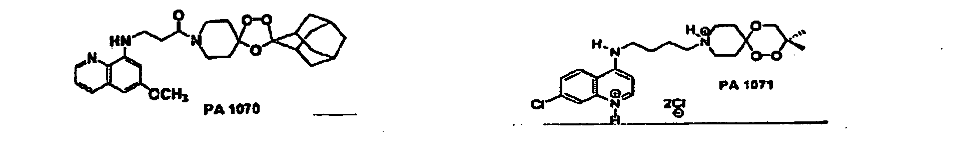

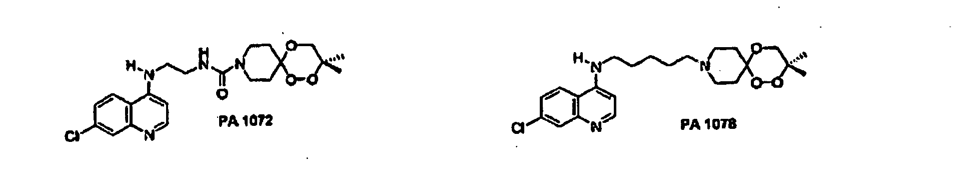

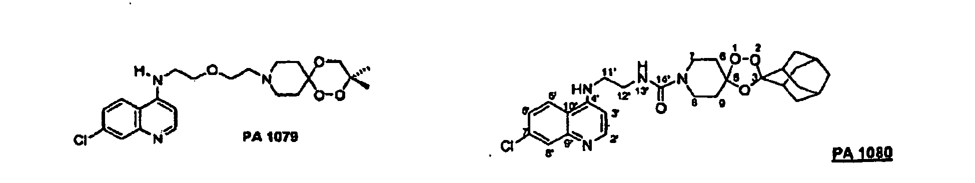

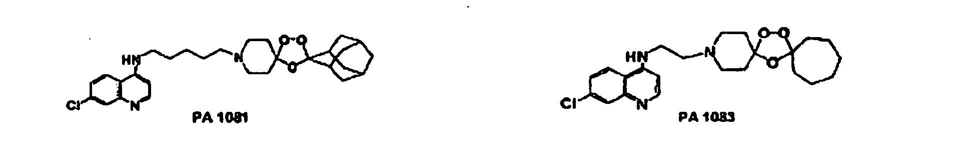

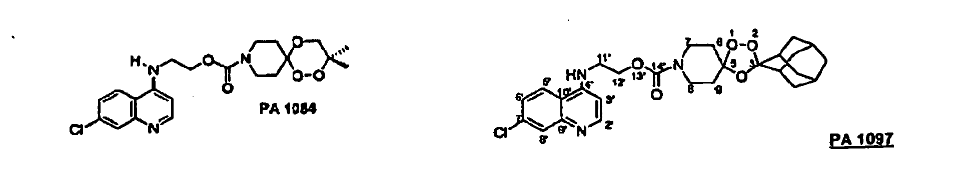

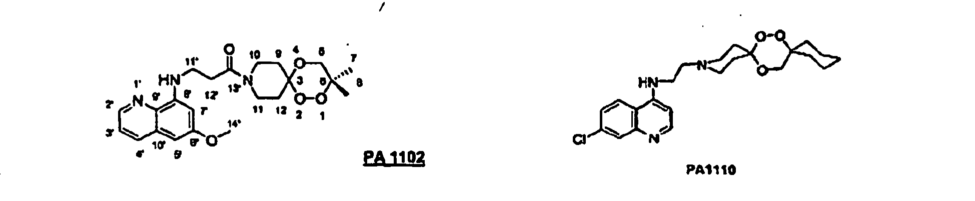

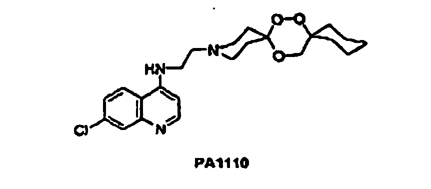

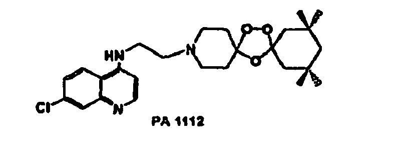

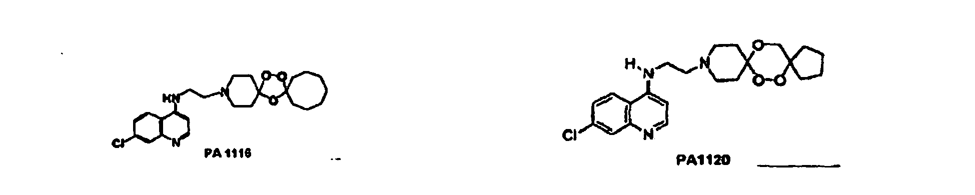

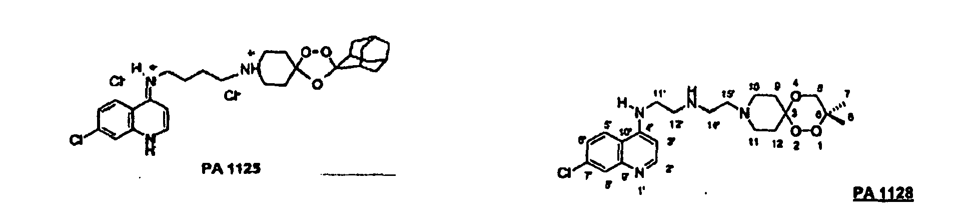

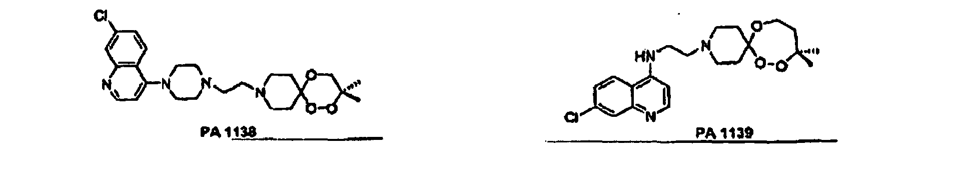

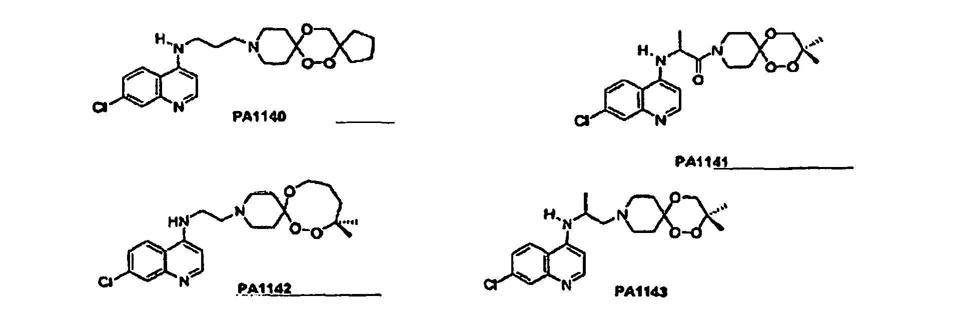

18. Procédé de préparation d'un composé de formule (I) selon l'une quelconque des revendications précédentes, **caractérisé en ce qu'**il comprend la réaction de dérivés réactifs de A qui comportent un groupe fonctionnel tel qu'un groupe hydroxyle ou un groupe partant tel qu'un atome d'halogène et de dérivés peroxydiques comportant les résidus Rₓ et R_{y} de manière à former, entre ces dérivés, un bras de couplage (Y₁)ₚ-(U)_{p'}-(Y₂)ₚ- tel que défini dans la revendication 1.

19. Procédé de préparation selon la revendication 18, **caractérisé en ce que** pour préparer des composés renfermant, comme dérivé A, une aminoquinoléine de formule (IIa) telle que définie dans la revendication 4, on fait réagir un composé de formule (XVII) : 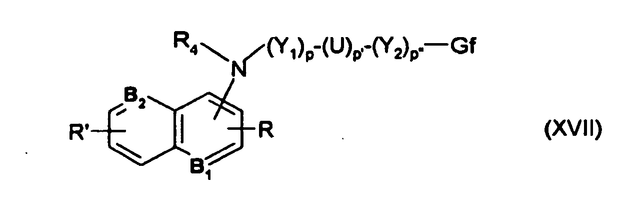 dans laquelle R, R', B₁, B₂, R₄, Y₁, Y₂, U, p, p' et p" sont tels que définis dans les revendications 1 ou 2 et Gf représente un groupe fonctionnel, avec un composé de formule (XVIII), dans laquelle Z₁, Z₂, R₁, R₂, Cj, Rₓ et R_{y} sont tels que définis dans la revendication 1 :

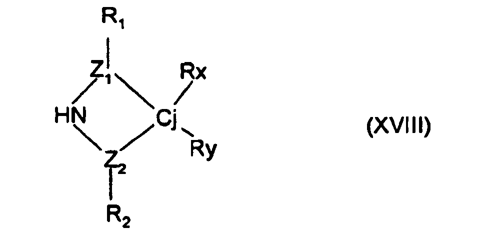

20. Procédé de préparation selon la revendication 18, **caractérisé en ce que** pour préparer des composés dans lesquels p = 1 et renfermant, comme dérivé A, une aminoquinoléine de formule (IIa) telle que définie dans la revendication 2, on fait réagir un composé de formule (XIX) : 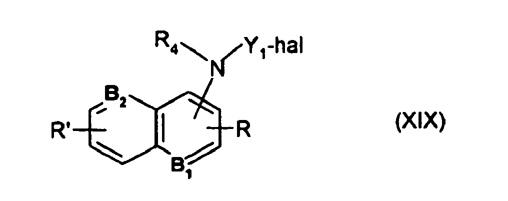 dans laquelle R, R', B₁, B₂, R₄ et Y₁ sont tels que définis dans les revendications 1 et 2, et « hal » représente un atome d'halogène, avec un composé de formule (XX), dans laquelle U, Y₂, p', p", Z₁, Z₂, R₁, R₂, Cj, Rₓ et R_{y} sont tels que définis dans la revendication 1 :

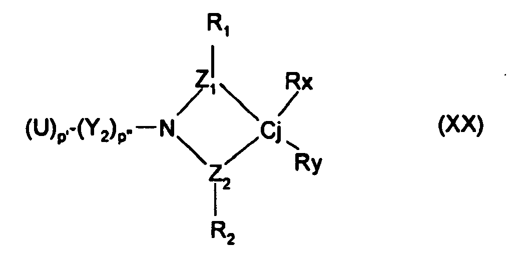

21. Composé de formule (XVIII) ou (XX), dans lesquelles U, Y₂, p', p", Z₁, Z₂, R₁, R₂ et Cj sont tels que définis dans la revendication 1 et Rₓ et R_{y} forment ensemble un peroxyde cyclique comprenant 6,7 ou 8 chaînons et comportant 1 ou 2 atomes d'oxygène supplémentaires dans la structure cyclique, Cj étant des sommets de ce péroxyde cyclique:

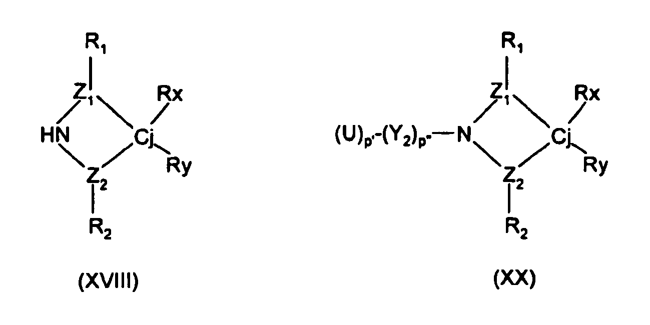

22. Composés de formules (XVIII) au (XX) selon la revendication 21, dans lesquelles Rₓ et R_{y} forment ensemble un trioxane de formule (XI):

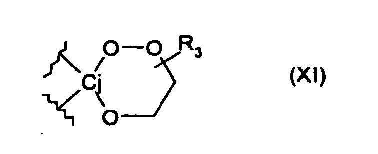

23. Médicament, **caractérisé en ce qu'**il comprend un composé de formule (I) selon l'une quelconque des revendications 1 à 17 ou un sel d'addition de ce composé à un acide pharmaceutiquement acceptable, ou encore un hydrate ou un solvat du composé de formule (I).

24. Composition pharmaceutique, **caractérisée en ce qu'**elle comprend un composé de formule (I) selon l'une quelconque des revendications 1 à 17, ou un sel pharmaceutiquement acceptable, un hydrate ou un solvat de ce composé, ainsi qu'au moins un excipient pharmaceutiquement acceptable.

25. Utilisation d'un composé de formule (I) selon l'une quelconque des revendications 1 à 17 pour la préparation d'un médicament destiné au traitement et à la prévention du paludisme.

## Claims

1. Compound corresponding to the formula (I): 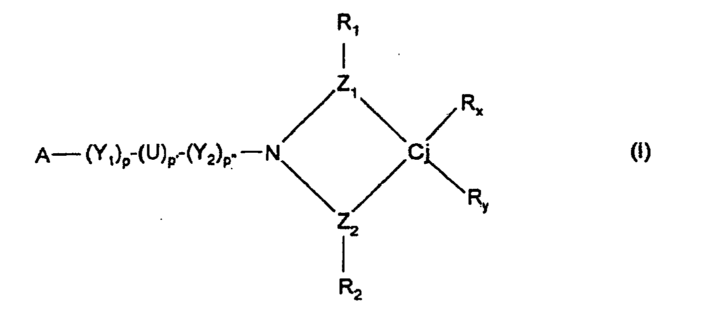 in which:
- A represents a residue of a molecule having an antimalaria activity and/or a residue which facilitates bioavailability, chosen from:
■ a nitrogenous heterocycle of formula (II): 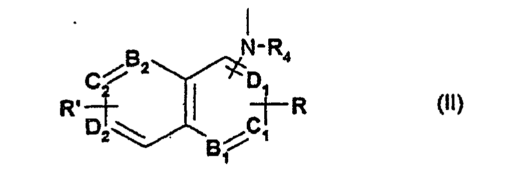
in which:
- R and R', which are identical or different, each represent one or more substituents which occupy distinct positions on the rings to which they are attached and which are chosen from hydrogen or halogen atoms and -OH, -CF₃, aryl, heteroaryl, alkyl, -O-alkyl, -NO₂ or -N(Rₐ,R_{b}) groups, or Rₐ and R_{b}, which are identical or different, represent hydrogen atoms or linear, branched or cyclic alkyl groups comprising from 1 to 5 carbon atoms; one at least of R or R' being other than a hydrogen atom;
- R₄ represents a hydrogen atom or a linear, branched or cyclic C₁ to C₅ alkyl radical, or R₄ forms, with a carbon or nitrogen atom present in the -(Y₁)ₚ-(U)_{p'}-(Y2)_{p"}- group of the formula (I), a 5- to 8-membered ring,
- the B₁, C₁, D₁, B₂, C₂ and D₂ radicals represent nitrogen atoms or -CH= ring members, given that either one of the B₁, C₁ and D₁ radicals represents a nitrogen atom and the other radicals represent -CH= ring members, or one of the B₂, C₂ and D₂ radicals represents a nitrogen atom and the other radicals represent -CH= ring members, or one of the B₁, C₁ and D₁ radicals and one of the B₂, C₂ and D₂ radicals represent nitrogen atoms and the other radicals represent -CH= ring members,
■ a residue of formula (IV), (V), (VI), (VII), (VIII), (IX) or (X), in which R, R', Rₐ and R_{b} are as defined in the claim for the compound of formula (II) and R₅ represents an aryl radical or a nitrogenous heterocyclic residue optionally substituted by one or more R groups:
R₅-CHOH- (IV) 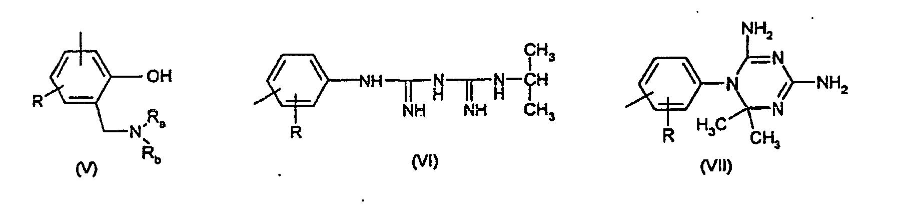 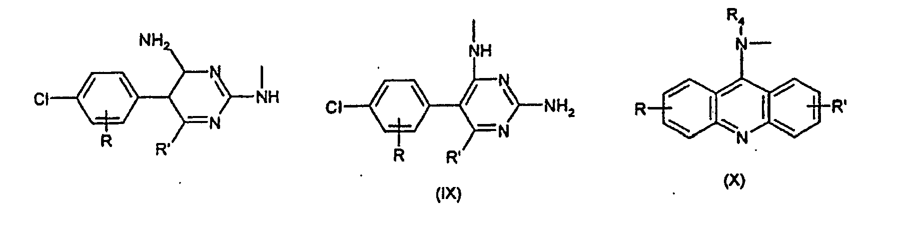
- p, p' and p" represent, independently of one another, 0 or 1, one at least of p or p" being equal to 1,
- Y₁ and Y₂, which are identical or different, represent a linear, branched or cyclic C₁ to C₆ alkylene chain optionally comprising one or more amine, amide, thioamide, sulphonyl, sulphonate, sulphonamide, carbonyl, thiocarbonyl, carboxyl, thiocarboxyl, ether or thioether radicals, this alkylene chain optionally being substituted by one or more groups chosen from halogen atoms, hydroxyl groups, acetal groups and linear, branched or cyclic C₁ to C₅ alkyl radicals,
- U represents an amine, amide, thioamide, sulphonyl, sulphonate, sulphonamide, carbonyl, thiocarbonyl, carboxyl, thiocarboxyl, ether or thioether radical,
- Z₁ and Z₂, which are identical or different, represent a saturated or unsaturated linear, branched or cyclic C₁ to C₄ alkylene radical, it being possible for one of Z₁ or Z₂ to be absent, or the Z₁ + Z₂ combination represents a polycyclic structure including N and the junction carbon Cj,
- R₁ and R₂, which are identical or different, represent a hydrogen atom or a functional group capable of increasing the solubility in water of the dual molecule,
- Rₓ and R_{y} together form a cyclic peroxide comprising from 4 to 8 ring members and comprising 1 or 2 additional oxygen atoms in the cyclic structure, Cj being one of the apexes of this cyclic peroxide,
■ the said cyclic peroxide being substituted by 1 to 8 R₃ groups, identical to or different from one another, occupying any of the positions on the carbon atoms of the peroxide ring and being chosen from the following atoms and groups:
hydrogen, halogen, -OH, -CF₃, -NO₂, aryl, heteroaryl, alkyl or -O-alkyl, the said alkyl groups comprising from 1 to 10 carbon atoms and being linear, branched or cyclic, or
mono-, bi- or tricyclic C₃ to C₁₈ structure which can additionally comprise one or more heteroatoms chosen from oxygen, nitrogen and sulphur and which is optionally substituted by one or more groups chosen from halogen atoms, hydroxyl groups and linear, branched or cyclic C₁ to C₆ alkyl groups,
■ one at least of the R₃ groups being other than a hydrogen atom,
■ it being possible for two R₃ groups carried by adjacent carbon atoms on the peroxide ring to together form a saturated or unsaturated 5- or 6-membered cyclic structure optionally substituted in any of the positions by one or more R₃ substituents as defined above,
■ it being possible for two R₃ groups carried by the same carbon atom of the peroxide ring to together form a mono-, bi- or tricyclic structure as defined above,
in the form of the base or of an addition salt with an acid, and also in the hydrate or solvate form.

2. Compound of formula (I) according to Claim 1, **characterized in that** A represents an aminoquinoline of formula (IIa) or a 1,5-naphthyridine of formula (III): 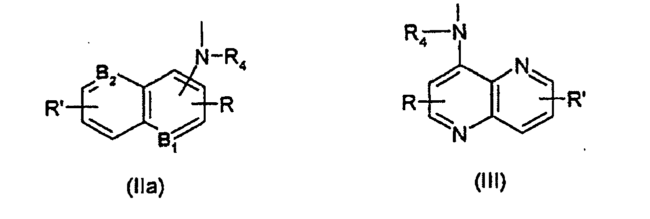 in which R, R' and R₄ are as defined in Claim 3 and either B₁ represents a nitrogen atom and B₂ represents a -CH= ring member or B₁ represents a -CH= ring member and B₂ represents a nitrogen atom,
in the form of the base or of an addition salt with an acid, and also in the hydrate or solvate form.

3. Compound of formula (I) according to either one of the preceding claims, **characterized in that** A represents an aminoquinoline of formula (IIb) or (IIc): 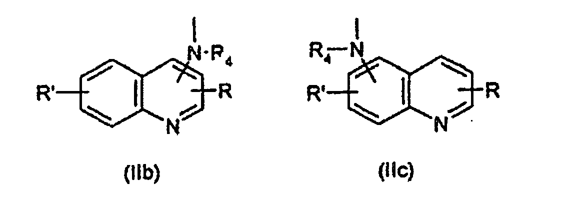 in which R, R' and R₄ are as defined in Claim 1,
in the form of the base or of an addition salt with an acid, and also in the hydrate or solvate form.

4. Compound of formula (I) according to any one of the preceding claims, **characterized in that** Rₓ and R_{y} together form a cyclic peroxide comprising 5 or 6 ring members and comprising one additional oxygen atom in the cyclic structure, Cj being one of the apexes of this cyclic peroxide, the said cyclic peroxide being substituted by 1 to 4 identical or different R₃ groups as defined in Claim 1,
in the form of the base or of an addition salt with an acid, and also in the hydrate or solvate form.

5. Compound of formula (I) according to Claim 4, **characterized in that** Rₓ and R_{y} together form a cyclic peroxide corresponding to the formula (XI) in which R₃ represents 1 to 4 identical or different groups as defined in Claim 1: 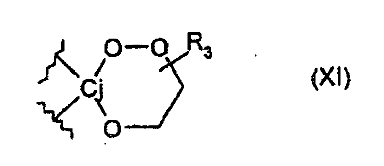 in the form of the base or of an addition salt with an acid, and also in the hydrate or solvate form.

6. Compound of formula (I) according to Claim 5, **characterized in that** Rₓ and R_{y} together form a cyclic peroxide corresponding to the formula (XIa): 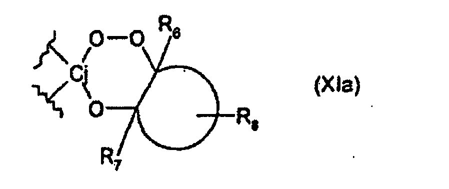 in which the arc of a circle represents a saturated or unsaturated 5- or 6-membered cyclic structure and R₆, R₇ and R₈, which are identical to or different from one another, are chosen from the following atoms and groups: hydrogen, halogen, -OH, -CF₃, -NO₂, aryl, heteroaryl, alkyl or -O-alkyl, the said alkyl groups comprising from 1 to 10 carbon atoms and being linear, branched or cyclic,
in the form of the base or of an addition salt with an acid, and also in the hydrate or solvate form.

7. Compound of formula (I) according to Claim 5 or Claim 6, **characterized in that** Rₓ and R_{y} together form a cyclic peroxide corresponding to the formula (XIb): 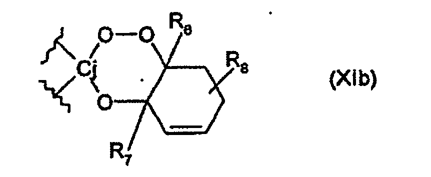 in which R₆, R₇ and R₈ are as defined in Claim 6, in the form of the base or of an addition salt with an acid, and also in the hydrate or solvate form.

8. Compound of formula (I) according to Claim 5, **characterized in that** Rₓ and R_{y} together form a cyclic peroxide corresponding to the formula (XIc): 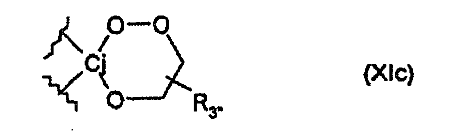 in which R_{3"} represents from 1 to 4 groups, identical to or different from one another, which occupy any of the positions on the carbon atoms of the peroxide ring and which are chosen from the following atoms and groups:
hydrogen, halogen, -OH, -CF₃, -NO₂, aryl, heteroaryl, alkyl or -O-alkyl, the said alkyl groups comprising from 1 to 10 carbon atoms and being linear, branched or cyclic, or
mono-, bi- or tricyclic C₃ to C₁₈ structure which can additionally comprise one or more heteroatoms chosen from oxygen, nitrogen and sulphur and which is optionally substituted by one or more groups chosen from halogen atoms, hydroxyl groups and linear, branched or cyclic C₁ to C₆ alkyl groups,
■ one at least of the R_{3"} groups being other than a hydrogen atom,
■ it being possible for two R_{3"} groups carried by the same carbon atom of the peroxide ring to together form a mono-, bi- or tricyclic structure as defined above,
in the form of the base or of an addition salt with an acid, and also in the hydrate or solvate form.

9. Compound of formula (I) according to Claim 4, **characterized in that** Rₓ and R_{y} together form a cyclic peroxide corresponding to the formula (XII): 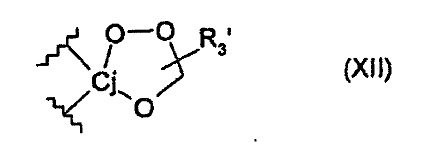 in which R_{3'} represents 1 or 2 identical or different groups chosen from the following atoms and groups:
hydrogen, halogen, -OH, -CF₃, -NO₂, aryl, heteroaryl, alkyl or -O-alkyl, the said alkyl groups comprising from 1 to 10 carbon atoms and being linear, branched or cyclic, or
mono-, bi- or tricyclic C₃ to C₁₈ structure which can additionally comprise one or more heteroatoms chosen from oxygen, nitrogen and sulphur and which is optionally substituted by one or more groups chosen from halogen atoms, hydroxyl groups and linear, branched or cyclic C₁ to C₆ alkyl groups,
■ one at least of the R_{3'} groups being other than a hydrogen atom,
■ it being possible for two R_{3'} groups carried by the same carbon atom of the peroxide ring to together form a mono-, bi- or tricyclic structure as defined above,
in the form of the base or of an addition salt with an acid, and also in the hydrate or solvate form.

10. Compound of formula (I) according to any one of Claims 1, 2, 4 and 5, **characterized in that** it corresponds to the formula (XIII): 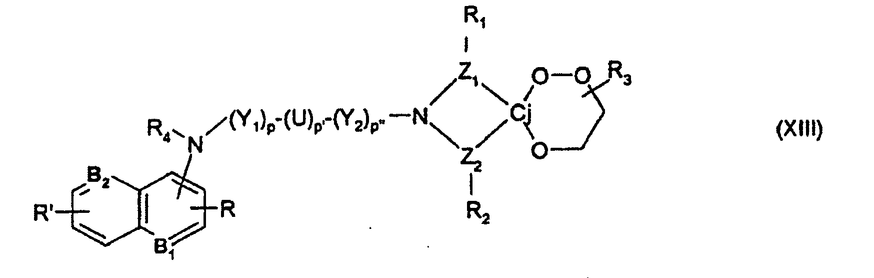 in which R, R', B₁, B₂, Y₁, U, Y₂, p_{'} p_{'}, p". Z₁, Z₂, Cⱼ, R₁, R₂, R₃ and R₄ are as defined in any one of Claims 1, 2, 4 and 5,
in the form of the base or of an addition salt with an acid, and also in the hydrate or solvate form.

11. Compound of formula (I) according to any one of Claims 1, 2, 4 and 9, **characterized in that** it corresponds to the formula (XIV): 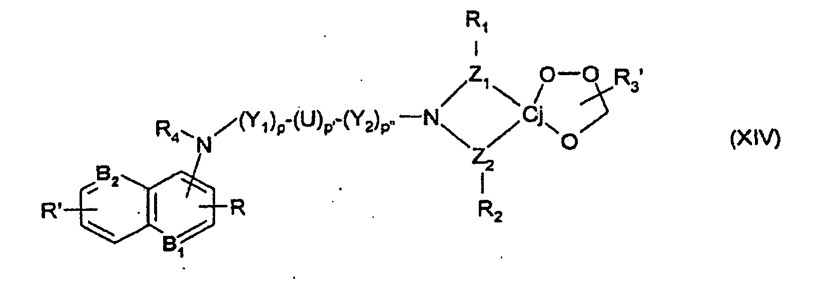 in which R, R', B₁, B₂, Y₁, U, Y₂, p, p' , p'' . Z₁.
Z₂, Cⱼ, R₁, R₂, R₃, and R₄ are as defined in any one of Claims 1, 2, 4 and 9,
in the form of the base or of an addition salt with an acid, and also in the hydrate or solvate form.

12. Compound of formula (I) according to any one of the preceding claims, **characterized in that**:
- Y₁ and Y₂, which are identical or different, represent a linear, branched or cyclic C₁ to C₆ alkylene chain optionally comprising one or more amine, amide, carbonyl or ether radicals, this C₁ to C₆ alkylene chain optionally being substituted by one or more groups chosen from halogen atoms, hydroxyl groups, acetal groups and linear, branched or cyclic C₁ to C₅ alkyl radicals,
- U represents an amine, amide, carbonyl, carboxyl or ether radical,
in the form of the base or of an addition salt with an acid, and also in the hydrate or solvate form.

13. Compound of formula (I) according to Claim 12, **characterized in that**:
- either p' = p" = 0, p = 1 and Y₁ represents a linear or branched C₁ to C₆ alkylene chain optionally substituted by one or more groups chosen from halogen atoms, hydroxyl groups, acetal groups and linear, branched or cyclic C₁ to C₅ alkyl radicals,
- or p = p' = 1, p" = 0, Y₁ represents a linear or branched C₁ to C₆ alkylene chain optionally substituted by one or more groups chosen from halogen atoms, hydroxyl groups, acetal groups and linear, branched or cyclic C₁ to C₅ alkyl radicals, and U represents an amine, amide, carbonyl, carboxyl or ether radical,
- or p = p' = p" = 1, Y₁ and Y₂ represent linear or branched C₁ to C₆ alkylene chains which are optionally substituted by one or more groups chosen from halogen atoms, hydroxyl groups, acetal groups and linear, branched or cyclic C₁ to C₅ alkyl radicals, and U represents an amine, amide, carbonyl, carboxyl or ether radical,
in the form of the base or of an addition salt with an acid, and also in the hydrate or solvate form.

14. Compound of formula (I) according to any one of the preceding claims, **characterized in that** Z₁ and Z₂, which are identical or different, each represent a linear or branched C₁ to C₄ alkylene radical,
in the form of the base or of an addition salt with an acid, and also in the hydrate or solvate form.

15. Compound of formula (I) according to any one of Claims 1, 2, 4, 5 and 10, **characterized in that** it corresponds to the formula (XV): 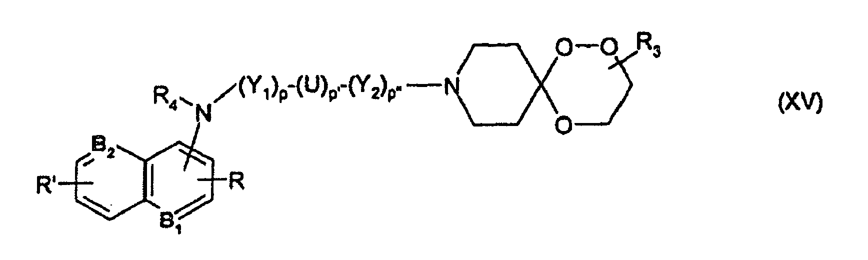 in which R, R', B₁, B₂, Y₁, U, Y₂, p, p', p", R₁,
R₂, R₃ and R₄ are as defined in any one of Claims 1, 2, 4, 5 and 10,
in the form of the base or of an addition salt with an acid, and also in the hydrate or solvate form.

16. Compound of formula (I) according to any one of Claims 1, 2, 4, 9 and 11, **characterized in that** it corresponds to the formula (XVI): 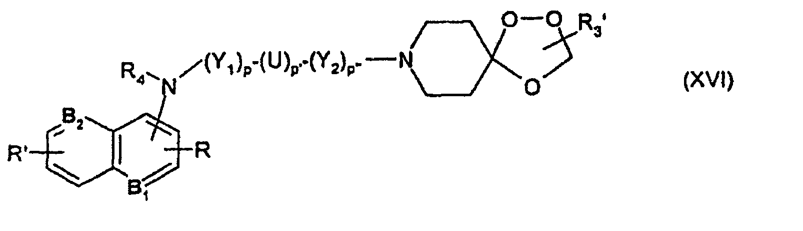 in which R, R', B₁, B₂, Y₁, U, Y₂, p, p', p", R₁,
R₂, R₃, and R₄ are as defined in any one of Claims 1, 2, 4, 9 and 11,
in the form of the base or of an addition salt with an acid, and also in the hydrate or solvate form.

17. Compound of formula (I) according to any one of the preceding claims, **characterized in that** it is chosen from the following compounds:

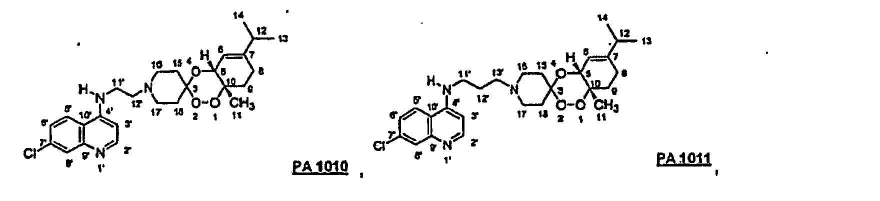

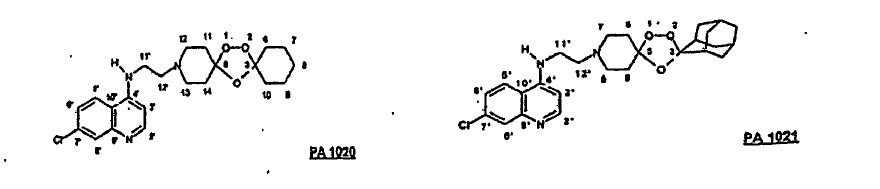

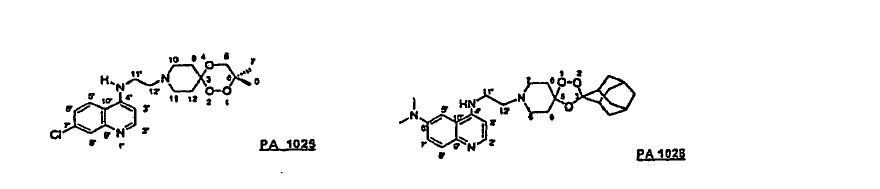

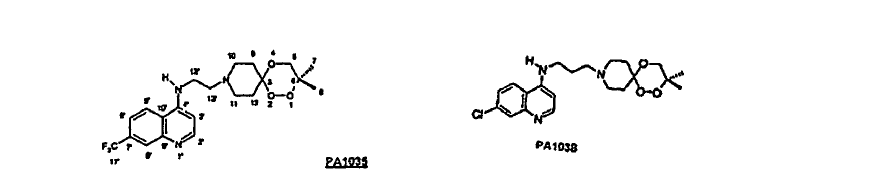

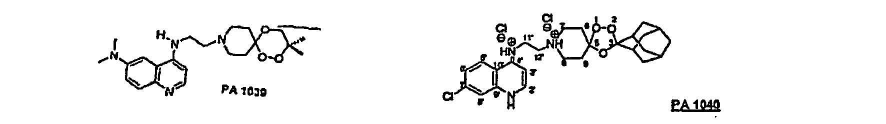

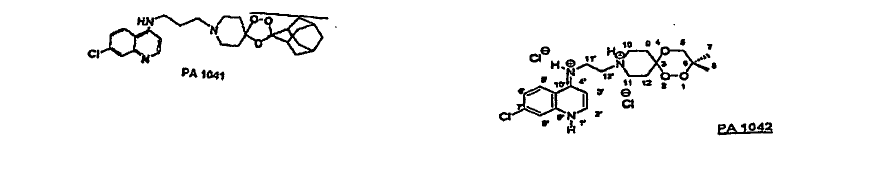

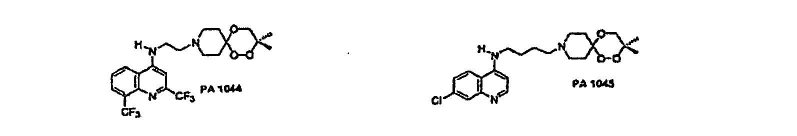

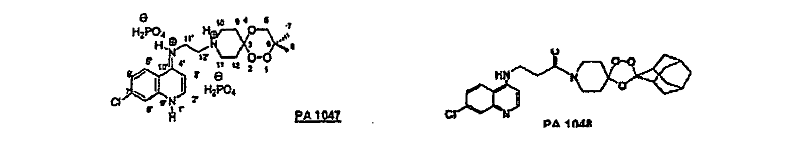

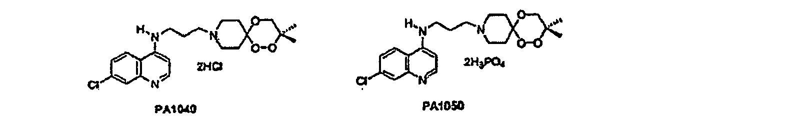

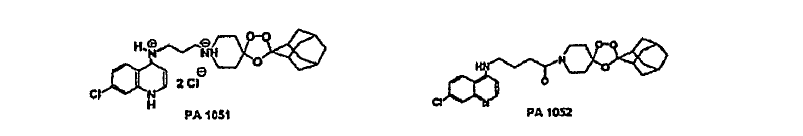

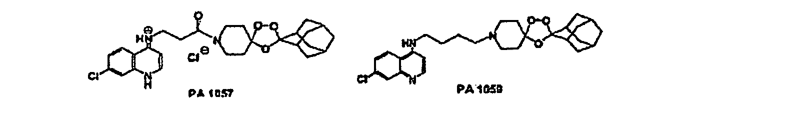

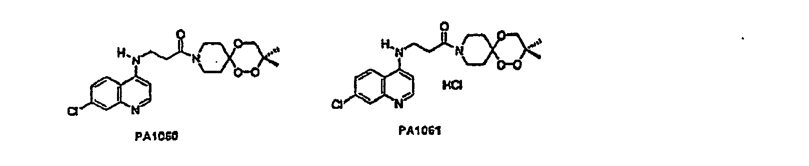

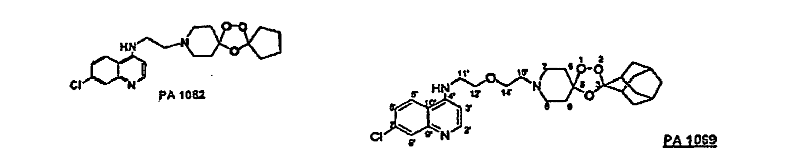

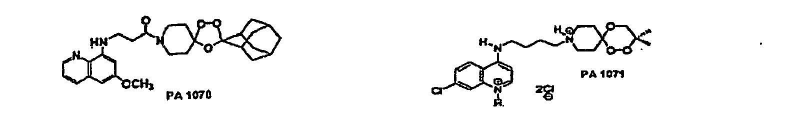

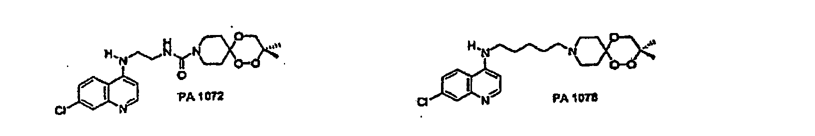

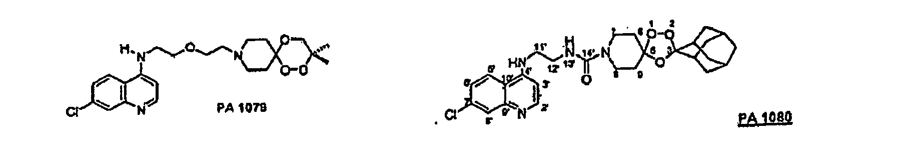

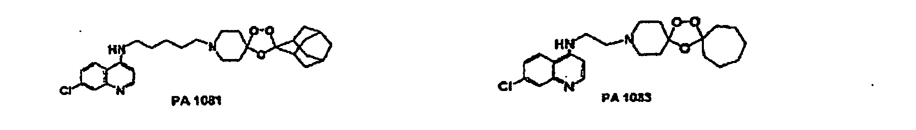

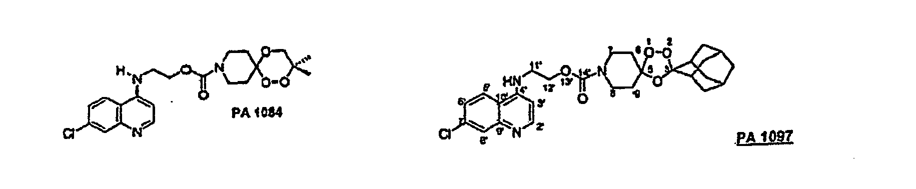

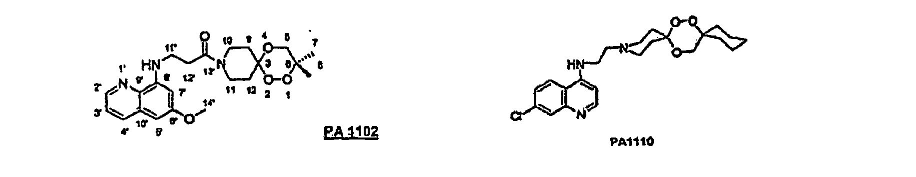

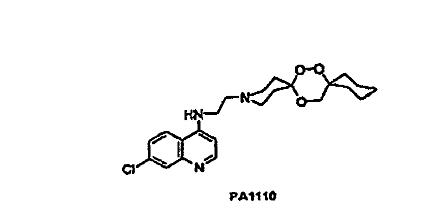

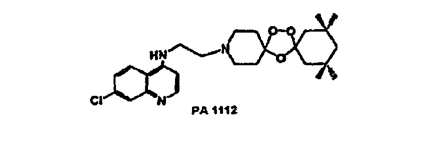

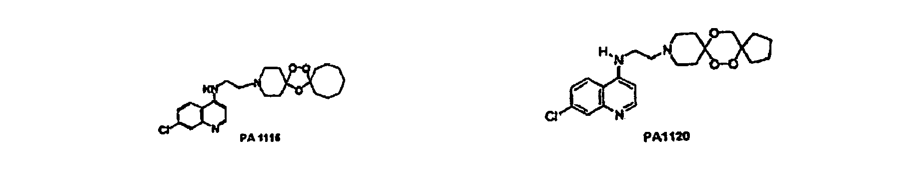

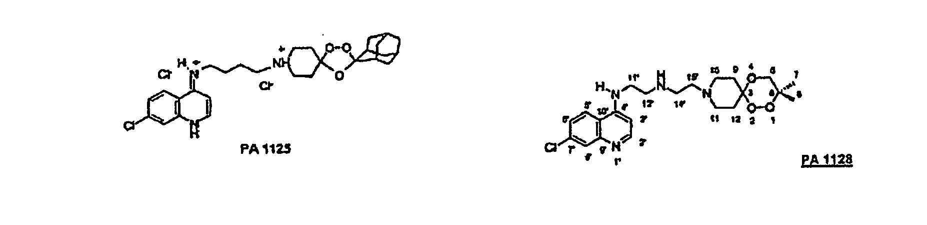

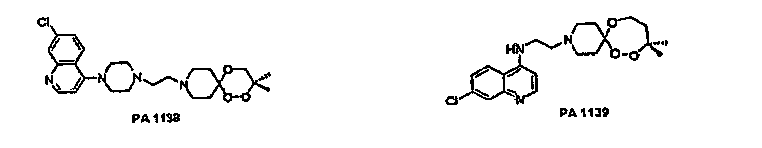

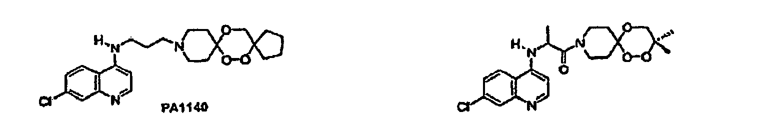

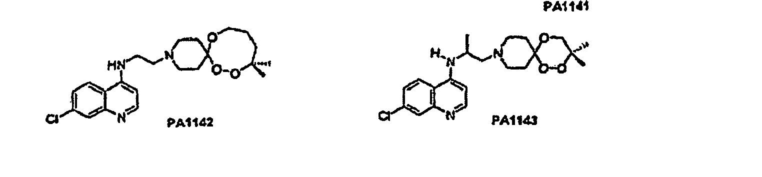

18. Process for the preparation of a compound of formula (I) according to any one of the preceding claims, **characterized in that** it comprises the reaction of reactive derivatives of A which comprise a functional group, such as a hydroxyl group, or a leaving group, such as a halogen atom, and of peroxide derivatives comprising the Rₓ and R_{y} residues, so as to form, within these derivatives, a coupling arm (Y₁)ₚ-(U)ₚ-(Y₂)_{p"} as defined in Claim 1.

19. Preparation process according to Claim 18, **characterized in that**, in order to prepare compounds including, as derivative A, an aminoquinoline of formula (IIa) as defined in Claim 4, a compound of formula (XVII) : 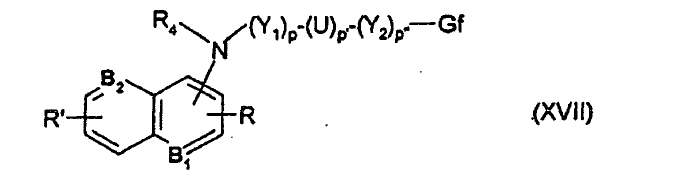 in which R, R', B₁, B₂, R₄, Y₁, Y₂, U, p, p' and p" are as defined in Claim 1 or Claim 2 and Gf represents a functional group, is reacted with a compound of formula (XVIII), in which Z₁, Z₂, R₁, R₂, Cj, Rₓ and R_{y} are as defined in Claim 1:

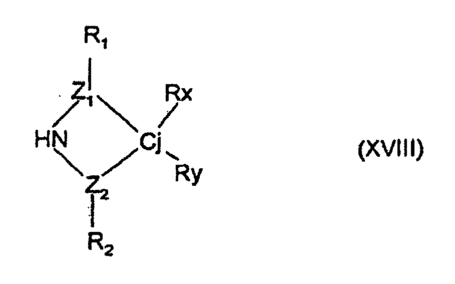

20. Preparation process according to Claim 18, **characterized in that**, in order to prepare compounds in which p = 1 and which include, as derivative A, an aminoquinoline of formula (IIa) as defined in Claim 2, a compound of formula (XIX): 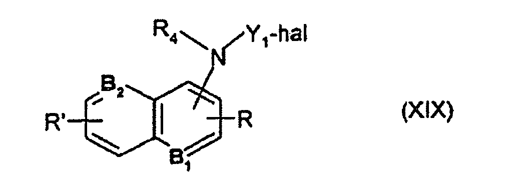 in which R, R', B₁, B₂, R₄ and Y₁ are as defined in Claims 1 and 2 and "hal" represents a halogen atom, is reacted with a compound of formula (XX), in which U, Y₂, p' , p'' , Z₁, Z₂, R₁, R₂, Cj, Rₓ and R_{y} are as defined in Claim 1:

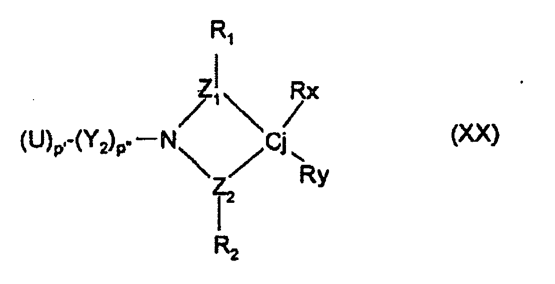

21. Compound of formula (XVIII) or (XX), in which U, Y₂, p', P'', Z₁, Z₂, R₁, R₂ and Cⱼ are as defined in Claim 1 and Rₓ and R_{y} together form a cyclic peroxide comprising 6, 7 or 8 ring members and comprising 1 or 2 additional oxygen atoms in the cyclic structure, Cj being apexes of this cyclic peroxide:

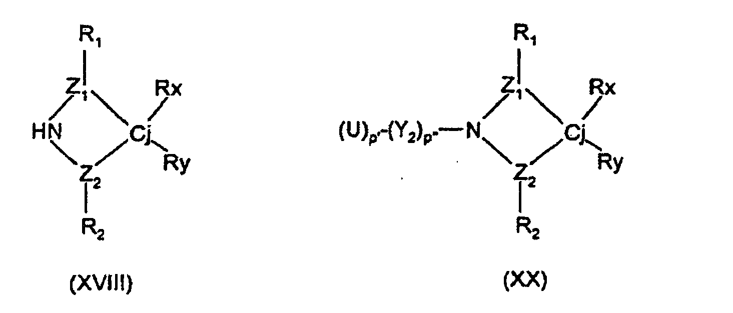

22. Compound of formula (XVIII) or (XX) according to Claim 21, in which Rₓ and R_{y} together form a trioxane of formula (XI):

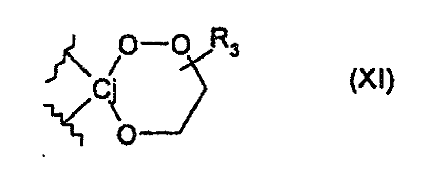

23. Medicament, **characterized in that** it comprises a compound of formula (I) according to any one of Claims 1 to 17 or an addition salt of this compound with a pharmaceutically acceptable acid or also a hydrate or a solvate of the compound of formula (I).

24. Pharmaceutical composition, **characterized in that** it comprises a compound of formula (I) according to any one of Claims 1 to 17 or a pharmaceutically acceptable salt, a hydrate or a solvate of this compound, and also at least one pharmaceutically acceptable excipient.

25. Use of a compound of formula (I) according to any one of Claims 1 to 17 in the preparation of a medicament intended for the treatment and prevention of malaria.

## Patentansprüche

1. Verbindung der Formel (I): 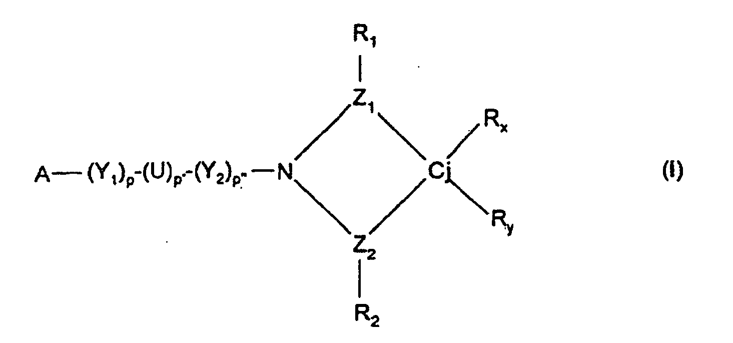 worin:
- A für einen Molekülrest mit Antimalariawirkung und/oder einen die Bioverfügbarkeit fördenden Molekülrest steht, der ausgewählt ist unter:
■ einem Stickstoffheterocyclus der Formel (II): 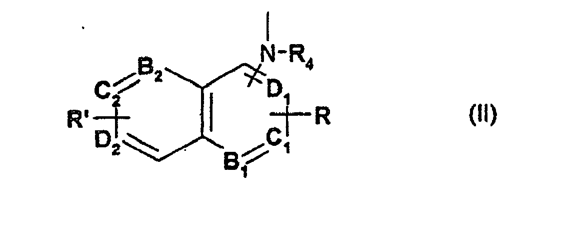
worin:
- R und R' gleich oder verschieden sind und jeweils für einen oder mehrere Substituenten, die an den Ringen, an die sie gebunden sind, verschiedene Positionen einnehmen, und unter Wasserstoff- oder Halogenatomen und -OH-, -CF₃-, Aryl-, Heteroaryl-, Alkyl- oder -O-Alkyl, -NO₂- oder -N(RaRb) - Gruppen ausgewählt sind, wobei Rₐund R_{b} gleich oder
verschieden sind und für Wasserstoffatome oder lineare, verzweigte oder cyclische Alkylgruppen mit 1 bis 4 Kohlenstoffatomen stehen; wobei mindestens eine der Gruppen R und R' von einem Wasserstoffatom verschieden ist;
- R₄ für ein Wasserstoffatom oder einen linearen, verzweigten oder cyclischen C1- bis C5-Alkylrest steht oder R₄ mit einem in der Gruppierung -(Y₁)p-(U)_{p'}-(Y₂)_{p"}- der Formel (I) vorhandenen Kohlenstoff- oder Stickstoffatom einen 5- bis 8-gliedrigen Ring bildet, .
- die Reste B₁, C₁, D₁, B₂, C₂ und D₂ für Stickstoffatome oder -CH=-Glieder stehen, mit der Maßgabe, daß entweder einer der Reste B₁, C₁ und D₁ für ein Stickstoffatom steht und die anderen Reste für -CH=-Glieder stehen oder einer der Reste B₂, C₂ und D₂ für ein Stickstoffatom steht und die anderen Reste für -CH=-Glieder stehen oder einer der Reste B₁, C₁ und D₁ und einer der Reste B₂, C₂ und D₂ für Stickstoffatome stehen und die anderen Reste für
- CH=-Glieder stehen;
■ einem Rest der Formel (IV), (V), (VI), (VII), (VIII), (IX) oder (X), worin R, R', Rₐ und R_{b} die im Anspruch für die Verbindung der Formel (II) angegebene Bedeutung besitzen und R₅ für einen Arylrest oder einen stickstoffhaltigen heterocyclischen Rest steht, der gegebenenfalls durch eine oder mehrere Gruppen R substituiert ist:
R₅-CHOH- (IV) 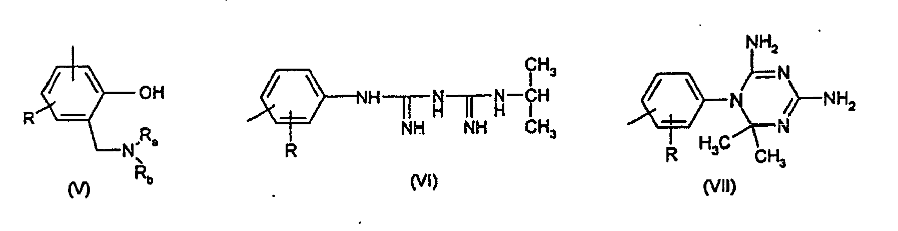 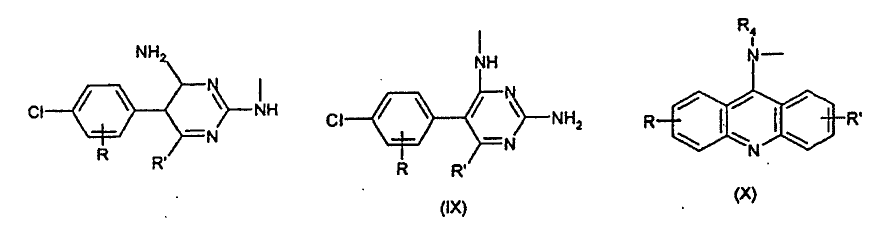
- p, p' und p" unabhängig voneinander für 0 oder 1 stehen, wobei mindestens eine der Variablen p und p" gleich 1 ist,
- Y₁ und Y₂ gleich oder verschieden sind und für eine lineare, verzweigte oder cyclische C1- bis C6-Alkylenkette, die gegebenenfalls einen oder mehrere Amin-, Amid-, Thioamid-, Sulfonyl-,
Sulfonat-, Sulfonamid-, Carbonyl-, Thiocarbonyl-, Carboxyl-, Thiocarboxyl-, Ether- oder Thioetherreste enthält, steht, wobei diese Alkylenkette gegebenenfalls durch eine oder mehrere unter Halogenatomen, Hydroxylgruppen, Acetalgruppen und linearen, verzweigten oder cyclischen C1- bis C5-Alkylresten ausgewählte Gruppen substituiert ist,
- U für einen Amin-, Amid-, Thioamid-, Sulfonyl-, Sulfonat-, Sulfonamid-, Carbonyl-, Thiocarbonyl-, Carboxyl-, Thiocarboxyl-, Ether- oder Thioetherrest steht,
- Z₁ und Z₂ gleich oder verschieden sind und für einen gesättigten oder ungesättigten, linearen, verzweigten oder cyclischen C1- bis C4-Alkylrest stehen, wobei eine der Gruppen Z₁ und Z₂ fehlen kann oder Z₁ + Z₂ gemeinsam eine polycyclische Struktur einschließlich N und des Verküpfungskohlenstoffs Cj bilden,
- R₁ und R₂ gleich oder verschieden sind und für ein Wasserstoffatom oder eine funktionelle Gruppe, die zur Erhöhung der Wasserlöslichkeit des dualen Moleküls befähigt ist, stehen,
- Rₓ und R_{y} gemeinsam ein 4- bis 8-gliedriges cylisches Peroxid mit 1 oder 2 zusätzlichen Sauerstoffatomen in der cyclischen Struktur bilden, wobei Cj eine der Spitzen dieses cyclischen Peroxids ist,
. wobei das cyclische Peroxid durch 1 bis 8 Gruppen R₃ substituiert ist, die gleich oder voneinander verschieden sind, beliebige Positionen an den Kohlenstoffatomen des Peroxidrings einnehmen und unter den folgenden Atomen und Gruppen ausgewählt sind:
Wasserstoff, Halogen, -OH, -CF₃, -NO₂, Aryl oder Heteroaryl, Alkyl oder -O-Alkyl, wobei die Alkylgruppen 1 bis 10 Kohlenstoffatome enthalten und linear, verzweigt oder cyclisch sind, oder einer mono-, bi- oder tricyclischen C3- bis C18-Struktur, die außerdem 1 oder mehrere unter Sauerstoff, Stickstoff und Schwefel ausgewählte Heteroarome enthalten kann und gegebenenfalls durch eine oder mehrere unter Halogenatomen, Hydroxylgruppen und linearen, verzweigten oder cyclischen C1- bis C6-Alkylgruppen ausgewählte Gruppen substituiert ist,
. wobei mindestens eine der Gruppen R₃ von einem Wasserstoffatom verschieden ist,
. wobei zwei Gruppen R₃, die an benachbarte Kohlenstoffatome des Peroxidrings gebunden sind, gemeinsam eine gesättigte oder ungesättigte 5-oder 6-gliedrige cyclische Struktur bilden können, die gegebenenfalls in beliebigen Positionen durch einen oder mehrere Substituenten R₃ gemäß obiger Definition substituiert ist,
. wobei zwei Gruppen R₃, die an dasselbe Kohlenstoffatom des Peroxidrings gebunden sind, gemeinsam eine mono-, bi- oder tricyclische Struktur gemäß obiger Definition bilden können, in Basen- oder Säureadditionssalzform sowie in Hydrat- oder Solvatform.

2. Verbindung der Formel (I) nach Anspruch 1, **dadurch gekennzeichnet, daß** A für ein Aminochinolin der Formel (IIa) oder ein 1,5-Naphtyridin der Formel (III) : 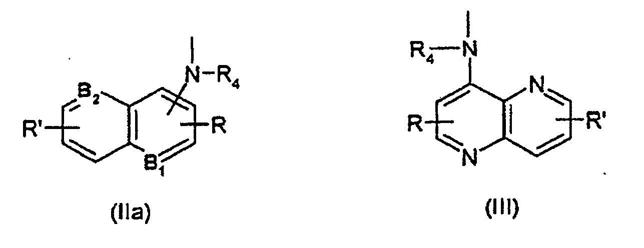 steht, worin R, R' und R₄ die in Anspruch 1 angegebene Bedeutung besitzen und entweder B₁ für ein Stickstoffatom steht und B₂ für ein -CH=-Glied steht oder B₁ für ein -CH=-Glied steht und B₂ für ein Stickstoffatom steht,
in Basen- oder Säureadditionssalzform sowie in Hydrat- oder Solvatform.

3. Verbindung der Formel (I) nach einem der vorhergehenden Ansprüche, **dadurch gekennzeichnet, daß** A für ein Aminochinolin der Formel (IIb) oder (IIc): 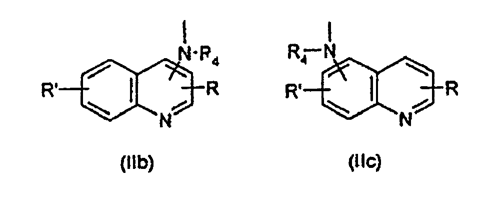 steht, worin R, R' und R₄ die in Anspruch 1 angegebene Bedeutung besitzen,
in Basen- oder Säureadditionssalzform sowie in Hydrat- oder Solvatform.

4. Verbindung der Formel (I) nach einem der vorhergehenden Ansprüche, Rₓ und R_{y} gemeinsam ein 5- oder 6-gliedriges cylisches Peroxid mit 1 zusätzlichem Sauerstoffatom in der cyclischen Struktur bilden, wobei Cj eine der Spitzen dieses cyclischen Peroxids ist, wobei das cyclische Peroxid durch 1 bis 4 gleiche oder verschiedene Gruppen R₃, die die in Anspruch 1 angegebene Bedeutung besitzen, substituiert ist,
in Basen- oder Säureadditionssalzform sowie in Hydrat- oder Solvatform.

5. Verbindung der Formel (I) nach Anspruch 4, **dadurch gekennzeichnet, daß** Rₓ und R_{y} gemeinsam ein cylisches Peroxid der Formel (XI) bilden, worin R₃ für 1 bis 4 gleiche oder verschiedene Gruppen, die die in Anspruch 1 angegebene Bedeutung besitzen, steht: 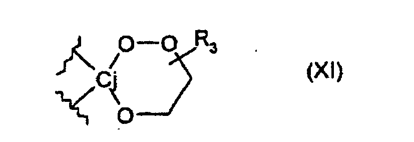 in Basen- oder Säureadditionssalzform sowie in Hydrat- oder Solvatform.

6. Verbindung der Formel (I) nach Anspruch 5, **dadurch gekennzeichnet, daß** Rₓ und R_{y} gemeinsam ein cylisches Peroxid der Formel (XIa) bilden: 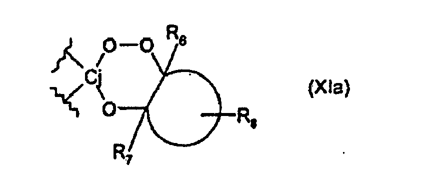 worin der Kreisbogen für eine gesättigte oder ungesättigte 5- oder 6-gliedrige cyclische Struktur steht und R₆, R₇ und R₈ gleich oder voneinander verschieden sind und unter den folgenden Atomen und Gruppen ausgewählt sind: Wasserstoff, Halogen, -OH, -CF₃, -NO₂, Aryl oder Heteroaryl, Alkyl oder -O-Alkyl, wobei die Alkylgruppen 1 bis 10 Kohlenstoffatome enthalten und linear, verzweigt oder cyclisch sind,
in Basen- oder Säureadditionssalzform sowie in Hydrat- oder Solvatform.

7. Verbindung der Formel (I) nach Anspruch 5 oder Anspruch 6, **dadurch gekennzeichnet, daß** Rₓ und R_{y} gemeinsam ein cylisches Peroxid der Formel (XIb) bilden: 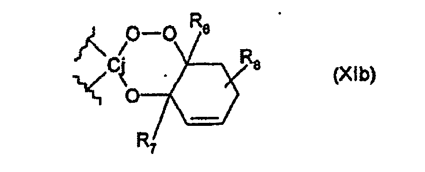 worin R₆, R₇ und R₈ die in Anspruch 6 angegebene Bedeutung besitzen,
in Basen- oder Säureadditionssalzform sowie in Hydrat- oder Solvatform.

8. Verbindung der Formel (I) nach Anspruch 5, **dadurch gekennzeichnet, daß** Rₓ und R_{y} gemeinsam ein cylisches Peroxid der Formel (XIc) bilden: 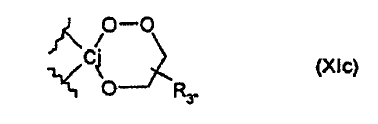 worin R_{3"} für 1 bis 4 gleiche oder voneinander verschiedene Gruppen steht, die beliebige Positionen an den Kohlenstoffatomen des Peroxidrings einnehmen und unter den folgenden Atomen und Gruppen ausgewählt sind:
Wasserstoff, Halogen, -OH, -CF₃, -NO₂, Aryl oder Heteroaryl, Alkyl oder -O-Alkyl, wobei die Alkylgruppen 1 bis 10 Kohlenstoffatome enthalten und linear, verzweigt oder cyclisch sind, oder einer mono-, bi- oder tricyclischen C3- bis C18-Struktur, die außerdem 1 oder mehrere unter Sauerstoff, Stickstoff und Schwefel ausgewählte Heteroarome enthalten kann und gegebenenfalls durch eine oder mehrere unter Halogenatomen, Hydroxylgruppen und linearen, verzweigten oder cyclischen C1- bis C6-Alkylgruppen ausgewählte Gruppen substituiert ist,
. wobei mindestens eine der Gruppen R_{3"} von einem Wasserstoffatom verschieden ist,
. wobei zwei Gruppen R_{3"}, die an dasselbe Kohlenstoffatom des Peroxidrings gebunden sind, gemeinsam eine mono-, bi- oder tricyclische Struktur gemäß obiger Definition bilden können,
in Basen- oder Säureadditionssalzform sowie in Hydrat- oder Solvatform.

9. Verbindung der Formel (I) nach Anspruch 4, **dadurch gekennzeichnet, daß** Rₓ und R_{y} gemeinsam ein cyclisches Peroxid der Formel (XII) bilden: 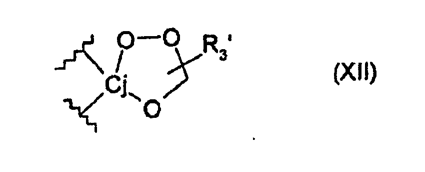 worin R₃. für 1 oder 2 gleiche oder verschiedene Gruppen steht, die unter den folgenden Atomen und Gruppen ausgewählt sind:
Wasserstoff, Halogen, -OH, -CF₃, -NO₂ Aryl oder Heteroaryl, Alkyl oder -O-Alkyl, wobei die Alkylgruppen 1 bis 10 Kohlenstoffatome enthalten und linear, verzweigt oder cyclisch sind, oder einer mono-, bi- oder tricyclischen C3- bis C18-Struktur, die außerdem 1 oder mehrere unter Sauerstoff, Stickstoff und Schwefel ausgewählte Heteroarome enthalten kann und gegebenenfalls durch eine oder mehrere unter Halogenatomen, Hydroxylgruppen und linearen, verzweigten oder cyclischen C1- bis C6-Alkylgruppen ausgewählte Gruppen substituiert ist,
. wobei mindestens eine der Gruppen R₃, von einem Wasserstoffatom verschieden ist,
. wobei zwei Gruppen R_{3'}, die an dasselbe Kohlenstoffatom des Peroxidrings gebunden sind, gemeinsam eine mono-, bi- oder tricyclische Struktur gemäß obiger Definition bilden können,
in Basen- oder Säureadditionssalzform sowie in Hydrat- oder Solvatform.

10. Verbindung der Formel (I) nach einem der Ansprüche 1, 2, 4 und 5, **dadurch gekennzeichnet, daß** sie der Formel (XIII) entspricht: 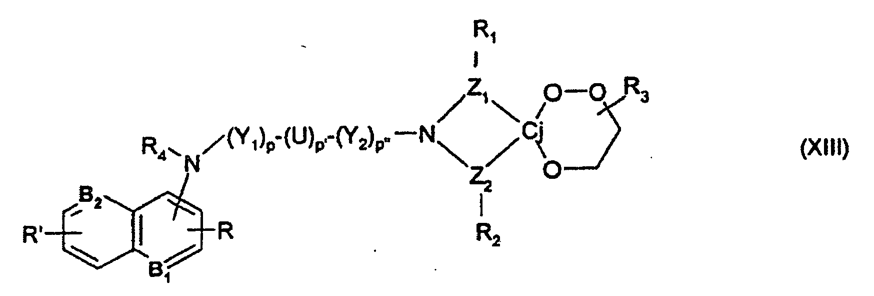 worin R, R', B₁, B_{2,} Y₁, U, Y₂, p, p', p", Z₁, Z₂. Cⱼ, R₁, R₂, R₃ und R₄ die in einem der Ansprüche 1, 2, 4 und 5 angegebene Bedeutung besitzen,
in Basen- oder Säureadditionssalzform sowie in Hydrat- oder Solvatform.

11. Verbindung der Formel (I) nach einem der Ansprüche 1, 2, 4 und 9, **dadurch gekennzeichnet, daß** sie der Formel (XIV) entspricht: 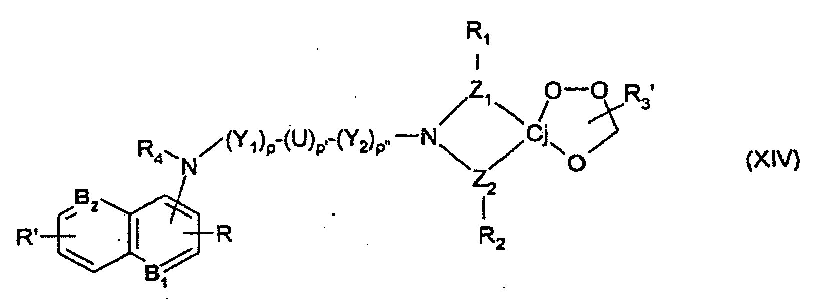 worin R, R', B₁, B₂, Y₁, U, Y₂, p, p', p", Z₁, Z₂, Cⱼ, R₁, R₂, R₃. und R₄ die in einem der Ansprüche 1, 2, 4 und 9 angegebene Bedeutung besitzen,
in Basen- oder Säureadditionssalzform sowie in Hydrat- oder Solvatform.

12. Verbindung der Formel (I) nach einem der vorhergehenden Ansprüche, **dadurch gekennzeichnet, daß**:
- Y₁ und Y₂ gleich oder verschieden sind und für eine lineare, verzweigte oder cyclische C1- bis C6-Alkylenkette, die gegebenenfalls einen oder mehrere Amin-, Amid-, Carbonyl- oder Etherreste enthält, steht, wobei diese C1- bis C6-Alkylenkette gegebenenfalls durch eine oder mehrere unter Halogenatomen, Hydroxylgruppen, Acetalgruppen und linearen, verzweigten oder cyclischen C1- bis C5-Alkylresten ausgewählte Gruppen substituiert ist,
- U für einen Amin-, Amid-, Carbonyl-, Carboxyl- oder Etherrest steht,
in Basen- oder Säureadditionssalzform sowie in Hydrat- oder Solvatform.

13. Verbindung der Formel (I) nach Anspruch 12, **dadurch gekennzeichnet, daß**:
- entweder p' = p" = 0, p = 1 und Y₁ für eine lineare oder verzweigte C1- bis C6-Alkylenkette, die gegebenenfalls durch eine oder mehrere unter Halogenatomen, Hydroxylgruppen, Acetalgruppen und linearen, verzweigten oder cyclischen C1- bis C5-Alkylresten ausgewählte Gruppen substituiert ist, steht,
- oder p = p' = 1, p" = 0, Y₁ für eine lineare oder verzweigte C1- bis C6-Alkylenkette, die gegebenenfalls durch eine oder mehrere unter Halogenatomen, Hydroxylgruppen, Acetalgruppen und linearen, verzweigten oder cyclischen C1- bis C5-Alkylresten ausgewählte Gruppen substituiert ist, steht und U für einen Amin-, Amid-, Carbonyl-, Carboxyl- oder Etherrest steht,
- oder p = p' = p" = 1, Y₁ und Y₂ für eine lineare oder verzweigte C1- bis C6-Alkylenkette, die gegebenenfalls durch eine oder mehrere unter Halogenatomen, Hydroxylgruppen, Acetalgruppen und linearen, verzweigten oder cyclischen C1- bis C5-Alkylresten ausgewählte Gruppen substituiert ist, steht und U für einen Amin-, Amid-, Carbonyl-, Carboxyl- oder Etherrest steht,
in Basen- oder Säureadditionssalzform sowie in Hydrat- oder Solvatform.

14. Verbindung der Formel (I) nach einem der vorhergehenden Ansprüche, **dadurch gekennzeichnet, daß** Z₁ und Z₂ gleich oder verschieden sind und jeweils für einen linearen oder verzweigten C1- bis C4-Alkylenrest stehen,
in Basen- oder Säureadditionssalzform sowie in Hydrat- oder Solvatform.

15. Verbindung der Formel (I) nach einem der Ansprüche 1, 2, 4, 5 und 10, **dadurch gekennzeichnet, daß** sie der Formel (XV) entspricht: 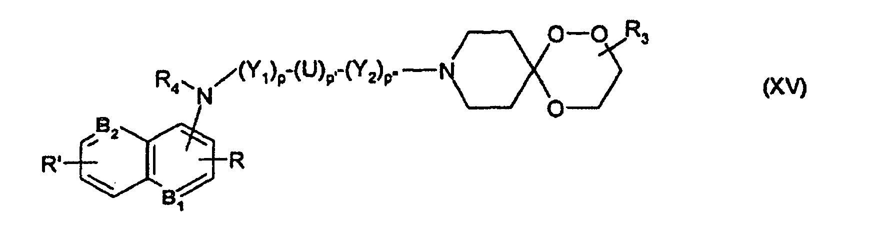 worin R, R', B₁, B₂, Y₁, U, Y₂, p, p', p", R₁, R₂, R₃ und R₄ die in einem der Ansprüche 1, 2, 4, 5 und 10 angegebene Bedeutung besitzen,
in Basen- oder Säureadditionssalzform sowie in Hydrat- oder Solvatform.

16. Verbindung der Formel (I) nach einem der Ansprüche 1, 2, 4, 9 und 11, **dadurch gekennzeichnet, daß** sie der Formel (XVI) entspricht: 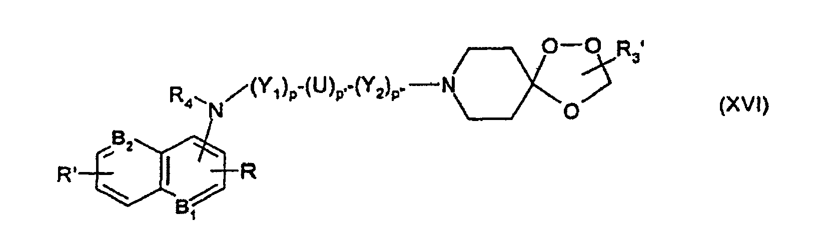 worin R, R', B₁, B₂, Y₁, U, Y₂, p, p', p", R₁, R₂, R₃. und R₄ die in einem der Ansprüche 1, 2, 4, 9 und 11 angegebene Bedeutung besitzen,
in Basen- oder Säureadditionssalzform sowie in Hydrat- oder Solvatform.

17. Verbindung der Formel (I) nach einem der vorhergehenden Ansprüche, **dadurch gekennzeichnet, daß** sie unter den folgenden Verbindungen ausgewählt ist:

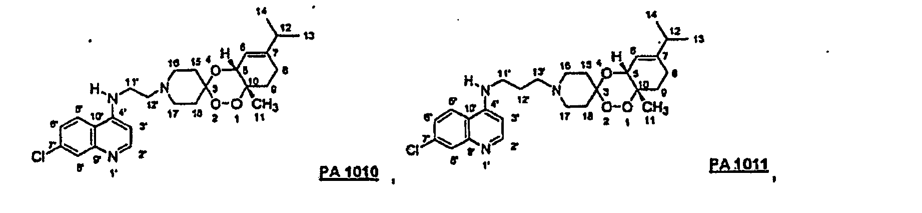

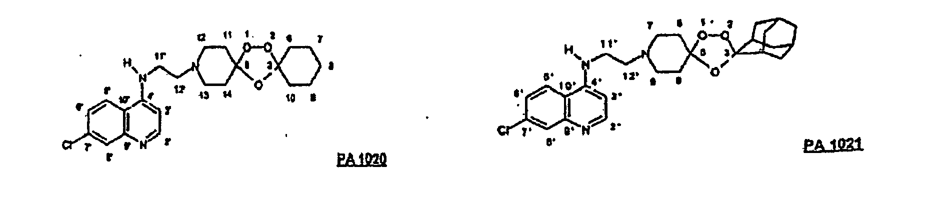

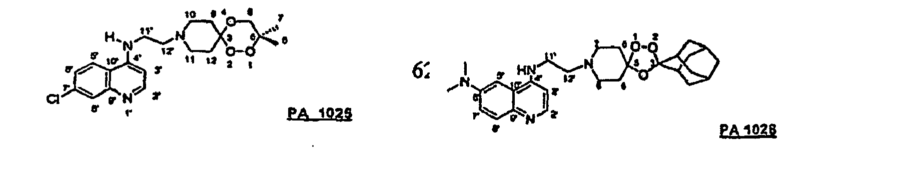

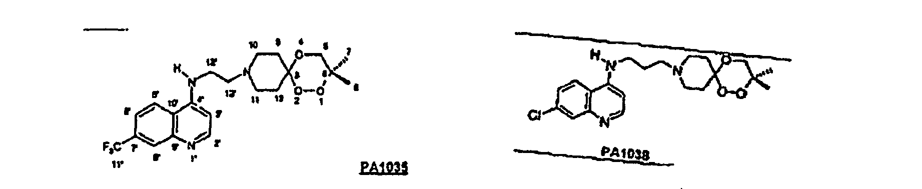

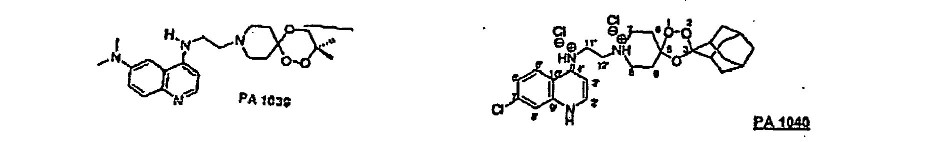

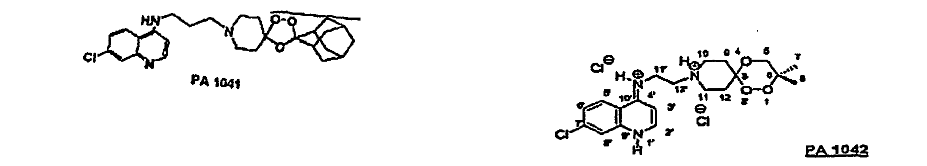

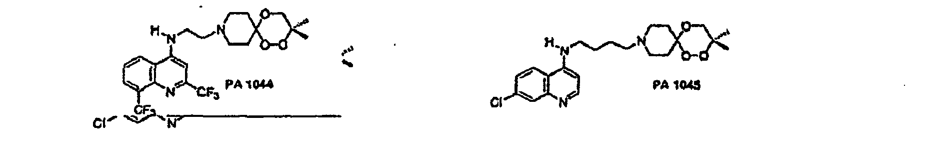

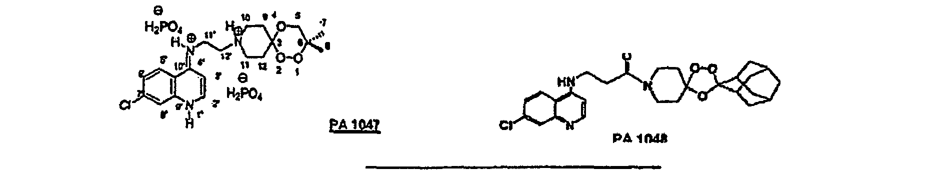

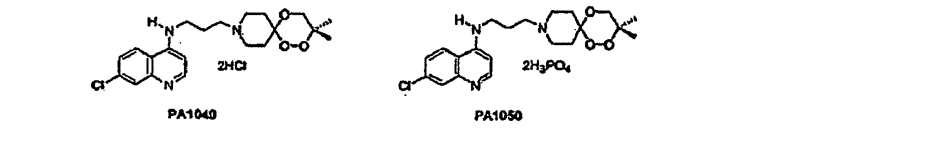

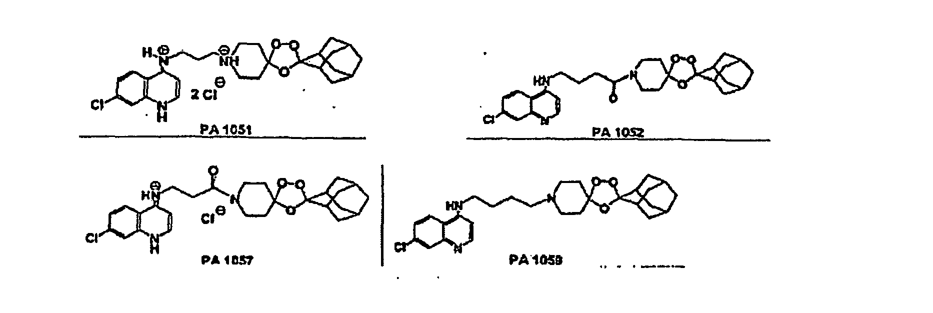

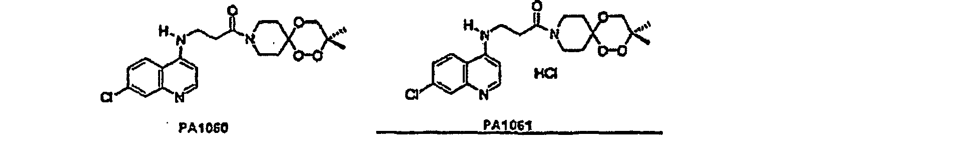

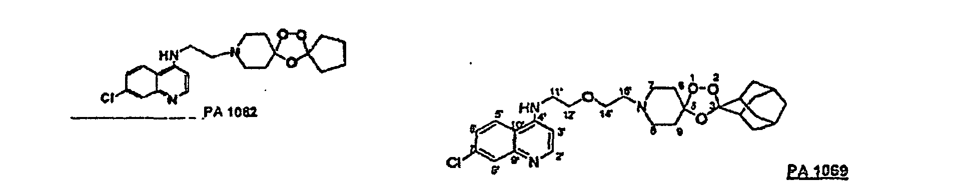

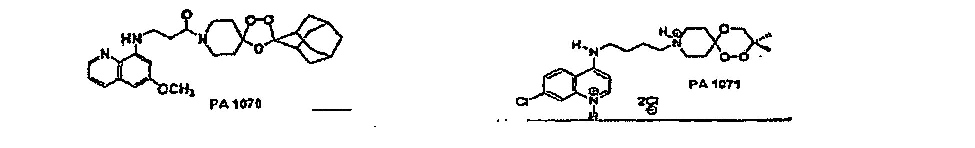

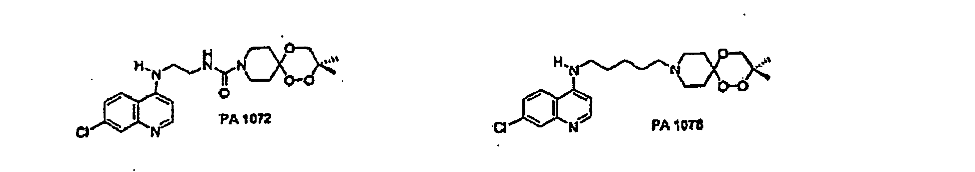

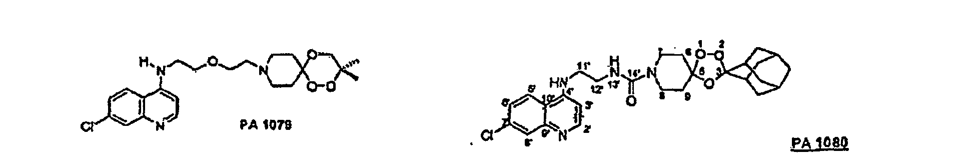

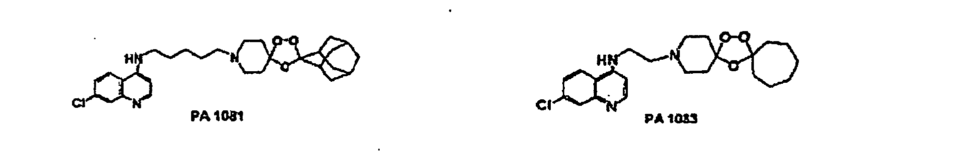

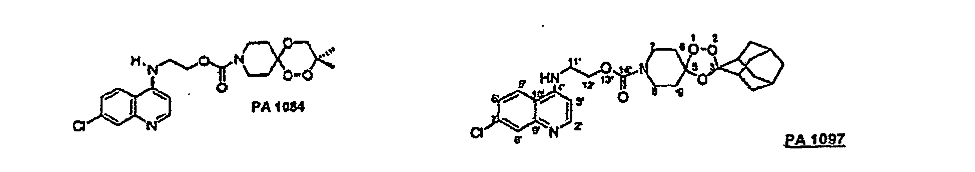

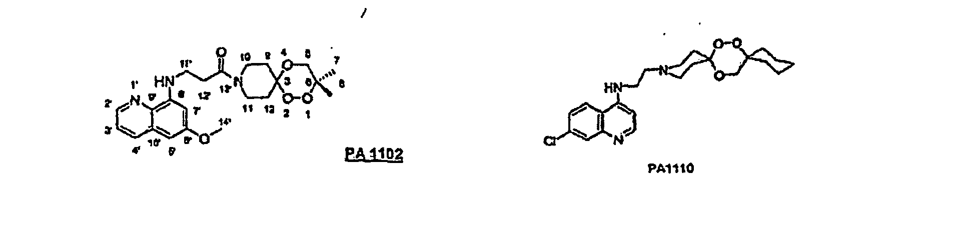

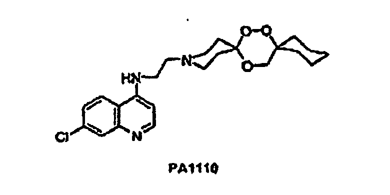

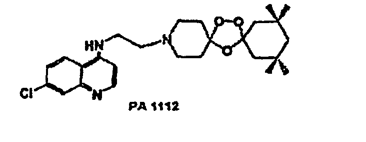

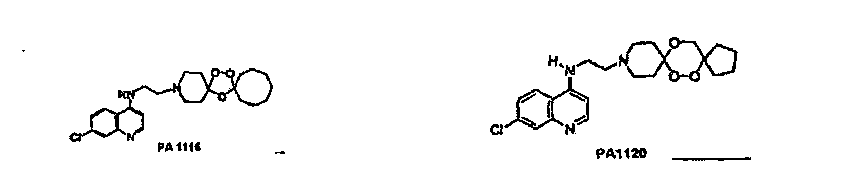

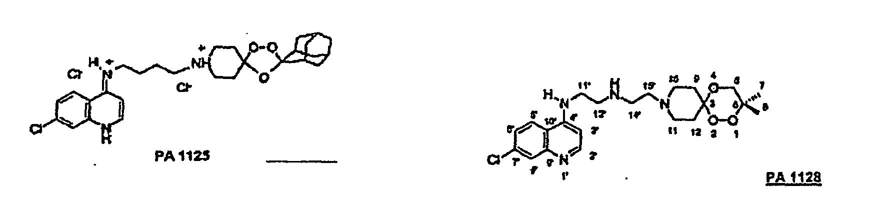

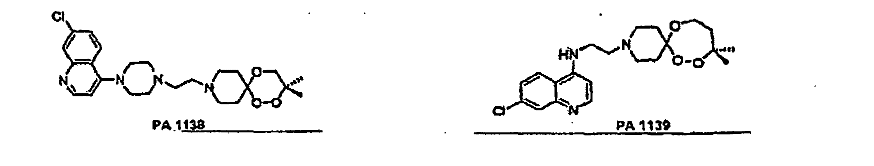

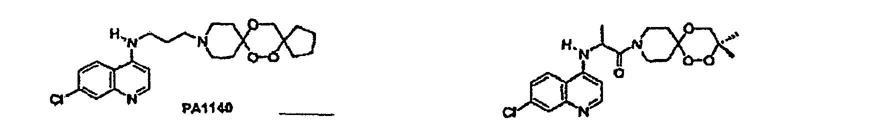

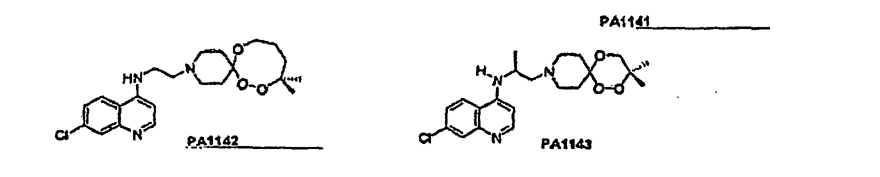

18. Verfahren zur Herstellung einer Verbindung der Formel (I) nach einem der vorhergehenden Ansprüche, **dadurch gekennzeichnet, daß** man reaktive Derivate von A, die eine funktionelle Gruppe wie eine Hydroxylgruppe oder eine Abgangsgruppe wie ein Halogenatom enthalten, und Peroxidderivate, die die Reste Rₓ und R_{y} enthalten, zur Ausbildung eines Kupplungsarms -(Y₁)ₚ-(U)_{p'}-(Y₂)_{p"}-gemäß der in Anspruch 1 angegebenen Definition zwischen diesen Derivaten umsetzt.

19. Herstellungsverfahren nach Anspruch 18, **dadurch gekennzeichnet, daß** man zur Herstellung von Verbindungen, die als Derivat A ein Aminochinolin der Formel (IIa) gemäß der in Anspruch 4 angegebenen Definition enthalten, eine Verbindung der Formel (XVII) : 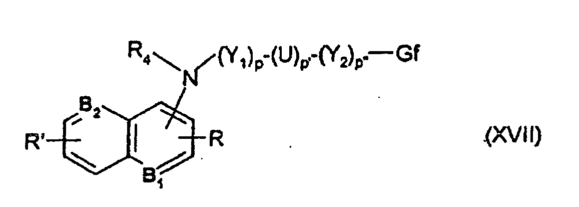 worin R, R¹, B₁, B₂, R₄, Y₁, Y₂, U, p, p' und p'' die in den Ansprüchen 1 oder 2 angegebene Bedeutung besitzen und Gf für eine funktionelle Gruppe steht, mit einer Verbindung der Formel (XVIII), worin Z₁, Z₂, R₁, R₂, Cj, Rₓ und R_{y} die in Anspruch 1 angegebene Bedeutung besitzen, umsetzt:

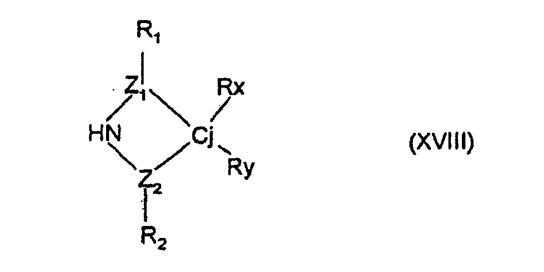

20. Herstellungsverfahren nach Anspruch 18, **dadurch gekennzeichnet, daß** man zur Herstellung von Verbindungen, in denen p = 1 und die als Derivat A ein Aminochinolin der Formel (IIa) gemäß der in Anspruch 2 angegebenen Definition enthalten, eine Verbindung der Formel (XIX): 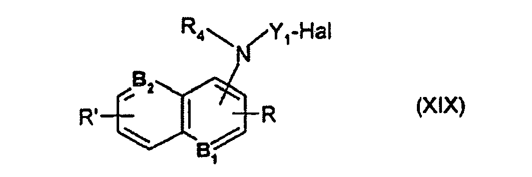 worin R, R', B₁, B₂, R₄ und Y₁ die in den Ansprüchen 1 oder 2 angegebene Bedeutung besitzen und "Hal" für ein Halogenatom steht, mit einer Verbindung der Formel (XX), worin U, Y₂, p' , p'' , Z₁, Z₂, R₁, R₂, Cj, Rₓ und R_{y} die in Anspruch 1 angegebene Bedeutung besitzen, umsetzt:

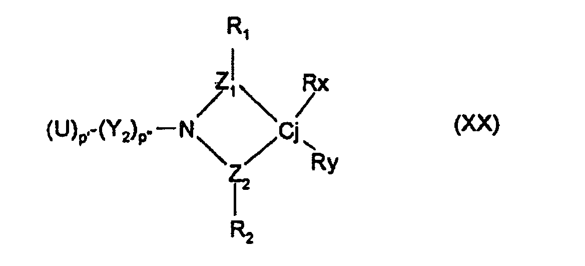

21. Verbindung der Formel (XVIII) oder (XX), worin U, Y₂, p', p", Z₁, Z₂, R₁, R₂ und Cj die in Anspruch 1 angegebene Bedeutung besitzen und Rₓ und R_{y} gemeinsam einen 6-, 7- oder 8-gliedrigen Peroxidring mit 1 oder 2 zusätzlichen Sauerstoffatomen in der cyclischen Struktur bilden, wobei Cj eine der Spitzen dieses cyclischen Peroxids ist:

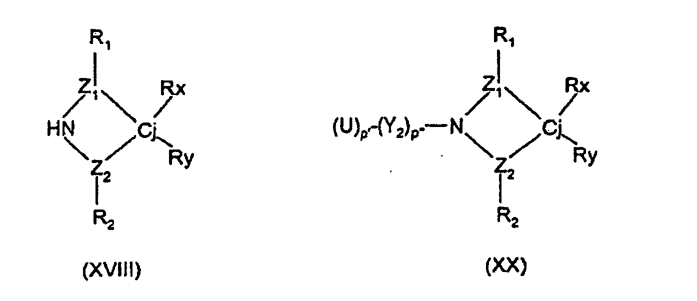

22. Verbindungen der Formel (XVIII) oder (XX) nach Anspruch 21, worin Rₓ und R_{y} gemeinsam ein Trioxan der Formel (XI) bilden:

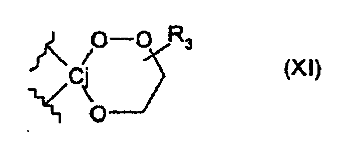

23. Arzneimittel, **dadurch gekennzeichnet, daß** es eine Verbindung der Formel (I) nach einem der Ansprüche 1 bis 17 oder ein Additionssalz dieser Verbindung mit einer pharmazeutisch unbedenklichen Säure oder auch ein Hydrat oder ein Solvat der Verbindung der Formel (I) enthält.

24. Phermazeutische Zusammensetzung, **dadurch gekennzeichnet, daß** sie eine Verbindung der Formel (I) nach einem der Ansprüche 1 bis 17 oder ein Additionssalz dieser Verbindung mit einer pharmazeutisch unbedenklichen Säure, ein Hydrat oder ein Solvat dieser Verbindung sowie mindestens einen pharmazeutisch unbedenklichen Hilfsstoff enthält.

25. Verwendung einer Verbindung der Formel (I) nach einem der Ansprüche 1 bis 17 zur Herstellung eines Arzneimittels zur Behandlung und Prävention von Malaria.
